# EUROPEAN PATENT SPECIFICATION

(11) **EP 4 351 691 B1**
(45) Date of publication and mention of the grant of the patent: **18.03.2026**
(21) Application number: 22740703.8
(22) Date of filing: 09.06.2022
(51) Int. Cl.: A61M 25/06, A61M 25/00, A61F 2/24, A61L 29/12

(54) **ATRAUMATIC TIP FOR EXPANDABLE AND RECOVERABLE SHEATH**
ATRAUMATISCHE SPITZE FÜR EXPANDIERBARE UND WIEDERVERWENDBARE HÜLLE
EMBOUT ATRAUMATIQUE POUR GAINE EXPANSIBLE ET RECOUVRABLE

(30) Priority: 10.06.2021 US 202163209337 P; 24.06.2021 US 202163214605 P; 24.06.2021 US 202163214349 P; 24.03.2022 US 202263323429 P
(43) Date of publication of application: 17.04.2024
(73) Proprietor: Edwards Lifesciences Corporation, Irvine, CA 92614 (US)
(72) Inventor: TRAN, Sonny, Irvine, CA 92614 (US); TAMIR, Ilan, Irvine, CA 92614 (US); LEE, Jeong Soo, Irvine, CA 92614 (US); FINE, Maxwell Harrison, Costa Mesa, CA 92627 (US); SALEH, Nasser William, Irvine, CA 92614 (US); MAK, Sovanpheap, Irvine, CA 92614 (US); NGUYEN, Tien Thi, Irvine, CA 92614 (US); BULMAN, Erik, Irvine, CA 92614 (US); MAIMON, David, 3079892 Caesarea (IL)
(74) Representative: Eisenführ Speiser
(86) International application number: PCT/US2022/032902
(87) International publication number: WO 2022/261368

(56) References cited:
- WO-A1-2017/151659
- WO-A1-2018/213222
- WO-A1-2020/055820
- WO-A1-2020/247907
- WO-A2-96/33763
- US-A1- 2010 094 392

## Description

### FIELD

The present application concerns aspects of a sheath for use with catheter-based technologies for repairing and/or replacing heart valves and delivering a prosthetic device, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (*e.g.*, the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced. The publication WO 2020/055820 Al discloses an introducer with expandable capabilities, whilst the publication WO 2018/213222 Al discloses a radially expandable introducer sheath.

However, delivery and/or removal of prosthetic devices and other material to or from a patient still poses a significant risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

Accordingly, there remains a need in the art for an improved introducer sheath for endovascular systems used for implanting valves and other prosthetic devices.

### SUMMARY

The aforementioned objectives are achieved according to the invention by means of an expandable sheath as defined in claim 1. Preferred embodiments are defined in dependent claims 2-15.

In certain aspects disclosed herein is an expandable sheath that can minimize trauma to the vessel by allowing for temporary expansion of a portion of the introducer sheath to accommodate a delivery system, followed by a return to the original diameter once the delivery system passes through. Aspects of the present disclosure are directed to a sheath with a smaller profile than that of prior art introducer sheaths. It is understood that in certain aspects, the use of the disclosed herein sheath can reduce the length of time a procedure takes, as well as reduce the risk of a longitudinal or radial vessel tear or plaque dislodgement because only one sheath is required, rather than several different sizes of sheaths. In further aspects, the introductions of the disclosed herein expandable sheaths require only a single vessel insertion, as opposed to requiring multiple insertions for the dilation of the vessel.

In certain aspects, disclosed herein is an expandable sheath comprising a proximal section having a proximal end, a distal end, and an elongated body that extends between the proximal end and distal end, wherein the elongated body defines a lumen extending therethrough, and a tip section extending distally from the distal end of the proximal section and defining a lumen extending therethrough, the tip section comprising: an inner liner layer; an intermediate layer disposed radially outward of the inner liner layer, the intermediate layer having a lower stiffness than the inner liner layer; an elastomeric outer layer disposed radially outward of the intermediate layer; and wherein the tip section has an initial unexpanded configuration wherein a distal end of the tip section has a first diameter, and an expanded configuration wherein the distal end of the tip section has a second diameter that is greater than the first diameter and a distal end; and wherein at least the inner liner layer defines a slit extending distally along at least a portion of the tip section.

Also disclosed are aspects of the sheath, wherein the slit does not extend to the distal end of the tip section in the initial unexpanded configuration, such that the distal end of the tip section is circumferentially continuous in the initial unexpanded configuration. While in other exemplary and unlimiting aspects, disclosed is a sheath wherein the slit extends to the distal end of the tip section when the tip section is in the expanded configuration, such that the distal end of the tip section is circumferentially discontinuous in the expanded configuration.

Also disclosed are aspects of the exemplary sheath wherein the slit is at least partially visually concealed when at least partially reflowed in the initial unexpanded configuration.

Also disclosed herein are methods of delivering a medical device into a patient, the method comprising: inserting an expandable sheath into the patient; advancing the medical device through a lumen extending through the expandable sheath causing at least local expansion of the lumen as the medical device passes therethrough; and splitting, by advancing the medical device, a tip section of the expandable sheath open at a slit extending distally along at least a portion of the tip section and through at least an inner liner layer and an outer elastomeric layer of the tip section; wherein splitting the tip expands the slit by spreading two side edges of the slit circumferentially away from each other. This and all other methods according to the present disclosure are not in accordance with the claims.

In yet still further aspects, the methods further comprise retracting at least a portion of the medical device into the lumen through the split tip section.

Additional aspects of the disclosure will be set forth, in part, in the detailed description, figures, and claims which follow, and in part will be derived from the detailed description or can be learned by practice of the disclosure. It is to be understood that both the foregoing general description and the following detailed description are exemplary and explanatory only and are not restrictive of the invention as disclosed.

### BRIEF DESCRIPTION OF THE DRAWINGS

**FIGURE 1** is an elevation view of a sheath according to the present disclosure, along with an endovascular delivery apparatus for implanting a prosthetic valve.
**FIGURES 2A** and **2B** are section views of aspects of a sheath for introducing a prosthetic device into a patient, and **FIGURE 2C** is a perspective view of one component of such a sheath.
**FIGURES 3A, 3B,** and **3C** are elevation views of three aspects of a sheath according to the present disclosure, having a uniform and varying rest *dᵣ* diameters.
**FIGURE 4A-4D** illustrates partial elevation views of various exemplary aspects of a braid structure with various PIC according to the present disclosure.
**FIGURES 5A** and **5B** illustrate a section view of one aspect of an exemplary inner liner: **FIGURE 5A** depicts an unexpanded configuration, while **FIGURE 5B** depicts an expanded configuration.
**FIGURES 6A-6E** **and** **6H** show section views of various aspects of exemplary sheaths. **FIGURES 6F, 6G****,** and **6I** show perspective views of various aspects of exemplary sheaths.
**FIGURE 7** illustrates a block diagram of one aspect of a method of making a sheath according to the present disclosure.
**FIGURE 8** illustrates a block diagram of another aspect of a method of making a sheath according to the present disclosure.
**FIGURES 9A-9K** illustrate section or side views of various method steps of the methods shown in **FIGURES 7-8****.**
**FIGURE 10** is an elevation view of an expandable sheath according to the present disclosure and representative housing.
**FIGURE 11** is an enlarged cutaway view of the distal end of the sheath of **FIGURE 10****.**
**FIGURE 12A-12D** are section views of the distal end of the exemplary sheath of **FIGURE 14****,** taken along line 37-37 in **FIGURE 11****.**
**FIGURES 13A-13D** are section views of a proximal section of the sheath of **FIGURE 10****,** taken along line 38-38 in **FIGURE 11****.**
**FIGURE 14** is a section view of the sheath of **FIGURE 10** in a rest (unexpanded) configuration, taken along line 39-39 in **FIGURE 11****.**
**FIGURE 15** shows a section view of the sheath in **FIGURE 14** in an expanded configuration.
**FIGURE 16** shows experimental data for an exemplary sheath in one aspect.
**FIGURE 17** shows experimental data for an exemplary sheath in another aspect.
**FIGURE 18** depicts an inner liner of an exemplary sheath in one aspect in a collapsed and unexpended configuration.
**FIGURE 19** depicts a cross-sectional view of an exemplary sheath in one aspect showing the inner liner as shown in **FIG.18** and an outer layer.
**FIGURES 20A-20C** depict exemplary manufacturing steps of the inner liner, as shown in **FIG. 18****.**
**FIGURES 21A-21B** depicts an exemplary sheath in one aspect showing an inner liner and an outer layer in a collapsed and unexpended configuration: **FIG. 21** **A** illustrates a cross-sectional schematic of the sheath and **FIG. 21B** depicts a side-view schematic of the sheath.
**FIGURES 22A-22B** depict an exemplary sheath in one aspect: FIG. 22A shows a schematic of a cross-sectional view of the sheath in a collapsed configuration (left) and an expanded configuration (right); **FIG. 22B** shows snapshots of an exemplary sheath expanding during the passage of a medical device.
**FIGURE 23** depicts a cross-sectional view schematic of an exemplary sheath in one aspect.
**FIGURES 24A-24B** depict a cross-sectional view schematic of an exemplary sheath in one aspect.
**FIGURES 25A-25H** depict various inner liner combinations for an exemplary sheath: **FIGS. 25A-25D** show a cross-sectional view of various liners prior to forming an exemplary inner liner; **FIGS. 25E-25H** show various cross-sectional views of an exemplary inner liner in a spiral configuration.
**FIGURE 26** depicts a side view of an exemplary lubricant pattern disposed on an exemplary inner liner in one aspect.
**FIGURES 27A-27B** depicts a cross-sectional view of an exemplary sheath in collapsed (FIG. **27A****)** and in an expanded configuration **(****FIG. 27B****)** with bonding between an inner liner and outer liner.
**FIGURES 28A-28C** depict various methods of forming a bond between an inner liner and an outer layer of an exemplary sheath.
**FIGURES 29A-29F** depict schematics of a reinforcing jacket effect **(****FIGS.25A-25D****)** and a ballooning guard effect on patient anatomy **(****FIGS. 25A-25B** and **25E-25F).**
**FIGURES 30A-30B** depict schematics of a reinforcing jacket present in an exemplary sheath in one aspect.
**FIGURE 31** depicts a method of making an exemplary sheath in one aspect.
**FIGURE 32** depicts an exemplary sheath in one aspect.
**FIGURE** 33 depicts an exemplary sheath in one aspect.
**FIGURE 34** depicts an exemplary sheath in one aspect.
**FIGURE 35** is a cross-section view of an exemplary sheath according to another aspect.
**FIGURE 36** is a cross-section view of an exemplary sheath according to another aspect.
**FIGURE** 37 is an elevation view of an exemplary outer layer according to another aspect.
**FIGURE 38** is a cross-section view of an exemplary elongated tube taken along section line A-A of **FIG. 37****.**
**FIGURE 39** is a section view of an exemplary outer layer in a rest (unexpanded) configuration, take along section lines B-B of **FIG 37****.**
**FIGURE 40** is a partial section view of an exemplary outer layer in one aspect.
**FIGURE 41** is a section view of another exemplary outer layer in a rest (unexpanded) configuration, including a single reinforcing member, taken along section line B-B of **FIG. 37****.**
**FIGURES 42A-42C** show an exemplary sheath having a two-layer construction in one aspect.
**FIGURES 43A-43B** show an exemplary sheath having at least one bonding site on an inner surface of the outer layer in one aspect.
**FIGURE 44** is a side elevation view of an exemplary sheath according to the invention having a proximal section and a tip section in an unexpanded configuration.
**FIGURE 45** is a perspective view of the tip section of the sheath of **FIG. 44** in the unexpanded configuration.
**FIGURE 46** is another perspective view of the exemplary sheath of **FIG. 44** with the tip section in an expanded configuration.
**FIGURE 47** is a side elevation view of the tip section of the sheath of **FIG. 44** in a split configuration.
**FIGURE 48** is a perspective view of the tip section of the sheath of **FIG. 44** in a split configuration.
**FIGURE 49** is a plan view of an inner liner layer with cuts for formation into a tip section.
**FIGURE 50** is a perspective view of the inner liner layer of **FIG. 49** in a rolled configuration.
**FIGURE 51** is a top plan view of the inner liner layer of **FIG. 50****.**
**FIGURE 52** is an enlarged view of a portion of the inner liner layer of **FIG. 51****.**
**FIGURE 53** is a plan view of an inner liner layer with an additional section removed for shaping the tip section.
**FIGURE 54** is a side elevation view of a rolled inner liner layer of another aspect according to the invention having a partially shaped distal tip section.
**FIGURE 55** is a perspective view of the distal tip section of **FIG. 54****.**
**FIGURE 56** is a top plan view of the distal tip section of **FIG. 54****.**
**FIGURE 57** is an enlarged view of a portion of **FIG. 56****.**
**FIGURE 58** is a side elevation view of the distal tip section of **FIG. 54** receiving an intermediate layer for further shaping the distal tip section.
**FIGURE 59** is a side elevation view of the distal tip section of **FIG. 58** further assembled.
**FIGURE 60** is a perspective view of the distal tip section of **FIG. 59** with the intermediate layer shaped such as by flow melting.
**FIGURE 61** is a side elevation view of the distal tip section of **FIG. 60****.**
**FIGURE 62** is a top plan view of the distal tip section of **FIG. 60****.**
**FIGURE 63** is a schematic of an assembly process for a distal tip section of an expandable delivery sheath of another aspect of the present disclosure, including rolling and scoring of an inner liner layer.
**FIGURE 64** is a schematic of an assembly process, including placement of an intermediate tie layer onto a shaped inner liner and incorporation of a radiopaque marker of a distal tip section.
**FIGURE 65** is a schematic of an assembly process, including further shaping of the intermediate layer of a distal tip section.
**FIGURE 66** is a side elevation view of a distal tip section of an expandable delivery sheath of another aspect according to the invention.
**FIGURE 67** is a side elevation view of an inner layer of a an aspect according to the invention with an intermediate layer at least partially shaped into a distal tip section.
**FIGURE 68** is a cross-sectional view of tube for forming an intermediate layer of another aspect.
**FIGURE 69** is a cross-sectional view of a tube for forming an intermediate layer having three sublayers of another aspect.

### DETAILED DESCRIPTION

The detailed description includes references to non-SI units ("inches"). These are to be converted to SI-units (for example mm) using the following factor:
1 inch = approximately 25.4 mm.

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "polymer" includes aspects having two or more such polymers unless the context clearly indicates otherwise.

It is also to be understood that the terminology used herein is for the purpose of describing aspects only and is not intended to be limiting. As used in the specification and in the claims, the term "comprising" can include the aspects "consisting of" and "consisting essentially of." Additionally, the term "includes" means "comprises."

For the terms "for example" and "such as," and grammatical equivalences thereof, the phrase "and without limitation" is understood to follow unless explicitly stated otherwise.

References in the specification and concluding claims to parts by weight of a particular element or component in a composition or article denotes the weight relationship between the element or component and any other elements or components in the composition or article for which a part by weight is expressed. Thus, in a composition or a selected portion of a composition containing 2 parts by weight of component X and 5 parts by weight component Y, X and Y are present at a weight ratio of 2:5 and are present in such ratio regardless of whether additional components are contained in the composition.

A weight percent of a component, unless specifically stated to the contrary, is based on the total weight of the formulation or composition in which the component is included.

Ranges can be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It should be further understood that the endpoints of each of the ranges are significant both in relation to the other endpoint and independently of the other endpoint.

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

As used herein, the term "substantially," when used in reference to a composition, refers to at least about 80%, at least about 85%, at least about 90%, at least about 91%, at least about 92%, at least about 93%, at least about 94%, at least about 95%, at least about 96%, at least about 97%, at least about 98%, at least about 99%, or about 100% by weight, based on the total weight of the composition, of a specified feature or component.

As used herein, the term "substantially," in, for example, the context "substantially free" refers to a composition having less than about 1 % by weight, e.g., less than about 0.5 % by weight, less than about 0.1 % by weight, less than about 0.05 % by weight, or less than about 0.01 % by weight of the stated material, based on the total weight of the composition.

As used herein, the terms "substantially identical reference composition" or "substantially identical reference article" refer to a reference composition or article comprising substantially identical components in the absence of an inventive component. In another exemplary aspect, the term "substantially," in, for example, the context "substantially identical reference composition," refers to a reference composition comprising substantially identical components and wherein an inventive component is substituted with a common in the art component.

Further, the terms "coupled" and "associated" generally mean electrically, electromagnetically, and/or physically (*e.g*., mechanically or chemically) coupled or linked and do not exclude the presence of intermediate elements between the coupled or associated items.

As used herein, the term "atraumatic" is commonly known in the art and refers to a device or a procedure that minimized tissue injury.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, an appropriate, effective amount will be readily determined by one of ordinary skill in the art using only routine experimentation.

Although the operations of exemplary aspects of the disclosed method may be described in a particular sequential order for convenient presentation, it should be understood that disclosed aspects can encompass an order of operations other than the particular sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### SHEATH

Disclosed herein one aspect of a sheath for delivering a medical device, wherein the sheath has a proximal and a distal end and comprises: a) an inner liner defining a lumen having a first rest diameter *dᵣ* and a second expanded diameter *dₑ*, wherein the lumen is configured to receive and pass through a medical device, wherein the inner liner comprises a sheet comprising a first portion having a first surface and an opposite second surface, wherein a first end of the first portion splits into a first segment having a first surface and an opposite second surface, and a third segment having a first surface and an opposite second surface, and wherein a second end of the first portion extends into a second segment having a first surface and an opposite second surface, wherein the sheet is rolled into a spiral configuration, such that at least a portion of the first surface of the second segment overlaps at least a portion of the second surface of the first segment, wherein at least a portion of the first surface of the third segment overlaps at least a portion the second surface of the second segment, and wherein at least a portion of the first surface of the third segment overlaps at least a portion of the second surface of the first segment, wherein the first surface of the first portion extends into the first surface of the first segment, the second segment and the third segment, and wherein the second surface of the first portion extends into the second surface of the second and the third segments; wherein each segment is configured to slidably move along each other upon passage of the medical device through the lumen; wherein the sheet comprises a polymer layer; and an outer layer.

In certain aspects, the rest diameter *dᵣ* can be substantially uniform along the longitudinal axis of the lumen. In yet other aspects, the rest diameter *dᵣ* varies along the longitudinal axis of the lumen and wherein the rest diameter *dᵣ* at the proximal end that is larger than the rest diameter *dᵣ* at the distal end. In one disclosed aspect, the expanded diameter *dₑ* is configured to accommodate the medical device passing through the lumen. In yet another aspect, the sheath can contract to the predetermined rest diameter *dᵣ* after passage of the medical device through the lumen.

In one disclosed aspect, the sheet of the sheath comprises a high-density polyethylene, polypropylene, polyamide, fluoropolymer, copolymers thereof, or blends thereof. In a still further aspect, the sheet can have a multilayer structure. In still further aspects, the inner surface of the sheet is at least partially ribbed.

In one exemplary aspect, the sheet is lubricious and has a coefficient of friction less than about 0.5.

In a still further aspect, an amount of a first lubricant is disposed between at least a portion of the inner liner and at least a portion of the outer layer. In yet other disclosed aspects, an amount of a second lubricant can be disposed between at least a portion of the overlying portion of the sheet and at least a portion of the sliding portions of the sheet.

In yet other disclosed aspects, the outer surface of the layer of the elastomeric polymer can define at least a portion of the outer surface of the outer layer. In some of the disclosed aspects, at least a portion of the inner surface of the layer of the elastomeric polymer is at least partially bonded to at least a portion of the outer surface of the sheet of the inner liner. In still further aspects, at least a portion of the inner surface of the layer of the elastomeric polymer can define at least a portion of the inner surface of the outer layer. While in other aspects, at least a portion of the braid or coil can define at least a portion of the inner surface of the outer layer.

In one disclosed aspect, the sheath can further comprise a first strip of the elastomeric polymer disposed along at least a portion of the longitudinal axis of the lumen between at least a portion of the outer surface of the sheet that does not comprise the overlaying portion of the sheet and the inner surface of the outer layer. In other aspects, the sheath can further comprise a second strip of the elastomeric polymer disposed between at least a portion of the outer surface of the sheet at the proximal end of the sheath and the inner surface of the outer layer. While in other aspects, the sheath can further comprise a third strip of the elastomeric polymer disposed between at least a portion of the outer surface of the sheet at the distal end of the sheath and the inner surface of the outer layer.

In still further aspects, the braid or coil is an expandable braid or coil. In still further aspects, the braid or coil can comprise at least one filament comprising stainless steel, nitinol, a polymer material, or a composite material. In certain aspects, the at least one filament can be a round filament or a flat filament. In aspects where the at least one filament comprises a polymer material, the polymer material can be polyester or nylon. In aspects where the at least one filament is round, the round filament can have a diameter of less than about 0.015". While in the aspects where the at least one filament is flat, the flat filament can have a height of less than about 0.006" and a width from greater than about 0.003" to about 0.015". In yet further aspects, the braid can have a per-inch crosses (PIC) count of less than 50. In yet still further aspects, the braid's PIC can vary along the longitudinal axis of the lumen.

In still further aspects, where the at least one filament is nitinol, the nitinol is heat set at *dₑ.* In yet other aspects, where the at least one filament comprises stainless steel or nitinol, the filament can be configured to be atraumatic at least at the distal end of the sheath.

In yet other aspects, the elastomeric polymer present in the outer layer comprises a styrene-based elastomer, polyurethane, latex, copolymers thereof, blends thereof, or co-extrudates thereof. In one aspect, the elastomeric polymer exhibits a Shore A durometer of less than 90. In certain aspects, the braid or coil can be at least partially embedded within at least a portion of the layer of the elastomeric polymer. And yet, in other aspects, a hydrophilic coating layer can be disposed on the outer surface of the outer layer.

Also disclosed herein is an aspect comprising methods of making a sheath having a proximal and a distal end. In certain aspect, the method of making such a sheath comprises forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid or coil; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

In some exemplary aspects, the step of forming the variable inner liner comprises rolling the sheet over a mandrel having a predetermined diameter to form the spiral configuration, wherein the predetermined diameter of the mandrel is substantially identical to the predetermined diameter *dᵣ* of the inner liner. In still further aspects, the rest diameter *dᵣ* is substantially uniform along the longitudinal axis of the lumen. While in other aspects, the rest diameter *dᵣ* varies along the longitudinal axis of the lumen and wherein the rest diameter *dᵣ* at the proximal end that is larger than the rest diameter *dᵣ* at the distal end. In still further aspects, the expanded diameter *dₑ* of the sheath is configured to accommodate the medical device passing through the lumen. While in other aspects, the sheath formed by the methods disclosed herein can contract to the predetermined rest diameter *dᵣ* after passage of the medical device through the lumen.

In still further aspects, the step of forming the outer layer comprises mounting the braid or coil on the inner liner. In still further aspects, the step of forming the outer layer further comprises mounting the elastomeric polymer on the braid or coil. In some exemplary aspects, the methods disclosed herein can further comprise at least partially embedding the braid or coil within at least a portion of the layer of the elastomeric polymer. In yet other exemplary aspects, the step of forming the outer layer can comprise mounting the layer of the elastic polymer on the braid or coil and then mounting the layer of the elastic polymer and the braid or coil on the inner liner positioned on the mandrel. In such exemplary aspects, the method can further comprise at least partially embedding the braid or coil within at least a portion of the layer of the elastomeric polymer before mounting on the inner liner. While in other aspects, the method can further comprise at least partially embedding the braid or coil within at least a portion of the layer of the elastomeric polymer after mounting on the inner liner. In still further aspects, the sheath is removed from the mandrel after the outer layer is mounted on the inner liner, and the binding is complete.

In other aspects, in the methods disclosed herein, the outer surface of the layer of the elastomeric polymer can define at least a portion of the outer surface of the outer layer. Yet, in other aspects, in the methods disclosed herein, at least a portion of the inner surface of the layer of the elastomeric polymer can define at least a portion of the inner surface of the outer layer. Still further disclosed herein are the aspects wherein at least a portion of the braid or coil can define at least a portion of the inner surface of the outer layer.

In certain aspects, the methods disclosed herein further comprise bonding at least a portion of the inner surface of the layer of the elastomeric polymer to at least a portion of the outer surface of the sheet of the inner liner. In one aspect, the bonding is performed by heating at a temperature from about 350 °F to about 550 °F for a time period effective to form a bond between at least a portion of the outer layer and at least a portion of the inner liner.

One aspect, as described herein methods, comprises a first strip of the elastomeric polymer be applied along at least a portion of the longitudinal axis of the lumen to at least a portion of the outer surface of the sheet that does not comprise the overlaying portion prior to or during the step of bonding the at least a portion of the inner surface of the layer of the elastomeric polymer to at least a portion of the outer surface of the sheet of the inner liner. In yet other aspects, a second strip of the elastomeric polymer can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. In still further aspects, a third strip of the elastomeric polymer can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner.

In still further aspects and as described herein, an amount of a first lubricant can be applied to at least a portion of the inner liner prior to the step of forming the outer layer such that the amount of the first lubricant is disposed between at least a portion of the inner liner and at least a portion of the outer liner in the sheath. While in other aspects, an amount of a second lubricant is applied to at least a portion of the overlying and sliding portions of the sheet prior to the step of forming the outer layer.

In other aspects, in the methods described herein, the sheet can comprise a high-density polyethylene, polypropylene, polyamide, fluoropolymer, copolymers thereof, or blends thereof. In yet other aspects, the sheet can have a multilayer structure. In still further aspects, the inner surface of the sheet can be at least partially ribbed. In yet other aspects, the sheet can be lubricious and have a coefficient of friction less than about 0.5, less than about 0.4, less than about 0.3, less than about 0.2, or less than about 0.1.

In yet further aspects, the methods disclosed herein comprise the braid or coil, wherein the braid or coil is an expandable braid or coil. In still further aspects, the braid or coil of the disclosed methods can comprise at least one filament comprising a stainless steel, nitinol, a polymer material, or a composite material. In certain aspects, the at least one of the filaments can be a round filament or a flat filament. In certain aspects, the polymeric material present in the braid can be a polyester or nylon. In the aspects where the at least one filament is the round filament, such a filament can have a diameter of less than about 0.015". In yet other aspects, where the at least one filament is the flat filament, such a filament can have a height of less than about 0.006" and a width greater than about 0.003" to about 0.015", including exemplary values of about 0.004", about 0.005", about 0.006", about 0.007", about 0.008", about 0.009", about 0.010", about 0.011", about 0.012", about 0.013", and about 0.014". In the aspects of the methods disclosed herein, the braid can have a per-inch crosses (PIC) count of less than 50, less than 45, less than 40, or even less than 35. In yet further exemplary aspects, the PIC can vary along the longitudinal axis of the lumen.

In the aspects where the at least one filament comprises nitinol, the nitinol is heat set at *dₑ.* In the aspects where the at least one filament comprises stainless steel or nitinol, the filament is configured to be atraumatic, at least at the distal end of the sheath.

In yet further aspects, the methods disclosed herein comprise the elastomeric polymer comprising a styrene-based elastomer, polyurethane, latex, copolymers thereof, blends thereof, or co-extrudates thereof. In yet further aspects, the elastomeric polymer can exhibit a Shore A durometer of less than 90. In still further aspects, the methods can further comprise disposing a hydrophilic coating layer on the outer surface of the layer of the elastomeric polymer.

In some methods, a soft tip portion can be coupled to a distal end of the expandable sheath to facilitate passing the expandable sheath through a patient's vasculature.

Disclosed aspects of an expandable sheath can minimize trauma to the vessel by allowing for temporary expansion of a portion of the introducer sheath to accommodate the delivery system, followed by a return to the original diameter once the device passes through. Some aspects can comprise a sheath with a smaller profile *(e.g.,* a smaller diameter in the rest configuration) than that of prior art introducer sheaths. Furthermore, present aspects can reduce the length of time a procedure takes, as well as reduce the risk of a longitudinal or radial vessel tear or plaque dislodgement because only one sheath is required, rather than several different sizes of sheaths. Aspects of the present expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices *(e.g.,* stents, prosthetic heart valves, stented grafts, etc.) into many types of vascular and non-vascular body lumens (*e.g.*, veins, arteries, esophagus, ducts of the biliary tree, intestine, urethra, fallopian tube, other endocrine or exocrine ducts, etc.).

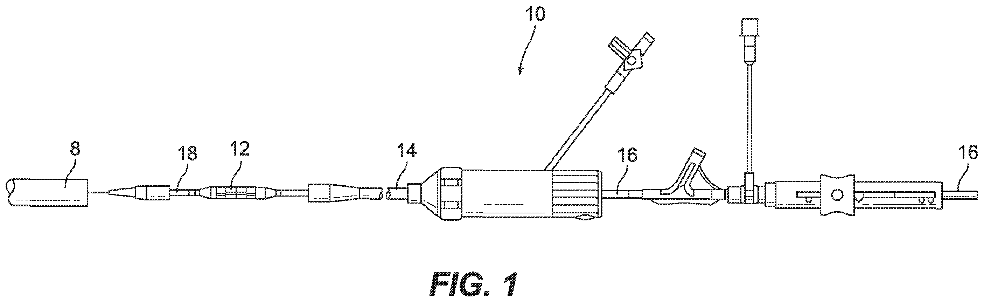

**FIG. 1** illustrates a sheath **8** according to the present disclosure, in use with a representative delivery apparatus **10,** for delivering a prosthetic device **12,** such as a tissue heart valve to a patient. The apparatus **10** can include a steerable guide catheter **14** (also referred to as a flex catheter), a balloon catheter **16** extending through the guide catheter **14,** and a nose catheter **18** extending through the balloon catheter **16.** The guide catheter **14,** the balloon catheter **16,** and the nose catheter **18** in the illustrated aspect are adapted to slide longitudinally relative to each other to facilitate delivery and positioning of the valve **12** at an implantation site in a patient's body as described in detail below. Generally, sheath **8** is inserted into a vessel, such as the transfemoral vessel, passing through the skin of the patient, such that the distal end of the sheath **8** is inserted into the vessel. Sheath **8** can include a hemostasis valve at the opposite, proximal end of the sheath. The delivery apparatus **10** can be inserted into the sheath **8,** and the prosthetic device **12** can then be delivered and implanted within the patient.

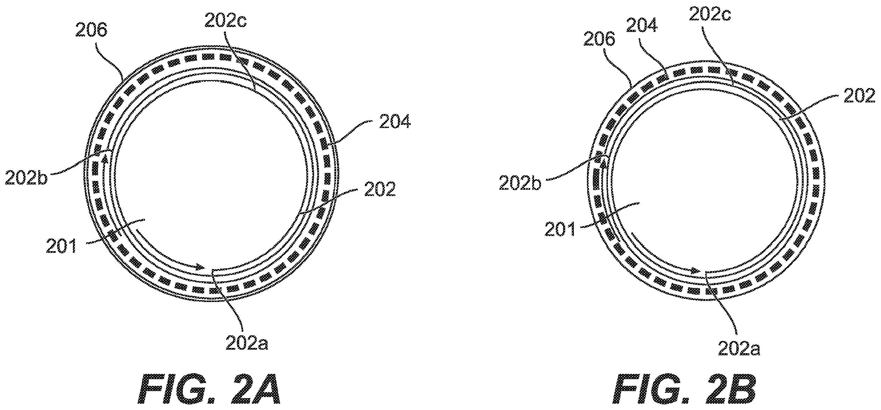

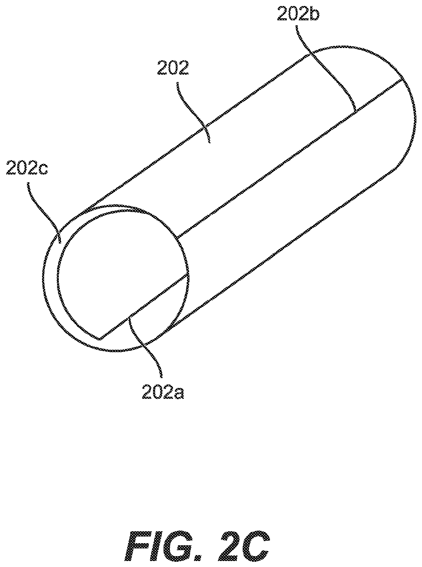

**FIGS. 2A** and **2B** show section views of aspects of two exemplary sheaths disclosed herein for use with a delivery apparatus such as that shown in **FIG. 1****.** **FIG. 2C** shows a perspective view of one aspect of an inner liner **202** for use with the disclosed sheath. As shown in **FIGS. 2A-2C****,** in some aspects, the disclosed sheath comprises an inner liner **202** rolled into a spiral configuration such that at least a portion of the inner surface of the sheet overlays at least a portion of the outer surface of the sheet, forming an overlaying portion **202c,** and wherein the first edge **202a** of the sheet is slidable along at least a portion the inner surface of the sheet and the second edge **202b** is slidable along at least a portion of the outer surface of the sheet. Sheath, as shown in **FIG. 2A** and **FIG. 2B** can further include an outer layer comprising a braid (or coil) **204** and a layer of elastomeric polymer **206.** In one aspect, and as shown in **FIG. 2A****,** the outer layer can comprise the braid (or coil) **204** that is not embedded in the layer of the elastomeric polymer **206.** While in the other aspect, and as shown in **FIG. 2B****,** the outer layer can comprise the braid (or coil) **204** that is embedded in the layer of the elastomeric polymer **206.**

The inner liner **202** defines a lumen **201** through which a delivery apparatus can travel into a patient's vessel in order to deliver, remove, repair, and/or replace a prosthetic device. The disclosed sheath can also be useful for other types of minimally invasive surgery, such as any surgery requiring introduction of an apparatus into a subject's vessel. For example, the disclosed sheath also can be used to introduce other types of delivery apparatus for placing various types of intraluminal devices (*e.g*., stents, stented grafts, etc.) into many types of vascular and non-vascular body lumens (*e.g*., veins, arteries, esophagus, ducts of the biliary tree, intestine, urethra, fallopian tube, other endocrine or exocrine ducts, etc.).

In still further aspects, the sheet used to make the inner liner **202** can comprise high-density polyethylene, polypropylene, polyamide, fluoropolymer, copolymers thereof, or blends thereof. In still further aspects, the sheet can comprise one or more layers. In some aspects, if one or more layers are present, each layer can comprise the same or different polymer. In still further aspects, the sheet can have a predetermined thickness, wherein the predetermined thickness can be defined by one of ordinary skill in the art depending on the specific application. In certain aspects, the predetermined thickness of the inner liner can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner **202** can be varied depending on the desired amount of radial expansion, as well as the strength required.

In still further aspects, the inner surface of the sheet can be at least partially ribbed. In yet further aspects, the sheet can also be lubricious. In some exemplary aspects, the sheet that forms the inner liner can have a coefficient of friction less than about 0.5, less than about 0.4, less than about 0.3, less than about 0.2, less than about 0.1, or less than about 0.05, or even less than about 0.01. It is further understood that the sheet can have a coefficient of friction having any value between any two foregoing values. Such a liner can facilitate passage of a delivery apparatus through the lumen **201** of the disclosed sheath. In some further exemplary aspects, materials that can be used to form suitable lubricious inner liners include materials that can reduce the coefficient of friction of the inner liner **202,** such as PTFE, polyethylene, polyvinylidene fluoride, and combinations thereof. Suitable materials for a lubricious liner also include other materials desirably having a coefficient of friction of about 0.1 or less, of about 0.09 or less, about 0.08 or less, about 0.07 or less, about 0.05 or less, about 0.04 or less, about 0.03 or less, about 0.02 or less, or about 0.01 or less.

In yet further aspects, the outer layer comprising the braid or coil and the layer of the elastomeric polymer can have any predetermined thickness. It is understood that the predetermined thickness of the outer layer can be dependent on the specific application of the sheath. For example, and without limitation, the thicknesses of the inner liner **202** and the outer layer comprising the braid (or coil) **204** and the layer of the elastomeric material **206** can also be varied depending on the particular application of the disclosed sheath. In some aspects, the thickness of the inner liner **202** ranges from about 0.0005 inches to about 0.010 inches, including exemplary values of about 0.0006, about 0.0007, about 0.0008, about 0.0009, about 0.001, about 0.002, about 0.003, about 0.004, about 0.005, about 0.006, about 0.007, about 0.008, about 0.009 inches, and in one particular aspect, the thickness can be about 0.002 inches. The outer layer comprising the braid (or coil) **204** and the layer of the elastomeric material **206** can have a thickness of from about 0.002 inches to about 0.015 inches, including exemplary values of about 0.003, about 0.004, about 0.005, about 0.006, about 0.007, about 0.008, about 0.009, and about 0.01 inches.

It is understood that the inner liner can have any shape or configuration depending on the desired application and the size of the delivery apparatus and prosthetic device. It is further understood that the inner liner is not limited to a specific shape or configuration. In yet further aspects, the outer layer comprising the braid or coil and the layer of the elastomeric polymer can conform to the shape or configuration of the inner liner. In certain aspects, the sheath disclosed herein is defined by the rest diameter *dᵣ,* and the outer diameter *dₒ.* As disclosed herein, the rest diameter *dᵣ* is defined by the inner liner, while the outer diameter can be defined by the inner liner and the outer layer, wherein the outer layer comprises the braid or coil and the layer of the elastomeric polymer.

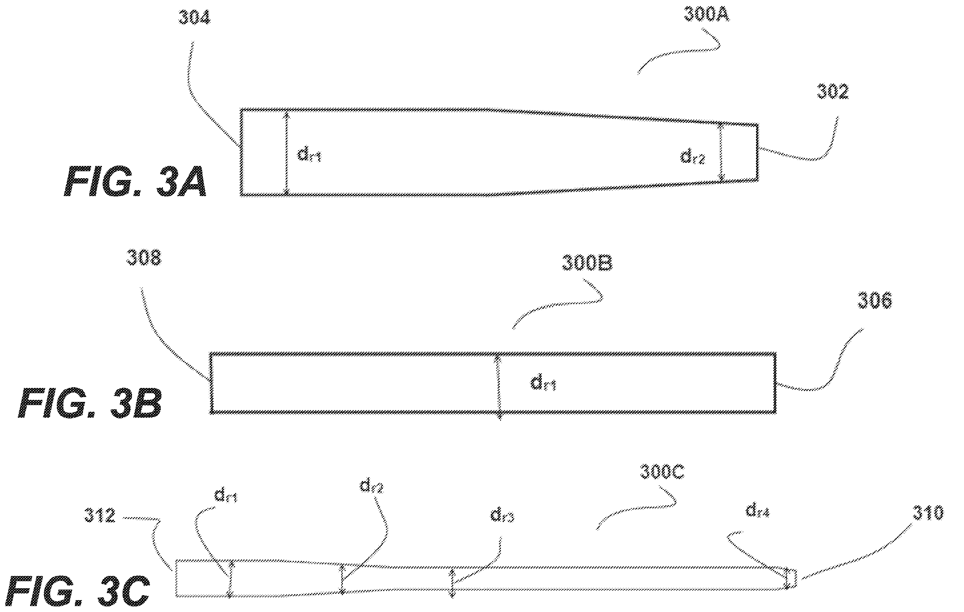

The rest diameter *dᵣ* of the inner liner **202** can vary depending on the application and size of the delivery apparatus and prosthetic device. **FIGS. 3A-3C** show various configurations and shapes of the inner liner. It is understood that in some aspects, and as shown in **FIG. 3B****,** the rest diameter *dᵣ* is substantially uniform along the longitudinal axis of the lumen without changing from the proximal end **308** to the distal end **306.** In yet other aspects, and as shown in **FIGS. 3A** and **3C****,** the rest diameter *dᵣ* can vary along the longitudinal axis (for example, *dᵣ₁* and *dᵣ₂* in **FIG. 3A****,** or *dᵣ₁*, *dᵣ₂*, *dᵣ₃*, and *dᵣ₄*, as shown in **FIG. 3C****)** of the lumen. In certain aspects, rest diameter *dᵣ₁* at the proximal end **304** or **312** is larger than the rest diameter *dᵣ₂*, as shown in **FIG. 3A** or *dᵣ₄*, as shown in **FIG. 3C** at the distal end **302** or **310** *dᵣ.* In yet further aspects, where the outer layer conforms to the shape of the inner liner, the outer diameter *dₒ* (not shown) comprises the overall diameter of the inner liner and the outer layer. In such aspects, the outer diameter *dₒ* is defined by the specific application of the sheath. Similar to the rest diameter *dᵣ*, the outer diameter *dₒ* of the unexpended sheath disclosed herein can be substantially uniform (constant) along the longitudinal axis of the lumen without changing from the proximal end to the distal end (not shown). In alternative aspects, the original unexpanded outer diameter *dₒ* of the disclosed sheath, similarly to the rest diameter *dᵣ*, can decrease from the proximal end to the distal end. In some aspects, and similarly to the rest diameter *dᵣ*, the original unexpanded outer diameter can decrease along a gradient, from the proximal end to the distal end; or it can incrementally step down along the length of the sheath having the largest original unexpanded outer diameter is near *dₒ* the proximal end, and the smallest original unexpanded outer diameter *dₒ* is near the distal end.

In some aspects, the rest diameter *dᵣ* can range from about 0.005 inches to about 0.400 inches, including exemplary values of about 0.01, about 0.02, about 0.03, about 0.04, about 0.05, about 0.06, about 0.07, about 0.08, about 0.09, about 0.1, about 0.2, and about 0.3 inches. As described above, in certain aspects, the sheath can comprise the inner liner having various *dᵣ.* In such aspects, the *dᵣ* can have any value between any two foregoing values and can depend on the specific application and the size and shape of the delivery apparatus and prosthetic device. Different sheaths can be provided with different expanded, and unexpanded rest diameter *dᵣ* and outer diameter *dₒ*, depending on the size requirements of the delivery apparatus for various applications. Additionally, some aspects can provide more or less expansion depending on the particular design parameters, the materials, and/or configurations used.

As disclosed herein, the outer layer of the sheath has an inner surface and an outer surface. The outer layer of the disclosed sheath extends about at least a portion of the variable diameter inner liner such that the inner surface of the outer layer is positioned adjacent to the outer surface of the inner liner. As disclosed herein, the outer layer comprises a braid (or coil) **204** and a layer of an elastomeric polymer **206** having a predetermined thickness and having an inner surface and outer surface (as shown in **FIGS. 2A** and **2B****).** In certain aspects, the braid or coil can be an expandable braid or coil. In yet further aspects, the braid or coil can comprise at least one filament comprising stainless steel, nitinol, a polymer material, or a composite material. In certain unlimiting aspects, the braid or coil comprises filaments comprising Nitinol and/or other shape memory alloys. In yet other unlimiting aspects, the braid can have filaments comprising polyester or nylon. In yet some other exemplary aspects, the braid can comprise filaments comprising spectra fiber, polyethylene fiber, aramid fiber, or combinations thereof.

It is understood that the braid or coil can have any configurations known in the art. In certain aspects, the braid (or coil) **204** is generally a thin, hollow, substantially cylindrical tube comprising an arrangement, pattern, structure, or configuration of filaments or struts, however other geometries can also be used. Suitable filaments can be round, having a diameter less than about 0.015", less than about 0.01", less than about 0.008", less than about 0.005", less than about 0.002", less than about 0.001", less than about 0.0008", or less than about 0.0005". In yet other aspects, suitable filaments can be round and having a diameter ranging from about 0.0005" inches thick to about 0.015" thick, including exemplary values of about 0.0006", about 0.0007", about 0.0008", about 0.0009", about 0.001", about 0.002", about 0.003", about 0.004", about 0.005", about 0.006", about 0.007", about 0.008", about 0.009", about 0.01", about 0.012", about 0.013", and about 0.014". In yet other aspects, the suitable filaments can be flat filaments having a height of less than about 0.006", less than about 0.005", less than about 0.004", less than about 0.003", less than about 0.001", less than about 0.0009", less than about 0.0008", less than about 0.0007", less than about 0.0006", and about 0.0005". In yet other aspects, the flat filaments can have a width from greater than about 0.003" to about 0.015", including exemplary values of about 0.004", about 0.005", about 0.006", about 0.007", about 0.008", about 0.009", about 0.01", about 0.012", about 0.013", and about 0.014". However, other geometries and sizes are also suitable for certain aspects.

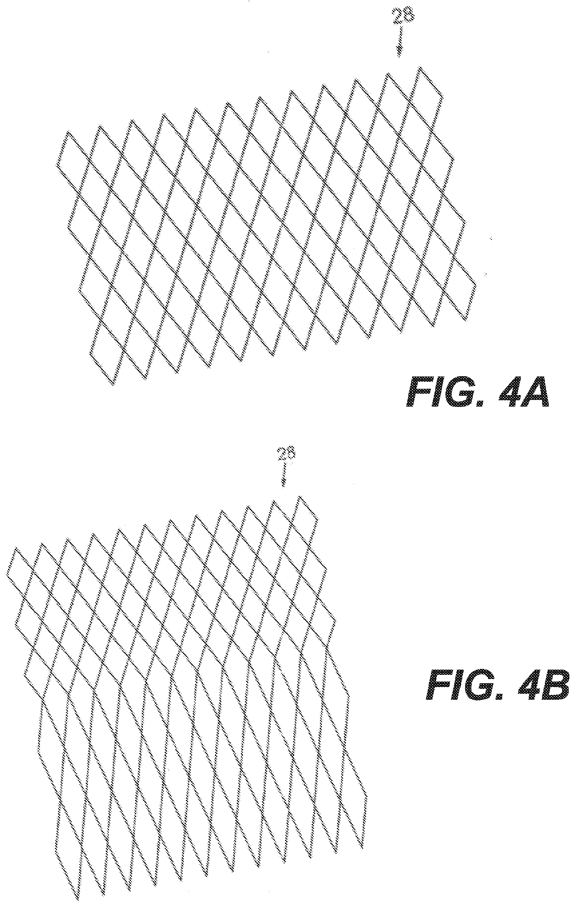

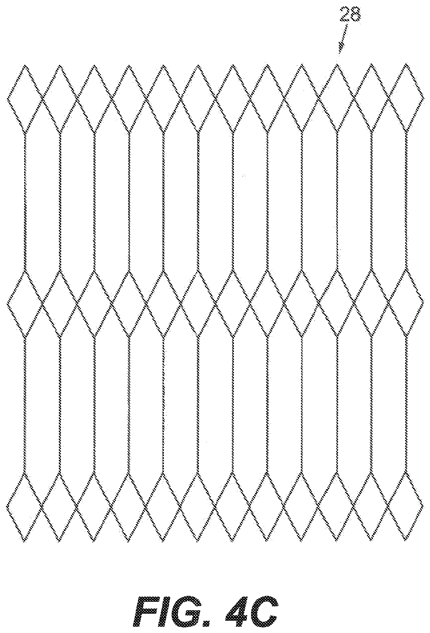

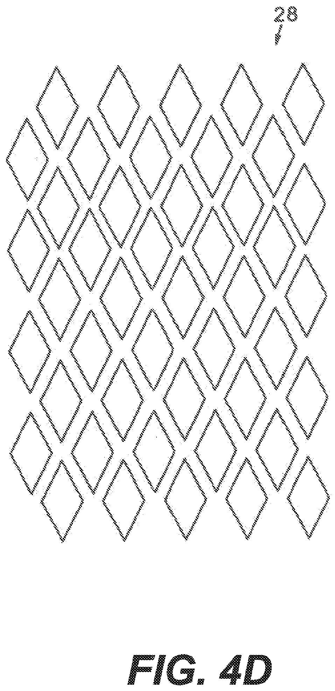

In yet further aspects, the braid can have a per-inch crosses (PIC) count of less than 50, less than 40, less than 30, less than 20. or less than 10. In yet other aspects, the braid can have the PIC count from 10 to 2, including exemplary values of 9, 8, 7, 6, 5, 4, and 3. In still further aspects, the PIC can vary along the longitudinal axis of the lumen. In yet other aspects, the braid pattern can vary along the longitudinal axis of the lumen. In the aspects where the braid or coil comprises filament that is nitinol, the nitinol is a heat-set at the expanded diameter *dₑ.* In yet further aspects, where the filament comprises stainless steel or nitinol, the filament is configured to be atraumatic, at least at the distal end of the sheath. **FIGS. 4A-4D** illustrate partial elevation views of various structures for the braid **28.** It is understood that the structure of the braid **28** can vary from section to section, changing along the length of the sheath. It is further understood that the structures shown in **FIGS. 4A-4D** are not necessarily drawn to scale and show just exemplary and unlimiting aspects. It is further understood that the braid is configured to provide the torquability of the sheath during the insertion of the prosthetic device.

In still further aspects, the outer layer comprises the layer of the elastomeric polymer **206,** as shown in **FIGS. 2A,** and **2B****.** In certain aspects, the elastomeric polymer can comprise a styrene-based elastomer, polyurethane, latex, copolymers thereof, blends thereof, or co-extrudates thereof. In certain and unlimiting aspects, the elastomeric polymer can comprise polyether block ester copolymer, polyesters, polyvinyl chloride, thermoset silicone, poly-isoprene rubbers, polyolefin, other medical grade polymers, or combinations thereof. In yet further aspects, the elastomeric polymer described herein can have any useful additives. In certain aspects, the elastomeric polymers can comprise at least one friction reduction additive. In some exemplary aspects, the friction reduction additives can comprise, for example, BaSO₄, ProPell^{™}, PTFE, any combination thereof, and the like. It is understood that this list of the friction reduction additives is not limiting, and any known in the art friction reduction additives can be utilized.

It is understood that the hardness of each layer of the disclosed sheath can also be varied depending on the particular application and desired properties of the sheath. In some aspects, the layer of the elastomeric polymer **206** has a Shore hardness of less than 90 Durometer, less than 80 Durometer, less than 70 Durometer, less than 60 Durometer, less than 50 Durometer, less than 40 Durometer, less than 30 Durometer, or less than 20 Durometer. In yet further exemplary aspects, the layer of the elastomeric polymer **206** has a Shore hardness from about 25 Durometer to about 75 Durometer, including exemplary values of about 30 Durometer, about 35 Durometer, about 40 Durometer, about 45 Durometer, about 50 Durometer, about 55 Durometer, about 60 Durometer, about 65 Durometer, and about 70 Durometer.

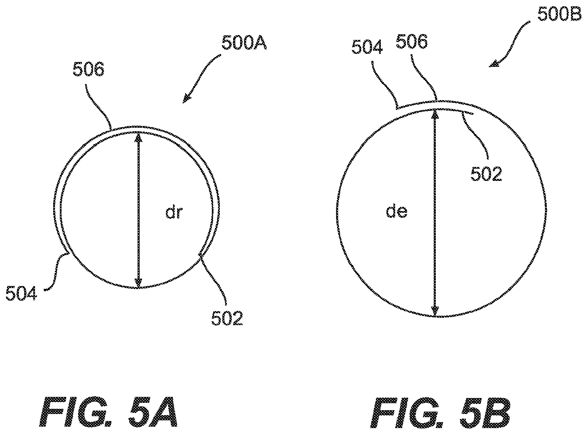

Alternative aspects of a sheath for introducing a prosthetic device are also described. For example, **FIGS. 5A-5B** illustrate a section view of the inner liners **500A** and **500B** of the disclosed sheath in unexpanded and expanded configurations **(****FIGS. 5A** and **5B****,** respectively). Upon introduction of the prosthetic device into the inner liner, the first edge **502** and the second edge **504** slid along and expand the inner liner from the rest diameter *dᵣ* to the expanded diameter *dₑ*, thereby shortening the overlaying portion **506** of the inner liner. It is understood that the expanded diameter *dₑ* is configured to accommodate the medical device passing through the lumen. In yet further aspects, the sheath contracts to the predetermined rest diameter *dᵣ* after passage of the medical device through the lumen.

In certain aspects, an amount of a first lubricant is disposed between at least a portion of the inner liner and at least a portion of the outer layer. In yet other aspects, an amount of a second lubricant is disposed between at least a portion of the overlying portion of the sheet and at least a portion of the sliding portions of the sheet. It is understood that the first lubricant and the second lubricant can be the same or different. In certain and unlimiting aspects, the first and/or second lubricants can comprise Christo Lube supplied by ECL or MED10/6670 supplied by Nusil. In still further aspects, it is understood that the amount of the first and/or second lubricant can be easily determined by one of ordinary skill in the art.

In still further aspects, the outer surface of the layer of the elastomeric polymer defines at least a portion of the outer surface of the outer layer. In yet other aspects, at least a portion of the inner surface of the layer of the elastomeric polymer is at least partially bonded to at least a portion of the outer surface of the sheet of the inner liner. In one aspect, wherein at least a portion of the inner surface of the layer of the elastomeric polymer defines at least a portion of the inner surface of the outer layer. While in the other aspect, at least a portion of the braid defines at least a portion of the inner surface of the outer layer. It is understood that the outer layer of the disclosed sheath is configured to provide hemostasis and prevent bleeding of the patient during the procedure.

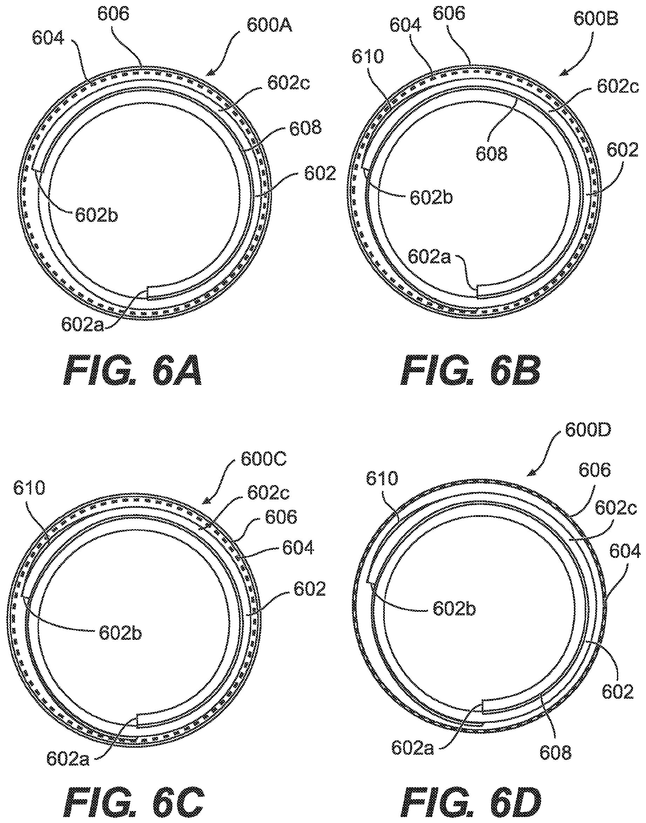

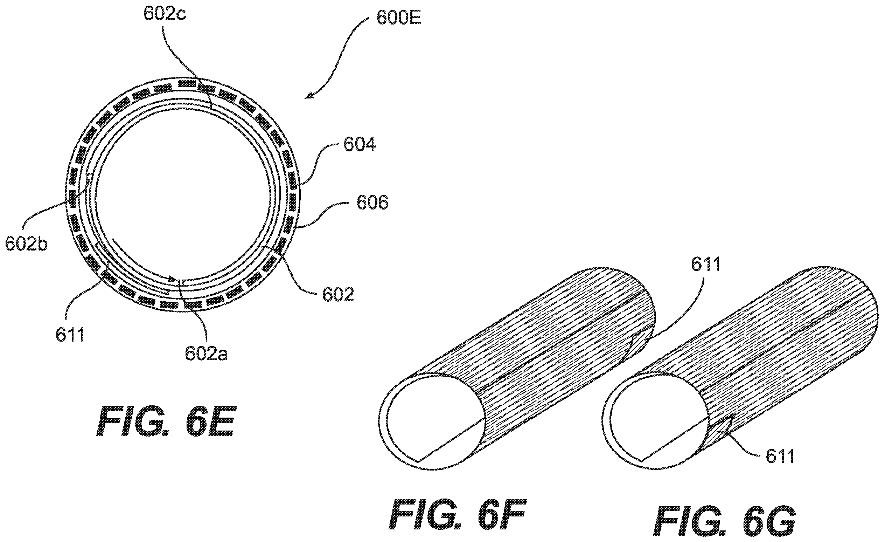

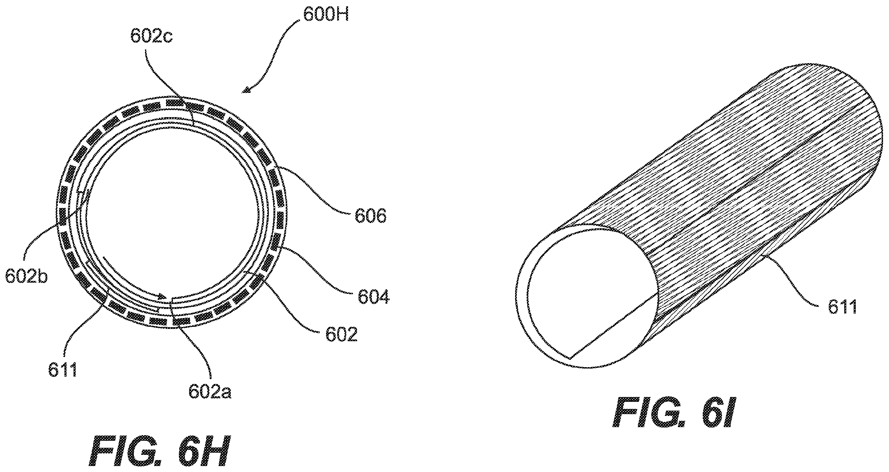

**FIGS. 6A-6D** show other alternative aspects of a sheath for introducing a prosthetic device. **FIG. 6A** shows the sheath **600A** comprising the inner liner **602** having the first edge **602a** and the second edge **602b,** and the overlaying portion **602c,** where the inner and outer surfaces of the inner liner overlay each other. The sheath **600A** further comprises an amount of the second lubricant **608** as disclosed herein that is disposed between the sliding and overlaying portions of the inner sheath. The sheath further comprises the braid **604** and the layer of the elastomeric polymer **606.** In this exemplary aspect, the braid **604** is not embedded in the layer of the elastomeric polymer **606.** **FIG. 6B** depicts an alternative aspect of the sheath **600B** where an amount of the first lubricant **610** is applied between the inner liner and the outer layer comprising the braid **604** and the layer of the elastomeric polymer **606.** An additional aspect of the sheath **600C** is shown in **FIG. 6C****.** In this aspect, the sheath **600C** comprises the inner liner **602,** having the first edge **602a** and the second edge **602b,** and the overlaying portion **602c,** where the inner and outer surfaces of the inner liner overlay each other. The sheath further comprises the braid **604** and the layer of the elastomeric polymer **606** that together form the outer layer of the sheath. The sheath **600C** further comprises an amount of the first lubricant **610,** as disclosed herein, that is disposed between the outer layer and the inner liner of the inner sheath. In this exemplary aspect, the braid **604** is not embedded in the layer of the elastomeric polymer **606.** In the exemplary aspect shown in **FIG. 6D****,** the exemplary sheath **600D** comprises the braid **604** embedded within the layer of the elastomeric polymer **606.**

In still further aspects, the sheath of the instant disclosure can comprise a hemostasis valve inside the lumen of the sheath, at or near the proximal end of the sheath. Additionally, the exemplary sheaths disclosed herein can comprise a soft tip at the distal end of the sheath. Such a soft tip can be provided with a lower hardness than the other portions of the sheath. In some aspects, the soft tip can have a Shore hardness from about 25 D to about 40 D, including exemplary values of about 26 D, about 27 D, about 28 D, about 29 D, about 30 D, about 31 D, about 32 D, about 33 D, about 34 D, about 35 D, about 36 D, about 37 D, about 38 D, and about 39 D. In yet other aspects, the soft tip can have a Shore hardness from about 25 A to about 40 A, including exemplary values of about 26 A, about 27 A, about 28 A, about 29 A, about 30 A, about 31 A, about 32 A, about 33 A, about 34 A, about 35 A, about 36 A, about 37 A, about 38 A, and about 39 A.

In certain aspects, the outer layer and the inner liner can be bonded together or otherwise physically associated with one another. It is understood that the amount of adhesion between the inner liner **602** and the outer polymer layer that comprises braid **604** and the layer of the elastomeric polymer **606** can be variable over the surfaces of the layers. The bonding between the layers can be created by, for example, thermal bonding. In certain aspects, the bonding can be facilitated by the presence of an additional portion of the elastomeric polymer. For example, in certain aspects, the sheath as described herein and as shown in **FIGS. 6H-6I** can further comprise a first strip **611** of the elastomeric polymer disposed along at least a portion of the longitudinal axis of the lumen between at least a portion of the outer surface of the sheet that does not comprise the overlaying portion **602c** of the sheet and the inner surface of the outer layer. In such aspects, the bonding between the outer layer and the inner liner can be facilitated by the first strip of the elastomeric polymer. In yet other aspects, the sheath can further comprise a second strip **611 (****FIGS. 6E-6F****)** of the elastomeric polymer disposed between at least a portion of the outer surface of the sheet at the proximal end of the sheath and the inner surface of the outer layer. In still further aspects, the sheath can further comprise a third strip **611** of the elastomeric polymer disposed between at least a portion of the outer surface of the sheet at the distal end of the sheath and the inner surface of the outer layer **(****FIGS. 6E** and **6G****).** Again, in such aspects, the bonding between the outer layer and the inner liner can be facilitated by the second and/or third strips of the elastomeric polymer.

Applications can utilize a sheath of the present disclosure with the rest diameter *dᵣ* of the lumen formed by the inner liner **602** that is expandable to an expanded diameter *dₑ* from about 3 Fr to about 26 Fr, including exemplary values of about 5 Fr, about 8 Fr, about 10 Fr, about 12 Fr, about 15 Fr, about 18 Fr, about 20 Fr, about 22 Fr, about 25 Fr. The expanded diameter can vary along the length of the disclosed sheath. For example, the expanded outer diameter at the proximal end of the sheath can range from about 3 Fr to about 28 Fr, including exemplary values of about 5 Fr, about 8 Fr, about 10 Fr, about 12 Fr, about 15 Fr, about 18 Fr, about 20 Fr, about 22 Fr, about 25 Fr, while the expanded outer diameter at the distal end of the sheath can range from about 3 Fr to about 25 Fr, including exemplary values of about 8 Fr, about 10 Fr, about 12 Fr, about 15 Fr, about 18 Fr, about 20 Fr, and about 22 Fr. Aspects of the disclosed sheath can expand to an expanded outer diameter that is from about 10% greater than the original unexpanded outer diameter to about 100% greater than the original unexpanded outer diameter, including exemplary values of about 15 % greater, about 20 % greater, about 25 % greater, about 30 % greater, about 35 % greater, about 40 % greater, about 45 % greater, about 50 % greater, about 55 % greater, about 60 % greater, about 65 % greater, about 70 % greater, about 75 % greater, about 80 % greater, about 85 % greater, about 90 % greater, and about 95 % greater than the original unexpanded outer diameter.

It is understood, and as described above, the disclosed sheath can expand from its rest position. The expansion of the disclosed sheath can result in an expansion of the rest diameter *dᵣ* of from about 10% or less to about 430% or more. In certain aspects, expansion of the sheath can result in expansion of the rest diameter *dᵣ* to about 10 % or less, to about 9 % or less, to about 8 % or less, to about 7 % or less, to about 6 % or less, to about 5 % or less, to about 4 % or less, to about 3 % or less, to about 2 % or less, to about 1 % or less. In yet other aspects, expansion of the disclosed sheath can result in expansion of the rest diameter *dᵣ* to about 10 % or more, about 20 % or more, about 30 % or more, about 40 % or more, about 50 % or more, about 60 % or more, about 70 % or more, about 80 % or more, about 90 % or more, about 100 % or more, about 125 % or more, about 150 % or more, about 175 % or more, about 200 % or more, about 225 % or more, or about 250 % or more.

As with previously disclosed aspects, the aspects illustrated in **FIGS. 6A-6D** can be applied to sheaths having a wide variety of rest diameters *dᵣ* and outer diameter *dₒ.* In some aspects, the outer diameter *dₒ* of the sheath gradually decreases from the proximal end of the sheath to the distal end of the sheath. For example, in one aspect, the outer diameter *dₒ* can gradually decrease from about 26 Fr at the proximal end to about 18 Fr at the distal end. The diameter *dₒ* of the sheath can transition gradually across substantially the entire length of the sheath. In other aspects, the transition or reduction of the diameter of the sheath can occur only along a portion of the length of the sheath. For example, the transition can occur along a length from the proximal end to the distal end, where the length can range from about 0.5 inches to about the entire length of the sheath, including any values between any two foregoing values. In yet further aspects, the *dₒ* is minimal and constant along the section of the sheath that passes through the vasculature. In such aspects, the tapered section is about 4" or less at the proximal side of the sheath.

In some aspects, the outer layer comprising the braid and the layer of the elastomeric polymer can comprise the same material or combination of materials along the entire length. In alternative aspects, the material composition of the outer layer can change along the length of the sheath. For example, the outer layer can be provided with one or more segments, where the composition changes from segment to segment. For example, in one segment, the braid can comprise nitinol having a different PIC count than another segment. In yet another exemplary aspect, the layer of the elastomeric material in one segment can be different from the layer of the elastomeric material in another segment. In still further exemplary aspects, one segment of the sheath can comprise the braid or coil embedded within the layer of the elastomeric polymer material, while another segment can comprise the braid or coil that is not embedded within the layer of the elastomeric polymer material. It is understood that the exemplary sheath disclosed herein is not limiting. In certain exemplary aspects, the sheath can comprise an *n* number of segments, wherein each segment can be the same or different. In still further exemplary aspects, the Durometer rating of the composition of the outer layer can also change along the length of the sheath such that segments near the proximal end comprise a stiffer material or combination of materials, while segments near the distal end comprise a softer material or combination of materials. This can allow for a sheath having a relatively stiff proximal end at the point of introducing a delivery apparatus while still having a relatively soft distal tip at the point of entry into the patient's vessel.

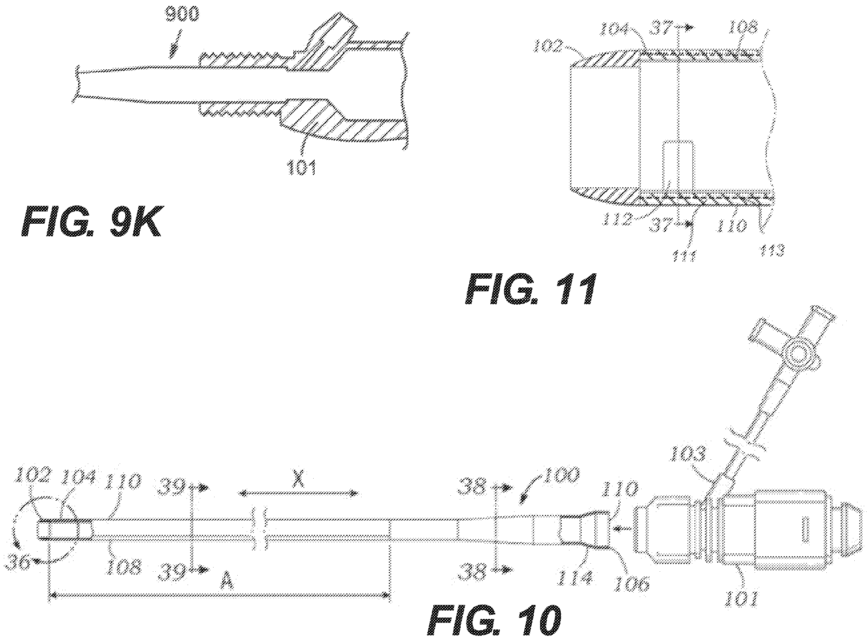

**FIGS. 10** and **11** illustrate an expandable sheath **100** according to the present disclosure, which can be used with a delivery apparatus for delivering a prosthetic device, such as a tissue heart valve, into a patient. In general, the delivery apparatus can include a steerable guide catheter (also referred to as a flex catheter), a balloon catheter extending through the guide catheter *(e.g.,* as depicted in **FIG. 1****).** The guide catheter, the balloon catheter, and the nose catheter can be adapted to slide longitudinally relative to each other to facilitate delivery and positioning of the valve at an implantation site in a patient's body. However, it should be noted that the sheath **100** can be used with any type of elongated delivery apparatus used for implanting balloon-expandable prosthetic valves, self-expanding prosthetic valves, and other prosthetic devices. Generally, sheath **100** can be inserted into a vessel *(e.g.,* the femoral or iliac arteries) by passing through the skin of a patient, such that a soft tip portion **102** at the distal end **104** of the sheath **100** is inserted into the vessel. The sheath **100** can also include a proximal flared end portion **114** to facilitate mating with an introducer housing **101** and catheters mentioned above (*e.g.,* the proximal flared end portion **114** can provide a compression fit over the housing tip and/or the proximal flared end portion **114** can be secured to the housing **101** via a nut or other fastening device or by bonding the proximal end of the sheath to the housing). The introducer housing **101** can house one or more valves that form a seal around the outer surface of the delivery apparatus once inserted through the housing, as known in the art. The delivery apparatus can be inserted into and through the sheath **100,** allowing the prosthetic device to be advanced through the patient's vasculature and implanted within the patient.

In exemplary aspects, the sheath **100** comprises an inner liner **108** and an outer layer **110** disposed around the inner liner **108.** The outer layer **110** comprises the braid (or coil) **111** and the layer of the elastomeric polymer **113.** **FIG. 11** depicts one and unlimiting aspect where the braid (or coil) **111** is embedded in the layer of the elastomeric polymer **113.** The inner liner **108** defines a lumen having the rest diameter *dᵣ* through which a delivery apparatus can travel into a patient's vessel in order to deliver, remove, repair, and/or replace a prosthetic device, moving in a direction along the longitudinal axis X. As the prosthetic device passes through the sheath **100,** the sheath locally expands from the resting diameter *dᵣ* to the expanded diameter *dₑ* to accommodate the prosthetic device. After the prosthetic device passes through a particular location of the sheath **100,** each successive expanded portion or segment of the sheath **100** at least partially returns to the resting diameter *dᵣ.* In this manner, the sheath **100** can be considered self-expanding in that it does not require the use of a balloon, dilator, and/or obturator to expand.

The inner and outer layers **108, 110,** as shown herein, can comprise any materials disclosed above.

Additionally, some aspects of a sheath **100** can include an exterior hydrophilic coating on the outer surface of the outer layer **110.** Such a hydrophilic coating can facilitate insertion of the sheath **100** into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g*., PTFE, polyethylene. polyvinylidene fluoride), are also suitable for use with the sheath **100.**

Best seen in **FIG. 11****,** the soft tip portion **102** can comprise, in some aspects, low-density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 A to about 40 A, including exemplary values of about 28 A, about 30 A, about 32 A, about 35 A, and about 38 A. It is further understood that Shore hardness can have any value between any two foregoing values. In yet other aspects, the soft tip **102** can have a Shore hardness from about 25 D to about 40 D. including exemplary values of about 28 D, about 30 D, about 32 D, about 35 D, and about 38 D. The tip portion **102** is configured to be radially expandable to allow a prosthetic device to pass through the distal opening of the sheath **100.**

As shown in **FIG. 11****,** the sheath **100** can optionally include at least one radiopaque filler or marker, such as a discontinuous or C-shaped band **112** positioned near the distal end **104** of the sheath **100.** The marker **112** can be associated with the inner liner and/or outer layer **108, 110** of the sheath **100.** Such a radiopaque tip marker can comprise materials such as those suitable for the radiopaque filler, platinum, iridium, platinum/iridium alloys, stainless steel, other biocompatible metals, or combinations thereof. Suitable materials for use as a radiopaque filler or marker include, for example, barium sulfite, bismuth trioxide, titanium dioxide, bismuth subcarbonate, or combinations thereof. The radiopaque filler can be mixed with or embedded in the layer of the elastomeric polymer used to form the outer layer and can comprise from about 5% to about 45% by weight of the outer layer, including exemplary values of about 10%, about 15%, about 20%, about 25%, about 30%, about 35%, and about 40% by weight of the outer polymeric tubular layer. The more or less radiopaque material can be used in some aspects, depending on the particular application.

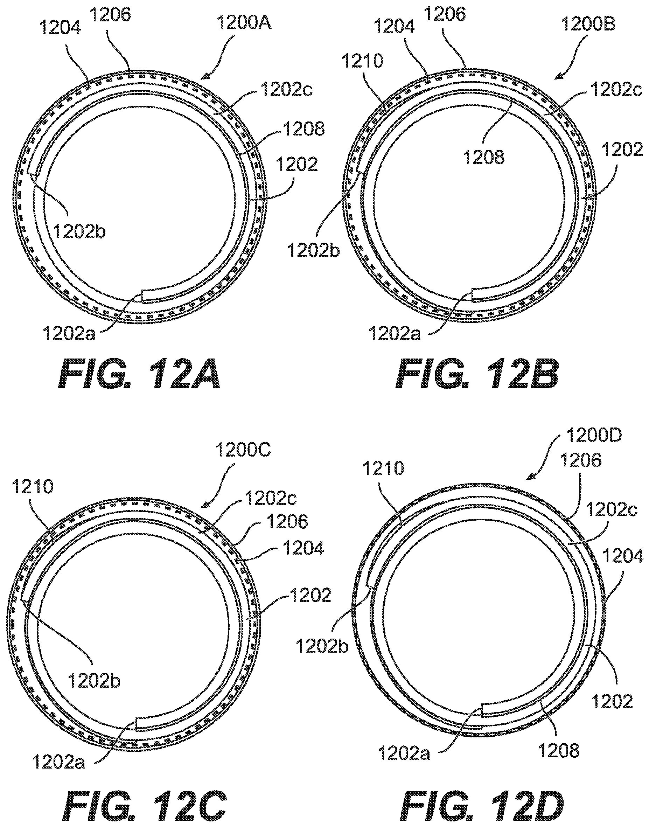

**FIGS. 12A-12B** shows a cross-section view of the sheath **100** taken near the distal end **104** of the sheath **100.**

**FIG. 12A** shows a section view of the exemplary sheath with a lubricant disposed between the sliding and overlaying portions of the sheet and the braid that is not embedded in the elastomeric polymer layer. More specifically, **FIG. 12A** shows the sheath **1200A** comprising the inner liner **1202** having the first edge **1202a** and the second edge **1202b,** and the overlaying portion **1202c,** where the inner and outer surfaces of the inner liner overlay each other. The sheath **1200A** further comprises an amount of the second lubricant **1208** as disclosed herein that is disposed between the sliding and overlaying portions of the inner sheath. The sheath further comprises the braid (or coil) **1204** and the layer of the elastomeric polymer **1206.** In this exemplary aspect, the braid (or coil) **1204** is not embedded in the layer of the elastomeric polymer **1206.**

**FIG. 12B** shows a section view of the exemplary sheath with a lubricant disposed between the sliding and overlaying portions of the sheet and a lubricant disposed between the inner liner and outer layer, where the braid that is not embedded in the elastomeric polymer layer. More specifically, **FIG. 12B** depicts an alternative aspect of the sheath **1200B** where an amount of the first lubricant **1210** is applied between the inner liner and the outer layer comprising the braid (or coil) **1204** and the layer of the elastomeric polymer **1206.**

An additional aspect of the sheath **1200C** is shown in **FIG. 12C. FIG. 12C** shows a section view of the exemplary sheath with a lubricant disposed between the inner liner and outer layer, where the braid that is not embedded in the elastomeric polymer layer; with and without the lubricant. More specifically, in this aspect, the sheath **1200C** comprises the inner liner **1202,** having the first edge **1202a** and the second edge **1202b,** and the overlaying portion **1202c,** where the inner and outer surfaces of the inner liner overlay each other. The sheath further comprises the braid (or coil) **1204** and the layer of the elastomeric polymer **1206** that together form the outer layer of the sheath. The sheath **1200C** further comprises an amount of the first lubricant **1210,** as disclosed herein, that is disposed between the outer layer and the inner liner of the inner sheath. In this exemplary aspect, the braid (or coil) **1204** is not embedded in the layer of the elastomeric polymer **1206.** **FIG. 12D** shows a section view of the exemplary sheath with a lubricant disposed between the sliding and overlaying portions of the sheet and a lubricant disposed between the inner liner and outer layer, where the braid is at least partially embedded in the elastomeric polymer layer. More specifically, In this exemplary aspect shown in **FIG. 12D****,** the exemplary sheath **1200D** comprises the braid (or coil) **1204** embedded within the layer of the elastomeric polymer **1206.**

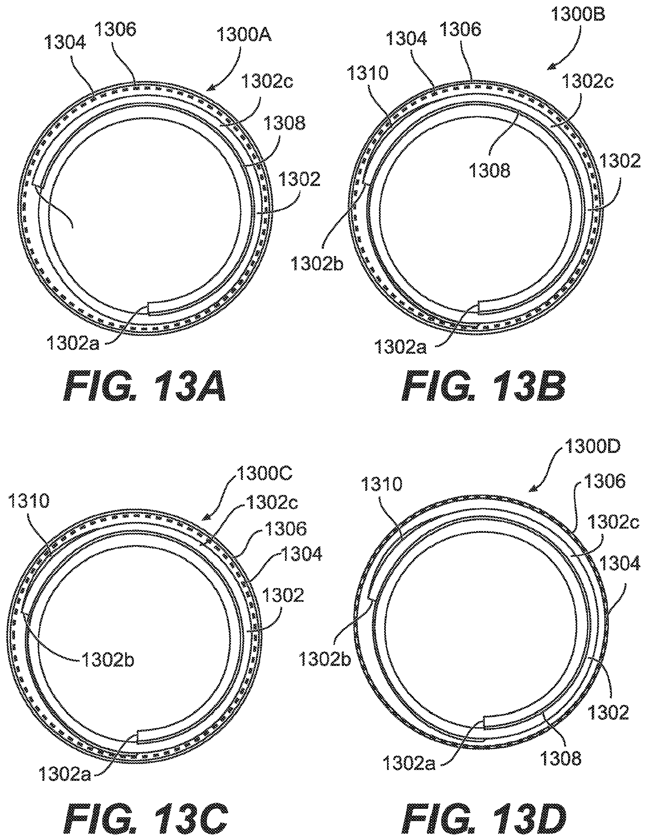

**FIGS. 13A-D** show a section view of a proximal section of the sheath of **FIGURE 10****,** taken along line 38-38.

**FIGURE 13A** shows a section view of the exemplary sheath with a lubricant disposed between the sliding and overlaying portions of the sheet and the braid that is not embedded in the elastomeric polymer layer. More specifically, **FIG. 13A** shows the sheath **1300A** comprising the inner liner **1302** having the first edge **1302a** and the second edge **1302b,** and the overlaying portion **1302c,** where the inner and outer surfaces of the inner liner overlay each other. The sheath **1300A** further comprises an amount of the second lubricant **1308** as disclosed herein that is disposed between the sliding and overlaying portions of the inner sheath. The sheath further comprises the braid (or coil) **1304** and the layer of the elastomeric polymer **1306.** In this exemplary aspect, the braid (or coil) **1304** is not embedded in the layer of the elastomeric polymer **1306.**

**FIG. 13B** shows a section view of the exemplary sheath with a lubricant disposed between the sliding and overlaying portions of the sheet and a lubricant disposed between the inner liner and outer layer, where the braid that is not embedded in the elastomeric polymer layer. More specifically, **FIG. 13B** depicts an alternative aspect of the sheath **1300B** where an amount of the first lubricant **1310** is applied between the inner liner and the outer layer comprising the braid (or coil) **1304** and the layer of the elastomeric polymer **1306.** An additional aspect of the sheath **1300C** is shown in **FIG. 13C. FIG. 13C** shows a section view of the exemplary sheath with a lubricant disposed between the inner liner and outer layer, where the braid that is not embedded in the elastomeric polymer layer; with and without the lubricant. More specifically, in this aspect, the sheath **1300C** comprises the inner liner **1302,** having the first edge **1302a** and the second edge **1302b,** and the overlaying portion **1302c,** where the inner and outer surfaces of the inner liner overlay each other. The sheath further comprises the braid (or coil) **1304** and the layer of the elastomeric polymer **1306** that together form the outer layer of the sheath. The sheath **1300C** further comprises an amount of the first lubricant **1310,** as disclosed herein, that is disposed between the outer layer and the inner liner of the inner sheath. In this exemplary aspect, the braid (or coil) **1304** is not embedded in the layer of the elastomeric polymer **1306.**

**FIG. 13D** shows a section view of the exemplary sheath with a lubricant disposed between the sliding and overlaying portions of the sheet and a lubricant disposed between the inner liner and outer layer, where the braid is at least partially embedded in the elastomeric polymer layer. In this exemplary aspect, the sheath **1300D** comprises the braid (or coil) **1304** embedded within the layer of the elastomeric polymer **1306.**

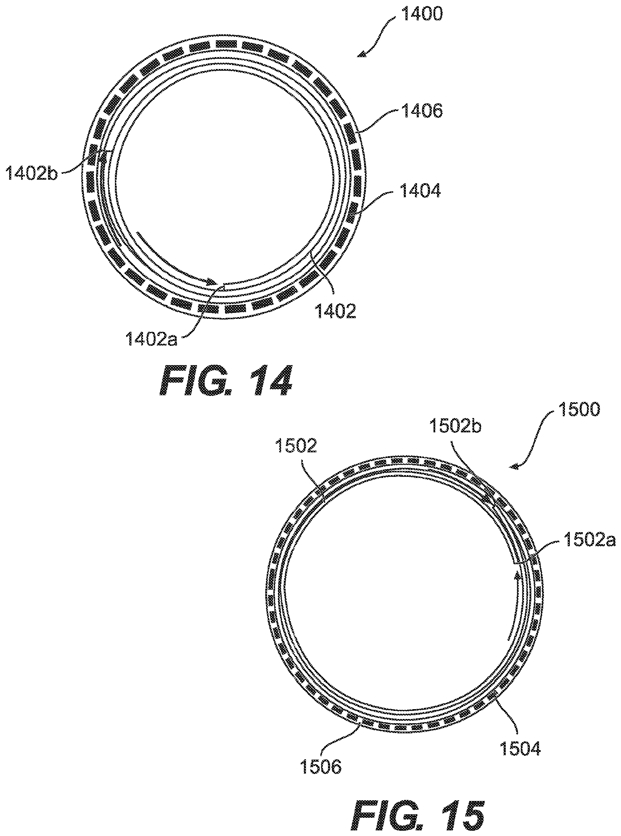

In yet further aspects, as shown in **FIG. 14****,** the sheath **1400,** whether with the braid or coil embedded within the layer of the elastomeric polymer (as shown in **FIG. 14****)** or with the braid or coil that is not embedded within the layer of the elastomeric polymer is configured to expand from a resting configuration to an expanded configuration shown in **FIG. 15****.** In such aspects, the first and the second edges **(1502a** and **1502b)** of the inner liner slide such that a length of the overlaying portion shortens. In some exemplary aspects, this movement can be facilitated by the presence of the first and/or second lubricant, as disclosed above.

The disclosed herein sheath can be configured such that it locally expands at a particular location corresponding to the location of the medical device along the length of the lumen and then locally contracts once the medical device has passed that particular location. Thus, a bulge may be visible, traveling longitudinally along the length of the sheath as a medical device is introduced through the sheath, representing continuous local expansion and contraction as the device travels the length of the sheath. In some aspects, each segment of the sheath can locally contract after removal of any radial outward (insertion) force such that it regains the original resting diameter of lumen *dᵣ.*

In some aspects, each segment of the sheath can locally contract after removal of any radial outward force such that it at least partially returns to the original resting diameter of lumen *dᵣ.*

Additional sheath **8** configurations that can be utilized with the delivery apparatus **10,** as shown in **FIG. 1** for delivery of a prosthetic device **12** are further disclosed.

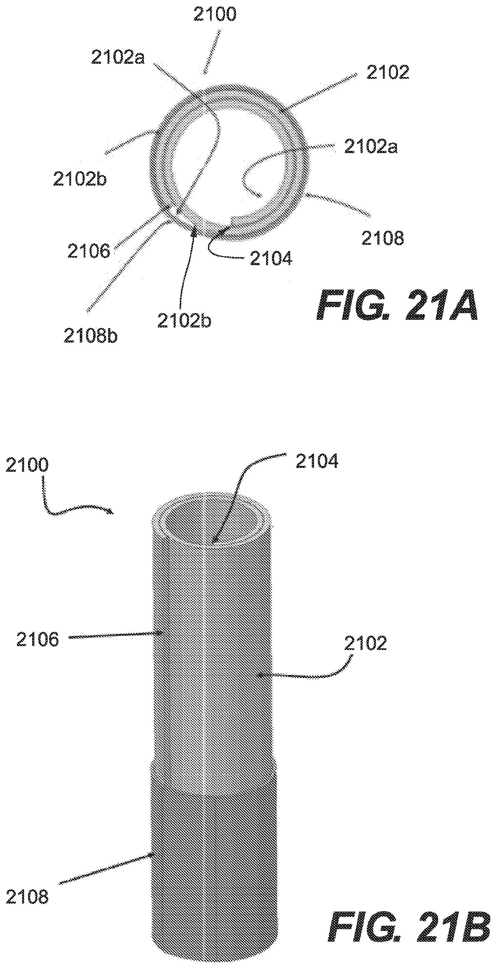

For example, and without limitations, **FIGS. 21A-21B** show one exemplary aspect of the disclosed herein sheath. In such aspects, the sheath comprises a proximal end and a distal end. The sheath **2100** can comprise a variable diameter inner liner **2102** comprising a sheet having a first edge **2104** and a second edge **2106** and is defined by an inner surface **2102a** and an outer surface **2102b.** When the sheet is wound in a spiral configuration, at least a portion of the inner surface **2102a** of the sheet overlays at least a portion of the outer surface **2102b** of the sheet. As can be seen in **FIGS. 21A-B**the first edge **2104** of the sheet is slidable along at least a portion of the inner surface **2102a** of the sheet, and the second **2106** edge is slidable along at least a portion of the outer surface **2102b** of the sheet. The sheath further comprises an outer layer **2108,** having an inner surface **2108a** and an outer surface **2108b.**

The inner surface **2102a** of the sheet further defines a lumen of the sheath through which a delivery apparatus can travel into a patient's vessel in order to deliver, remove, repair, and/or replace a prosthetic device. The disclosed sheath can also be useful for other types of minimally invasive surgery, such as any surgery requiring introduction of an apparatus into a subject's vessel. For example, the disclosed sheath also can be used to introduce other types of delivery apparatus for placing various types of intraluminal devices (*e.g.,* stents, stented grafts, etc.) into many types of vascular and non-vascular body lumens (*e.g.,* veins, arteries, esophagus, ducts of the biliary tree, intestine, urethra, fallopian tube, other endocrine or exocrine ducts, etc.).

It is further understood that the sheath can also comprise additional layers. Some of these additional layers are disclosed in detail below or above. For example, as disclosed in some exemplary aspects above, the sheath can also comprise a braid or coil disposed between the inner liner and the outer layer and/or a braid or coil that is embedded in the outer layer.

In exemplary aspects shown in **FIGS. 21A-21B****,** the sheath does not comprise a braid or coil disposed along a length of the sheath between the inner liner and outer layer or being embedded in the outer layer.

Similar to other aspects of the sheath, an exemplary sheath of **FIGS. 21A-21B** can have an inner liner having various depending on the desired application and the size of the delivery apparatus and prosthetic device. It is further understood that the inner liner is not limited to a specific shape or configuration. In certain aspects, the sheath disclosed herein is defined by the rest diameter *dᵣ,* and the outer diameter *dₒ.* As disclosed herein, the rest diameter *dᵣ* is defined by the inner liner, while the outer diameter can be defined by the inner liner and the outer layer.

The rest diameter *dᵣ* of the inner liner **2102** can vary depending on the application and size of the delivery apparatus and prosthetic device. The sheath disclosed herein can have configurations similar to the configurations depicted in **FIGS. 3A-3C** and described above. It is understood that in some aspects, and as shown in **FIG. 3B****,** the rest diameter *dᵣ₁* is substantially uniform along the longitudinal axis of the lumen without changing from the proximal end **308** to the distal end **306.** In yet other aspects, and as shown in **FIGS. 3A** and **3C****,** the rest diameter *dᵣ* can vary along the longitudinal axis (for example, *dᵣ₁* and *dᵣ₂* in **FIG. 3A****,** or *dᵣ₁*, *dᵣ₂*, *dᵣ₃*, and *dᵣ₄*, as shown in **FIG. 3C****)** of the lumen. In certain aspects, rest diameter *dᵣ₁* at the proximal end **304** or **312** is larger than the rest diameter *dᵣ₂,* as shown in **FIG. 3A** or *dᵣ₄*, as shown in **FIG. 3C** at the distal end **302** or **310** *dᵣ.* In yet further aspects, where the outer layer conforms to the shape of the inner liner, the outer diameter *dₒ* (not shown) comprises the overall diameter of the inner liner and the outer layer. In such aspects, the outer diameter *dₒ* is defined by the specific application of the sheath. Similar to the rest diameter *dᵣ*, the outer diameter *dₒ* of the unexpended sheath disclosed herein can be substantially uniform (constant) along the longitudinal axis of the lumen without changing from the proximal end to the distal end. In alternative aspects, the original unexpanded outer diameter *dₒ* of the disclosed sheath, similarly to the rest diameter *dᵣ*, can decrease from the proximal end to the distal end. In some aspects, and similarly to the rest diameter *dᵣ*, the original unexpanded outer diameter can decrease along a gradient, from the proximal end to the distal end; or it can incrementally step down along the length of the sheath having the largest original unexpanded outer diameter is near *dₒ* the proximal end, and the smallest original unexpanded outer diameter *dₒ* is near the distal end.

In some aspects, and similarly to other sheath configurations disclosed herein, the rest diameter *dᵣ* of the sheath as shown in **FIGS. 21A-21B** can also range from about 0.005 inches to about 0.400 inches, including exemplary values of about 0.01, about 0.02, about 0.03, about 0.04, about 0.05, about 0.06, about 0.07, about 0.08, about 0.09, about 0.1, about 0.2, and about 0.3 inches. As described above, in certain aspects, the sheath can comprise the inner liner having various *dᵣ.* In such aspects, the *dᵣ* can have any value between any two foregoing values and can depend on the specific application and the size and shape of the delivery apparatus and prosthetic device. Different sheaths can be provided with different expanded, and unexpanded rest diameter *dᵣ* and outer diameter *dₒ*, depending on the size requirements of the delivery apparatus for various applications. Additionally, some aspects can provide more or less expansion depending on the particular design parameters, the materials, and/or configurations used.

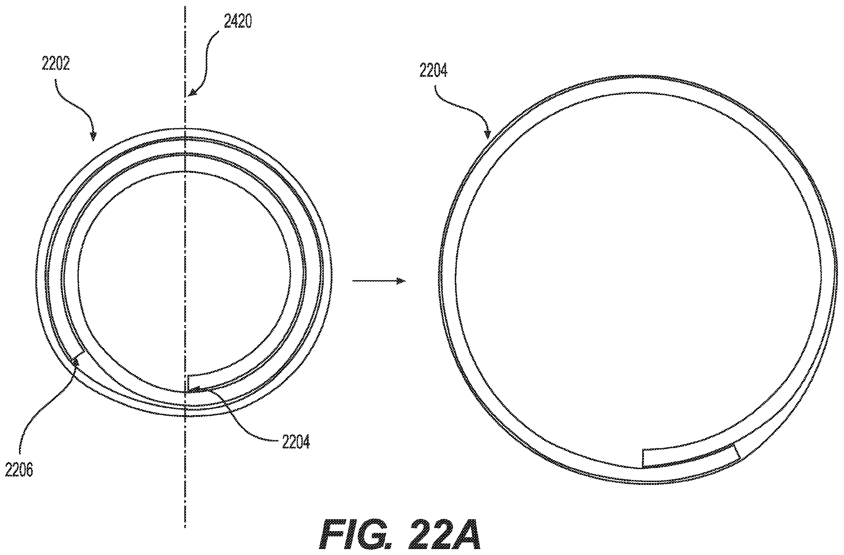

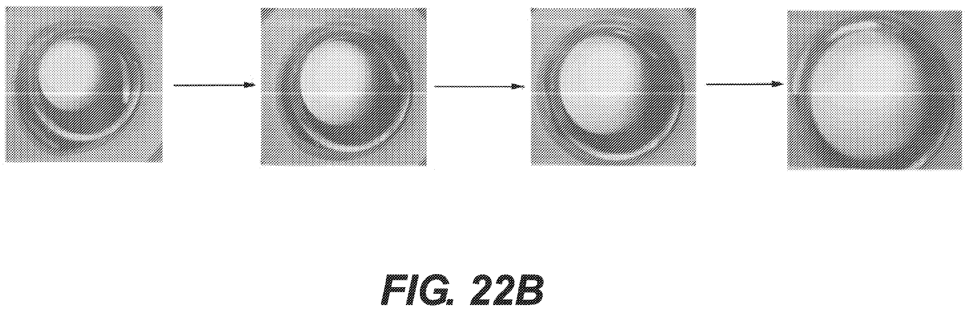

**FIGS. 22A-22B** depict expansion process of an exemplary sheath, as shown, for example, in **FIGS. 21-21B****.** The sheath **2202,** as shown in **FIG. 22A** can expand from the collapsed configuration to expanded configuration **2204** during the passage of the medical device by sliding the first and the second longitudinal edges along each other and decreasing the overlapping portion of the spiral configuration. Again, the expanded diameter *dₑ* can be dependent on a diameter of a medical device passing through. In still further aspects and as discussed in detail below, the outer layer is configured to impart an inward radial force on the inner liner to contract the sheath to a diameter that is substantially identical to *dᵣ* after the medical device passed through the lumen. It is further understood that any of the sheath configurations described herein can locally expand and collapse upon passage of the medical device. **FIG. 22B** shows images of sequential moments of the sheath expansion during the passage of the exemplary medical device.

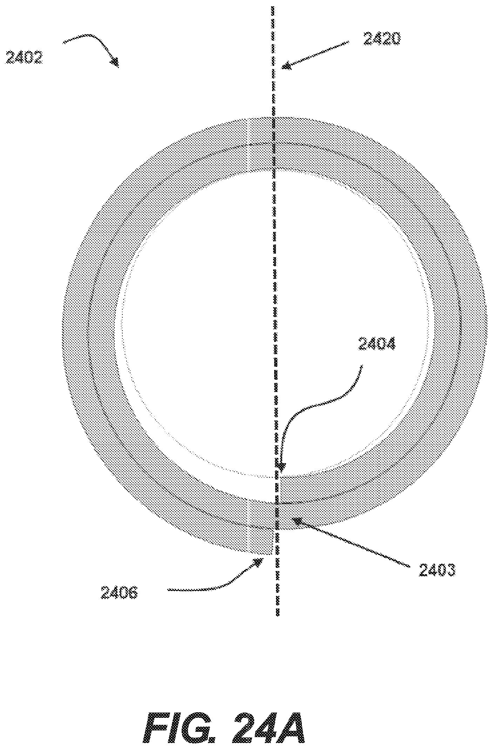

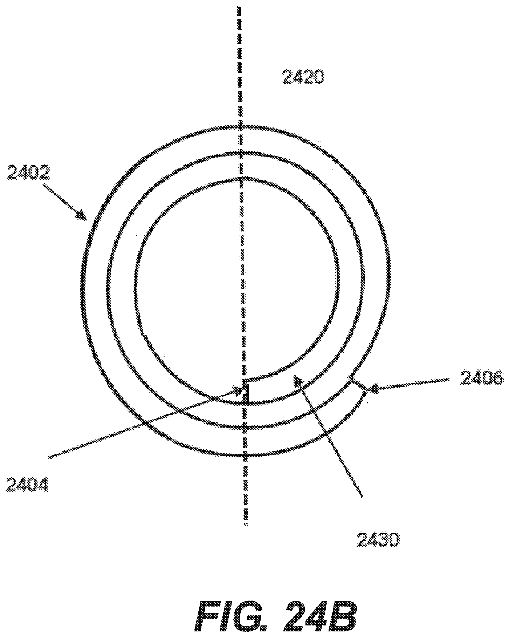

Additional aspects of the sheath are disclosed in **FIGS. 24A-24B****.** For example, **FIG. 24** **A** shows the inner liner of the sheath having a configuration similar to the sheath disclosed in **FIG. 22****,** where the first end **2404** and the second **2406** are substantially aligned in a spaced relationship along a vertical axis **2420** passing through a thickness of the sheath. In such a configuration, a portion of the sheet **2403** is positioned between the first edge and the second edge along the vertical axis. As it can be seen in **FIG. 24A****,** when the inner liner **2402** is in an unexpanded rest state, the inner liner comprises at least two layers of the sheet overlaying each other along at least a portion of a circumference of the sheath. Yet, and as seen in **FIG.24****,** the inner liner can comprise at least two layers of the sheet overlaying each other along a whole circumference of the sheath.

**FIG. 24B** shows a different configuration of the liner in an expanded state. For example, when liner **2402** is in an unexpanded rest state, the inner liner can comprise a portion along a circumference of the sheath that can have three layers of the sheet **2430.**

Referring to **FIG. 22A****,** for example, there are also configurations of the inner liner, where the first edge **2204** is substantially aligned with a vertical axis **2420** passing through a thickness of the sheath and the second edge **2406** circumferentially offset from the vertical axis. In such an aspect, along at least a portion of a circumference of the sheath, the inner liner comprises one layer of the sheet without any overlaying portion.

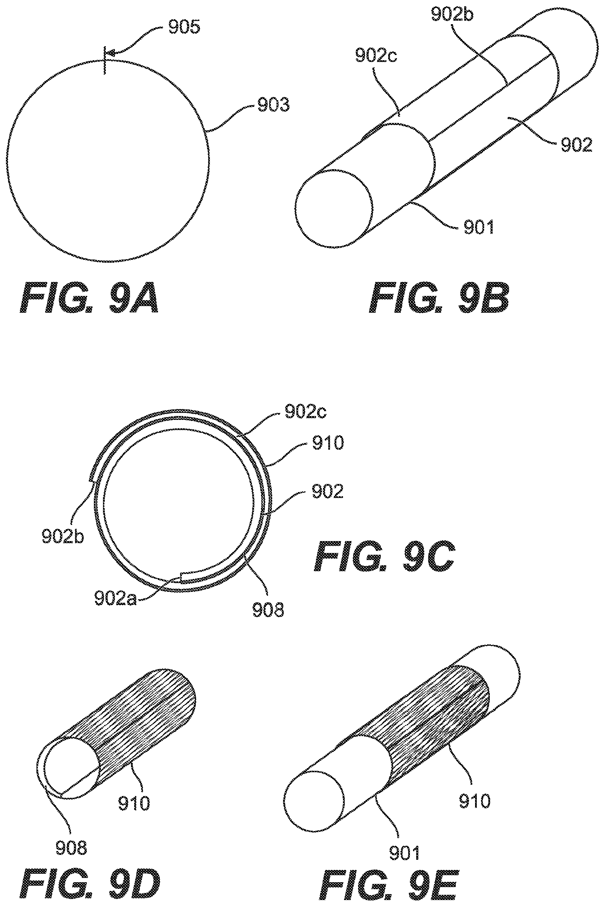

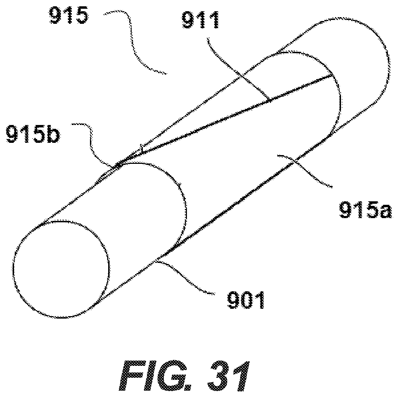

Additional configurations of the sheath are also shown in **FIGS. 31-32****.** Some of the sheath configurations disclosed above are formed by providing an elongated tubing and cutting the tubing along its length to form a sheet having a first longitudinal edge and a second longitudinal edge. The sheet is formed by forming a slit. In various configurations, the specific type of slit can be formed depending on the final desired application. For example, for various sheath configurations disclosed above, the slit **905,** as shown in **FIG. 9A****,** for example, is substantially straight along the length of the tubing forming the inner liner. In such exemplary aspects, the slit formed during the cut is substantially straight from the proximal end of the inner liner to the distal end of the inner liner. In such aspects, the spiral configuration formed when the sheet is wound into the scroll is substantially straight along the length of the sheath.

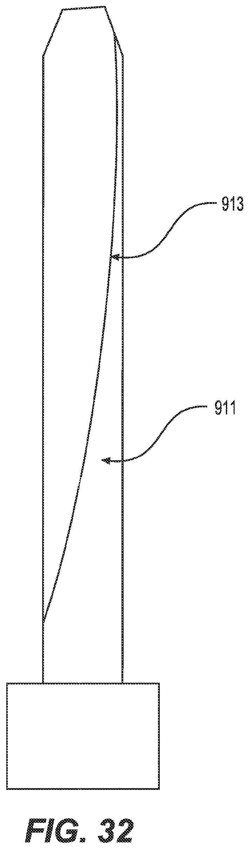

However, disclosed herein are also aspects where the inner liner is wound in helical scroll configuration. An exemplary view of such configuration is shown in **FIG. 32****.** In such aspects, the elongated tube that will form the inner liner **915** is provided and cut **(****FIG. 31****)** such that a longitudinal slit **911** is formed between the proximal and distal ends of the tubing, forming the first longitudinal edge and a second longitudinal edge of the tubular inner liner **915.** As shown in **FIG. 31****,** for example, the slit in these aspects is not formed along the longitudinal axis of the liner but with at least some offset from it. The slit is formed such that the inner liner is wound in a helical scroll configuration, such that in the helical scroll configuration, at least a portion of an inner surface of the inner liner **915b** helically overlays at least a portion of an outer surface **915a** of the inner liner (the overlap is not shown in **FIG. 31****).** The overlapping portion **913** can be seen in **FIG. 32****.** In still further aspects, in this helical sheath configuration, the first longitudinal edge of the inner liner is slidable along at least a portion of the inner surface of the inner liner, and the second longitudinal edge is slidable along at least a portion of the outer surface of the inner liner.

In such exemplary aspects, the tubular inner liner is also configured to expand from a rest diameter *dᵣ* to an expanded diameter *dₑ* by helically sliding the first edge of the inner liner along at least a portion of the inner surface and helically sliding the second edge of the inner liner along the at least a portion of the outer surface of the inner liner during application of a radial outward force by passage of a medical device through the lumen of the inner liner.

When the sheath is inserted into the patient, for example, through the femoral artery, and is passed along the arterial path, which can often be a twisting and tortuous path, forcing the sheath to assume a bent configuration there-along. In addition, when a larger-diameter prosthetic valve (or any other prosthetic device that can be advanced through the sheath) passes through the sheath, the combination of the expansion forces with the bent configuration of the sheath can result in layers of the sheath potentially splitting the longitudinal edges that can cause the formation of an undesired gap between the two of longitudinal edges. The sheath having a helical configuration can avoid this potentially undesirable effect. In some aspects, the exemplary helical configuration of the sheath can allow expansion of the inner liner with substantially no gap formed between the longitudinal edges of the inner liner defined by the slit. Even further in these exemplary aspects, the tubular inner liner of the disclosed sheath is configured to bend while passing through a patient's natural anatomy also without forming a gap between the first longitudinal edge and the second longitudinal edge of the tubular inner liner.

It is understood that the split can be formed in any orientation that would allow the helical configuration of the scroll. For example, and without limitations, in some aspects, the longitudinal slit can extend from the proximal end of the tubular inner liner to the distal end of the tubular inner liner in a direction offset from a longitudinal axis of the tubular inner liner. In yet a further aspect, the direction is diagonal from the proximal end of the tubular inner liner to the distal end of the tubular inner. In yet still further aspects, the longitudinal slit extends from the proximal end of the tubular inner liner at an angle greater than about 90 degrees, for example, about 100 degrees, about 110 degrees, about 120 degrees, about 130 degrees, about 140 degrees, about 150 degrees, about 160 degrees, and about 170 degrees, or less than about 90 degrees, for example, about 80 degrees, about 70 degrees, about 60 degrees, about 50 degrees, about 40 degrees, about 30 degrees, about 20 degrees, and about 10 degrees across a length of the tubular inner liner to the distal end of the tubular inner.

In still further aspects, the longitudinal slit can be formed between a proximal end of the tubular inner liner and a distal end of the tubular inner in a pattern that is different from a straight line. For example, and without limitations, the slit can be formed such that a specific pattern of the sheet can be formed. For example, in such aspects, the first and the second longitudinal edges have substantially not straight lines. In some aspects, the slit can be formed such that each of the first and the second longitudinal edges can have a zig-zag form or can have angles that are greater or less than 180 degrees or 0 degrees. For example, each of the edges can have a protruding and corresponding recessed shape, or concave and corresponding convex shape, curved shape, and the like, depending on the orientation of the slit. Again, it is understood that any orientation of the slit that can form a helical scroll configuration of the inner liner is contemplated and described.

In still further aspects, the slit can be formed such that the helical configuration of the inner liner has a predetermined pitch. In certain aspects, the pitch can be at least about 2, at least about 3, at least about 4, at least about 5, or at least about 6 revolutions over every 10 cm of the sheath.

In still further aspects, the rest diameter dᵣ and the expanded diameter dₑ of this exemplary sheath can be similar to anyone described in the previous aspects.

In still further aspects, and similarly to other sheath configurations, the tubular inner liner having the helical scroll configuration can comprise a high density polyethylene, polypropylene, polyamide, fluoropolymer, copolymers thereof, or blends thereof. In further aspects, and as disclosed in the other configurations, the inner liner can have a multilayer structure. In some aspects, the internal surface of the inner liner can be substantially smooth. While in other aspects, the internal surface of the tubular inner liner can be at least partially ribbed. In yet still further aspects, the tubular inner liner is lubricious and can have a coefficient of friction less than about 0.5, less than about 0.4, less than about 0.3, less than about 0.2, less than about 0.1.

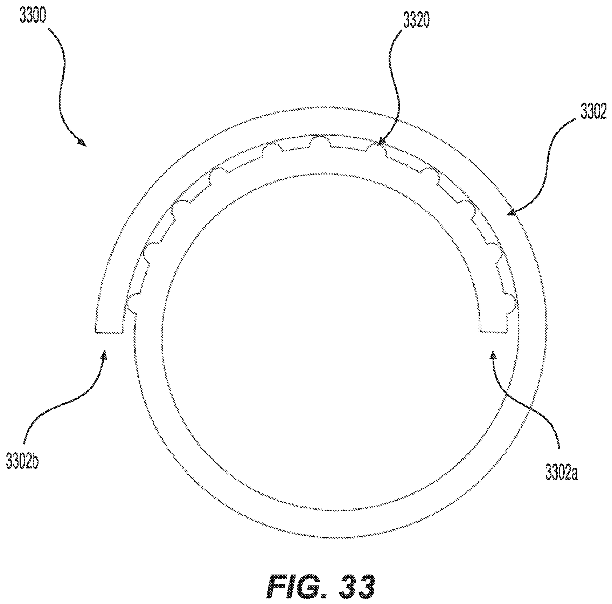

Additional configuration of the sheath is also shown in **FIG. 33****.** In this aspect, a variable diameter inner liner **3300** comprises a sheet **3302** having a first longitudinal edge **3302a** and a second longitudinal edge **3302b.** The sheet is wound in a spiral configuration, as shown in **FIG. 33****,** such that at least a portion of the inner surface of the sheet overlays at least a portion of the outer surface of the sheet forming an overlapping portion; wherein at least a portion of the outer surface of the sheet abutting the first longitudinal edge **3302a** comprises a first plurality of protrusions **3320.** In still further aspects, the at least a portion of the first plurality of protrusions is disposed within the overlapping portion, thereby reducing a contact area between the inner surface and the outer surface of the sheet within the overlapping portion.

In still further aspects, in the disclosed spital configuration of the inner liner, the first longitudinal edge **3302a** of the sheet is slidable along at least a portion the inner surface of the sheet, and the second longitudinal edge **3302b** is slidable along at least a portion of the outer surface of the sheet.

It is understood, however, that the plurality of protrusions can be present on the inner surface of the sheet in addition or in alternative to the outer surface. In such aspects (not shown), a second plurality of protrusions **3320** can also be disposed on the inner surface at at least abut the second longitudinal edge **3302b.** It is also understood that the first and the second plurality of protrusions can be the same or different. In such exemplary aspects, for example, the composition of the first and second plurality of protrusions can be the same while the shape is different. Other exemplary aspects are also envisioned that comprise the first and second plurality of protrusions that are different both in the composition and the shape or both different in the composition and the shape.

In still further aspects, the inner surface of the sheet defines a lumen of the sheath having a longitudinal axis.

In yet still further aspects, the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In still further aspects, the sheet is rolled to form a spiral with no section having no more than three predetermined thicknesses in the radial direction and a small portion having only one predetermined thickness in the radial direction. In certain aspects, when the medical device is passed through the expandable variable diameter inner liner, the applied radial forces are high, and any friction or stickiness between the sliding portions undesirable increase the push force.

In still further aspects, the sheet has a predetermined thickness. In such aspects, the sheet can have any thickness from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner of any of the disclosed herein configurations can be varied depending on the desired amount of radial expansion, as well as the strength required.

In still further aspects, the first and/or second plurality of protrusions can be disposed in any possible arrangement or pattern suitable for the desired application on the inner and/or outer surface of the sheet. In some aspects, the first and/or second plurality of protrusions can be disposed in a predetermined pattern to reduce the contact area between the inner surface and the outer surface of the sheet within the overlapping portion. It is understood that the reduction in the contact can be useful during the expansion procedures, as the two portions do not stick to each other and thus reduce the push force required to pass the medical device through the sheath.

In still further aspects, the first and/or second plurality of protrusions can have any shape that allows achieving the desired result. In certain aspects, the first and/or second plurality of protrusions can comprise a regular shape, for example, continuous stripes along the length of the inner liner, or discontinuous patterns such as discontinuous circular shape protrusions, rectangular, rhombic, trapezoid, and the like. In yet other aspects, the shape can be irregular, for example, star shape or any one other shape. In yet other aspects, the plurality of protrusions can have a combination of various shapes.

In still further aspects, the first and/or second plurality of protrusions can have an average height up to about 1 %, about 5 %, about 10 %, about 15 %, or up to about 20 % of the predetermined thickness of the sheet itself. It is understood that in some aspects, there is no requirement for each of the first and/or second plurality of protrusions to have the same height. In yet other aspects, the first and/or second plurality of protrusions can have a substantially identical height.

In still further aspects, the sheet comprises a first polymer composition. In such aspects, the first polymer composition can comprise high density polyethylene, polypropylene, polyamide, fluoropolymer, copolymers thereof, or blends thereof. While in still further aspects, the sheet can comprise a multilayer structure. In aspects where more than one layer is present, each layer can comprise the same material or different material.

In still further aspects, the first and/or second plurality of protrusions can comprise a second polymer composition. Again, it is understood that the second polymer composition can be the same or different from the first polymer composition depending on the specific application. Again, it is understood that the first and/or second plurality of protrusion can be disposed on the outer surface and/or inner surface of the sheet, respectively. In the aspects when the first and second plurality of protrusions are present, the second plurality of the protrusions present on the inner surface can be the same or different from the first plurality of protrusions present on the outer surface of the sheet. It is understood that in the aspects where the second polymer composition is different from the first polymer composition, these two compositions are compatible with each other and capable of bonding together.

In still further aspects, it is understood that the portion of the sheet that comprises the first and/or second plurality of protrusions is not limited to the overlapping portion.

In some aspects, at least a portion of the sheet that has the first and/or second plurality of protrusions can be greater than the overlapping portion. In still further aspects, the at least a portion having the first and/or second plurality of protrusions is substantially identical to a circumference of the sheet in the spiral configuration. It is understood that in such aspects, the first and/or second plurality of protrusions are disposed over the entire outer and/or inner surface of the sheet, respectively.

In yet other aspects, the first and/or second plurality of protrusions can be disposed on the outer surface and/or inner surface of the sheet, respectively, along at least a portion of a length of the sheath. While in other aspects, the first and/or second plurality of protrusions can be disposed on the outer surface and/or inner surface of the sheet, respectively, abut the distal end of the sheath. In still further aspects, the first and/or second plurality of protrusions can be disposed on the outer surface and/or inner surface of the sheet, respectively, abut the proximal end of the sheath. While in still further aspects, the first and/or second plurality of protrusions can be disposed on the outer surface and/or inner surface of the sheet, respectively, along a full length of the sheath.

It is further understood that in some exemplary aspects, the first and/or second plurality of protrusions can have various shapes as disclosed above, and those shapes can be continuous along at least a portion of the length of the inner member or they can be fully discontinuous. The size of each of the first and/or second plurality of protrusions can vary depending on the desired application. In still further aspects, the average size of the first and/or second plurality of protrusions can be less than about 50 %, less than about 40 %, less than about 30 %, less than about 20 %, less than about 10 %, or even less than about 5 % of the predetermined thickness of the sheet.

Similar to other sheath configurations, in this sheath configuration, the rest diameter *dᵣ* can be substantially uniform along a longitudinal axis of the lumen, or it can vary along a longitudinal axis of the lumen.

In some aspects, the internal surface of the inner liner can be substantially smooth. While in other aspects, the internal surface of the tubular inner liner can be at least partially ribbed. In yet still further aspects, the tubular inner liner is lubricious and can have a coefficient of friction less than about 0.5, less than about 0.4, less than about 0.3, less than about 0.2, less than about 0.1.

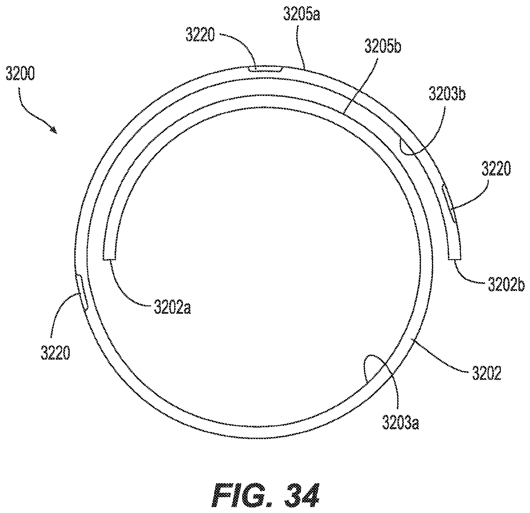

Additional configuration of the sheath is also shown in FIG. 34. In this aspect, a variable diameter inner liner 3400 comprises a sheet 3402 having a first longitudinal edge 3402a and a second longitudinal edge 3402b. The sheath inner liner 3400 has an inner surface 3403a and an outer surface 3405a. The sheet is wound in a spiral configuration, as shown in **FIG. 34****,** such that at least a portion of the inner surface **3403b** of the sheet overlays at least a portion of the outer surface **3405b** of the sheet forming an overlapping portion. As further shown in **FIG. 34****,** at least a portion of the outer surface **3405a** of the sheet can comprise a plurality of bonding sites **3420** that are at least partially embedded within the sheet. As exemplified in **FIG. 34****,** these plurality of bonding sites **3420** are disposed such that the outer surface **3405b** of the sheet in the overlapping portion is substantially free of these bonding sites.

In still further aspects, in the disclosed spital configuration of the inner liner, the first longitudinal edge **3402a** of the sheet is slidable along at least a portion the inner surface of the sheet, and the second longitudinal edge **3402b** is slidable along at least a portion of the outer surface of the sheet.

In still further aspects, the sheet has a predetermined thickness. In such aspects, the sheet can have any thickness from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner of any of the disclosed herein configurations can be varied depending on the desired amount of radial expansion, as well as the strength required. In such aspects, the plurality of bonding sites as shown in the exemplary sheath of **FIG. 34** can have a depth (the depth of the bonding sites embedded within the sheet itself) of no more than about 50% of any of the predetermined values of the sheet thickness disclosed above. For example, the depth of the plurality of bonding sites can be no more than about 50 %, no more than about 45 %, no more than about 40 %, no more than about 35 %, no more than about 30 %, no more than about 25 %, no more than about 20 %, no more than about 15 %, no more than about 15 %, no more than about 10 %, or no more than about 5 % of the disclosed above predetermined thickness.

In yet further aspects, the bonding sites are disposed at the outer surface and are not embedded or only partially embedded within the sheet. In such aspects, the plurality of bonding sites can also at least partially extend from the outer surface of the sheet. In such exemplary aspects, the extended portion of the plurality of bonding sites can have a height of no more than about 50 %, no more than about 45 %, no more than about 40 %, no more than about 35 %, no more than about 30 %, no more than about 25 %, no more than about 20 %, no more than about 15 %, no more than about 15 %, no more than about 10 %, or no more than about 5 % of the disclosed above predetermined thickness. In still further aspects, where the plurality of bonding sites are both extend from the outer surface of the sheet and at least partially embedded within the sheet, the height and the depth of the plurality of bonding sites is no more than about 50 %, no more than about 45 %, no more than about 40 %, no more than about 35 %, no more than about 30 %, no more than about 25 %, no more than about 20 %, no more than about 15 %, no more than about 15 %, no more than about 10 %, or no more than about 5 % of the disclosed above predetermined thickness.

In still further aspects, the plurality of bonding sites can have an average depth up to about 1 %, up to about 5 %, up to about 10 %, up to about 15 %, up to about 20 %, up to about 25%, up to about 30%, up to about 35 %. up to about 40 %, up to about 45 %, or up to about 50 % of the predetermined thickness of the sheet itself. In still further aspects, the plurality of bonding sites can have an average depth and an average height if at least a portion of such bonding sites extends above the outer surface up to up to about 1 %, up to about 5 %, up to about 10 %, up to about 15 %, up to about 20 %, up to about 25%, up to about 30%, up to about 35 %, up to about 40 %, up to about 45 %, or up to about 50 % of the predetermined thickness of the sheet itself.

In aspects where the plurality of bonding sites both embedded in the sheet and extend above the outer surface, the depth and the height of such bonding sites can be the same or different depending on the specific application. In still further aspects, the depth and/or height of each of the bonding sites in the plurality of bonding sites can be the same or different as well.

In still further aspects, the sheet comprises a first polymer composition. In such aspects, the first polymer composition can comprise high density polyethylene, polypropylene, polyamide, fluoropolymer, copolymers thereof, or blends thereof. While in still further aspects, the sheet can comprise a multilayer structure. In aspects where more than one layer is present, each layer can comprise the same material or different.

In still further aspects, the plurality of bonding sites can comprise a second polymer composition. In such aspects, the second polymer composition is different from the first polymer composition. It is understood, however, that the first and second polymer compositions can also be substantially identical if needed and depending on the specific application. In still further aspects, the second polymer composition comprises a polyethylene, a polypropylene, a graft modified polyethylene or polypropylene, or a combination thereof. In some exemplary aspects, the second polymer composition can comprise grafted low-density polyethylene (LDPE), grafted medium density polyethylene, grafted ultra-low-density polyethylene (ULDPE), grafted high density polyethylene (HDPE), grafted heterogeneously branched linear low-density polyethylene (LLDPE), grafted homogeneously branched linear ethylene polymers and substantially linear ethylene polymers, grafted polypropylene, or ethylene-vinyl acetate (EVA), or any combination thereof.

For example and without limitations, the sheet can comprise HDPE, while bonding sites can comprise LDPE, or a terpolymer such as maleic anhydride modified polyolefin, for example, and without limitation, Orevac^{®} (commercially available from Arkema), ethylene acrylic acid copolymers, such as DOW Chemical Primacor^{®}, ethylene acrylate copolymers such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymers such as Lotader^{®} or Orevac^{®} (commercially available from Arkema), or a combination thereof.

In still further aspects, the first polymer composition and the second polymer composition can be coextruded together to form the inner liner. It is understood that in the aspects where the second polymer composition is different from the first polymer composition, these two compositions are compatible with each other and do not cause delamination.

In still further aspects, that plurality of bonding sites can be disposed along at least a portion of a length of the inner liner. In still further aspects, the plurality of bonding sites can be disposed along all the length of the inner liner. While in other aspects, the plurality of bonding sites can be disposed on the outer surface (that is not in the overlapping portion) of the sheet abut the distal end of the sheath. In still further aspects, the plurality of bonding sites can be disposed on the outer surface (that is not in the overlapping portion) of the sheet abut the proximal end of the sheath.

In the aspects, the plurality of bonding sites are disposed in a predetermined pattern allowing coupling with the outer layer and preventing an axial movement of the outer layer during passage of the medical device through the lumen of the inner liner. In still further aspects, the pattern can be any pattern that is desired for the specific application and allowing bonding with the outer layer.

In some aspects, the plurality of bonding sites are disposed in a predetermined pattern to bind the inner liner to the outer layer without compromising the expansion of the inner liner upon the passage of the medical device. It is understood that since the expansion can be achieved by sliding the first and the second longitudinal edges and decreasing the overlapping portion, no bonding sites are disposed within the overlapping portion to avoid undesirable binding and restrictions in the sliding.

In still further aspects, the plurality of bonding sites can have any desired shape. For example, and without limitations, the plurality of bonding sites can have a regular shape, irregular shape, or any combination thereof. In certain aspects, the plurality of bonding sites can comprise a regular shape, for example, continuous stripes along the length of the inner liner, or discontinuous shapes such as discontinuous circular shape, rectangular, rhombic, trapezoid shape, and the like. Again, it is understood that the plurality of bonding sites can be at least partially embedded within the sheet, or fully embedded within the sheet, or at least partially extend above the outer surface of the sheet. In any of these aspects, the shape of each of the plurality of bonding sites can be the same or different, or it can be any variation of the shapes.

In still further aspects, each of the plurality of bonding sites has a width of no more than about 50 %, no more than about 45 %, no more than about 40 %, no more than about 35 %, no more than about 30 %, no more than about 25 %, no more than about 20 %, no more than about 15 %, no more than about 15 %, no more than about 10 %, or no more than about 5 % of the disclosed above predetermined thickness. While in still other aspects, each of the plurality of bonding sites has a width up to about 1 %, up to about 5 %, up to about 10 %, up to about 15 %, up to about 20 %, up to about 25%, up to about 30%, up to about 35 %, up to about 40 %, up to about 45 %, or up to about 50 % of the disclosed above predetermined thickness.

In yet other aspects, each of the plurality of bonding sites has a width of about 1X to about 10X of the thickness of the plurality of bonding sites, including exemplary values of about 2X, about 3X, about 4X, about 5X, about 6X, about 7X, about 8X, and about 9X of the thickness of the plurality of bonding sites.

In still further aspects, each of the plurality of bonding sites has a width from about 0.01" to about 0.15", including exemplary values of about 0.015", about 0.02", about 0.025", about 0.03", about 0.035", about 0.04", about 0.045", about 0.05", about 0.055", about 0.06", about 0.065", about 0.07", about 0.075", about 0.08", about 0.085", about 0.09", about 0.095", about 0.1", about 0.11", about 0.12", about 0.13", and about 0.14".

In still further aspects, the plurality of bonding sites can comprise one bonding site. While in other aspects, the plurality of bonding sites comprise at least two bonding sites. It is understood that the number of the bonding sites can be specifically chosen depending on the desired application. It is further understood that each of the plurality bonding sites can be disposed at a predetermined distance from each other, where this predetermined distance can be chosen depending on the desired application. In still further aspects, the number and location of the bonding sites can be chosen to allow for a section of the outer jacket to expand as the inner member expands but supply adequate anchoring strength to substantially prevent axial movement of the outer jacket (elastomeric outer component) relative to the inner member during insertion and withdraw of the sheath into the body and upon the passing of the medical device.

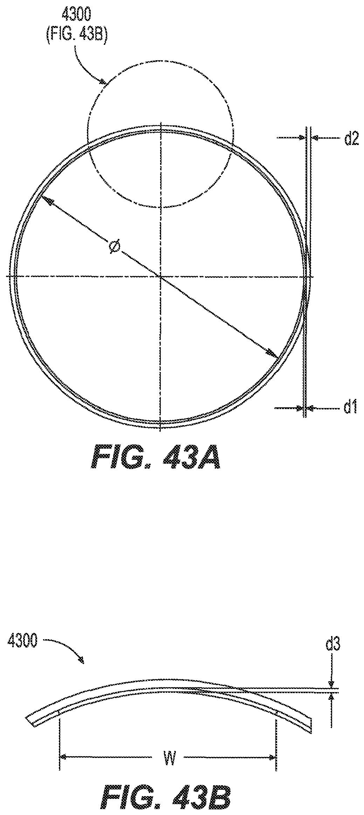

In still further aspects, instead of being disposed on the outer surface of the inner layer (liner) of the sheath, any of the disclosed plurality of bonding sites can be disposed on an inner surface of the outer layer of the sheath. In such exemplary aspects, as shown for example, in **FIGS.43A-43B****,** the inner liner does not comprise any bonding sites, while the inner surface of the outer layer can comprise one or more bonding sites. For example, the inner liner in such an aspect can have any thickness as disclosed above. The outer layer, as disclosed below, can also have a predetermined thickness. For example, the predetermined thickness of the outer layer can vary along a length of the sheath. While in other aspects, the predetermined thickness of the outer layer is the same along a length of the sheath. Yet, in further aspects, the predetermined thickness of the outer layer is greater at the proximal end. In still further aspects, the predetermined thickness of the outer layer is up to 0.015", for example, and without limitation from about 0.001" to about 0.015", including exemplary values of about 0.0015", about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009", 0.0095", about 0.01", about 0.0105", about 0.011", about 0.01105", about 0.012", about 0.01205", about 0.013", about 0.01305", about 0.014", and about 0.01405.

The close-up 4300 of the bonding sites is shown in **FIG. 43B****.** It can be seen that the outer layer can have one or more bonding sites having a width w. In such aspects, the width can have any value from about 0.01" to about 0.15", including exemplary values of about 0.015", about 0.02", about 0.025", about 0.03", 0.035", about 0.04", 0.045", about 0.05", about 0.055", about 0.06", about 0.065", about 0.07", about 0.075", about 0.08", about 0.085", about 0.09", about 0.095", about 0.1", about 0.11", about 0.12", about 0.13", and about 0.14".

In yet still further aspects, the bonding site can have a thickness having any value from about 0.0001" to about 0.005", including exemplary values of about 0.0002", about 0.0003", about 0.0004", about 0.0005", about 0.0006", about 0.0007", about 0.0008", about 0.0009", about 0.001", about 0.0015", about 0.002", about 0.0025", about 0.003", about 0.0035", about 0.004", and about 0.0045".

Any of the disclosed herein inner liners, outer layers, and materials used to form the bonding sites can be utilized without limitations. It is further understood that the plurality of bonding sites disposed on the inner surface of the outer layer (outer jacket) can have the same shape as the plurality of bonding sites disposed on the outer surface of the inner liner as disclosed above. Any of the characteristics or features of the bonding sites disposed on the outer surface of the inner liner are applicable to the plurality of bonding sites disposed on the inner surface of the outer jacket.

In still further aspects, the inner surface of the sheet defines a lumen of the sheath having a longitudinal axis.

In yet still further aspects, the variable diameter inner liner is configured to expand from a first rest diameter *dᵣ* to a second expanded diameter *dₑ* by sliding the first edge of the sheet along at least a portion of the inner surface and sliding the second edge of the sheet along the at least a portion of the outer surface, during application of a radial outward force by passage of a medical device through the lumen of the inner liner.

In still further aspects, the sheet is rolled to form a spiral with no section having no more than three predetermined thicknesses in the radial direction and a small portion having only one predetermined thickness in the radial direction. In certain aspects, when the medical device is passed through the expandable variable diameter inner liner, the applied radial forces are high, and any friction or stickiness between the sliding portions undesirable increase the push force.

Similar to other sheath configurations, in this sheath configuration, the rest diameter *dᵣ* can be substantially uniform along a longitudinal axis of the lumen, or it can vary along a longitudinal axis of the lumen.

In some aspects, the internal surface of the inner liner can be substantially smooth. While in other aspects, the internal surface of the tubular inner liner can be at least partially ribbed. In yet still further aspects, the tubular inner liner is lubricious and can have a coefficient of friction less than about 0.5, less than about 0.4, less than about 0.3, less than about 0.2, less than about 0.1.

In yet still, further aspects, sheaths with the inner liner examples disclosed herein can further comprise any of the disclosed herein outer layers, braids, tie layers, lubricants, and the like.

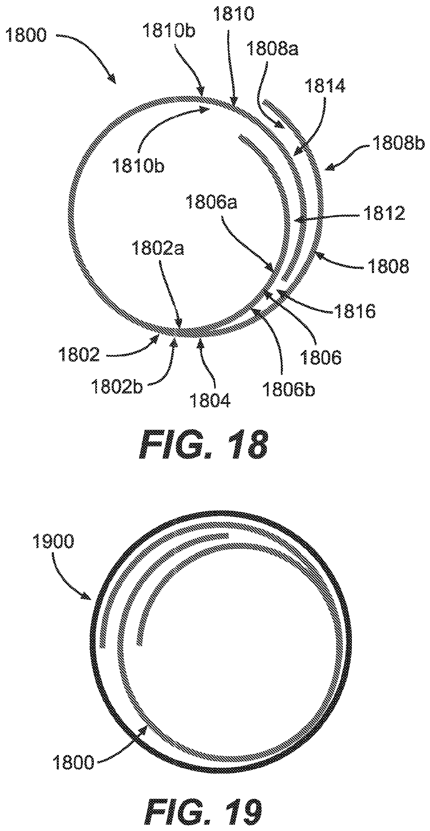

An additional configuration of the inner liner is also depicted in FIG. 18. FIG. 18 shows an inner liner **1800** comprising a sheet rolled into a spiral configuration wherein the sheet comprises a first portion **1802** having a first surface **1802a** and an opposite second surface **1802,** wherein a first end **1804** of the first portion **1802** splits into a first segment **1806** having a first surface **1806a** and an opposite second surface **1806b** and a third segment **1808** having a first surface **1808a** and an opposite second surface **1808b,** and wherein a second end of the first portion extends into a second segment **1810** having a first surface **1810a** and an opposite second surface **1810b.**

As it can be seen in **FIG. 18****,** in a spiral configuration, at least a portion of the first surface **1810a** of the second segment **1810** overlaps at least a portion of the second surface **1806b** of the first segment **1806,** wherein at least a portion of the first surface **1808a** of the third segment **1808** overlaps at least a portion of the second surface **1810b** of the second segment **1810,** and wherein at least a portion of the first surface **1808a** of the third segment overlaps at least a portion of the second surface **1806b** of the first segment **1806.** It can also be seen that wherein the first surface **1804a** of the first portion **1804** extends into the first surface **1806a** of the first segment **1806,** the first surface **1810a** of the second segment **1810,** and the first surface **1808a** of the third segment **1808.** It can also be seen that the second surface **1804b** of the first portion **1804** extends into the second surface **1810b** of the second segment **1810** and into the second surface **1808b** of the third segment **1808.** Each of these segments can slide along each other during the passage of the medical device and expand the sheath.

**FIG. 19** shows an exemplary sheath having an inner liner **1800,** as depicted in **FIG.18** and an outer layer **1900.**

Referring back to **FIG. 18****,** when the at least a portion of the first surface **1810a** of the second segment **1810** overlaps the at least a portion of the second surface **1806b** of the first segment **1806,** a first gap **1812** is formed between the at least a portion of the first surface **1810a** of the second segment **1810** and the at least a portion of the second surface **1804b** of the first segment **1806.**

Further, a second gap **1814** can be formed when the at least a portion of the first surface **1808a** of the third segment **1808** overlaps the at least a portion of the second surface **1810b** of the second segment **1810.** Still, further, a third gap **1816** can be formed when the at least a portion of the first surface **1808a** of the third segment **1808** overlaps the at least a portion of the second surface **1806b** of the first segment **1806.**

It is understood that the first gap can have a substantially uniform width along overlapping portions, or its width can vary. Similarly, the second gap has a substantially uniform width along overlapping portions, or the width of the second gap can vary along overlapping portions. Still, further, the third gap can have a substantially uniform or a variable width along overlapping portions. In still further aspects, the width between the gaps can be any width as desired. It is understood, for example, that width of each of the three gaps can be the same, or it can be different. In certain aspects, the width of some of the gaps is the same, while the width of the others is different.

When the sheath as disclosed in **FIGS. 18** and **19** is utilized, upon passage of the medical device through the lumen, the first segment **1806,** the second segment **1810,** and the third segment **1808** are configured to slidably move along each other such that an overlapping portion between the first segment and the second segment and between the second segment and the third segment decreases, while an overlapping portion between the first segment and the third segment increases.

Similar to any of the disclosed herein sheath configurations, a diameter of the lumen of the sheath shown in **FIG.19** increases from the first rest diameter *dᵣ* to the second expanded diameter *dₑ.* After passage of the medical device through the lumen, the first segment **1806,** the second segment **1808,** and the third segment **1810** are configured to slidably move back along each other such that the overlapping portion between the first segment and the second segment, between the second segment and the third segment, and between the first segment and the third segment increases. After the passage of the device, the diameter of the lumen decreases from the second expanded diameter *dₑ* to a diameter that is substantially identical to the first rest diameter *dᵣ.*

It is further understood that the *dᵣ,* as disclosed above, can be uniform along a length of the sheath or vary from the proximal end of the sheath to the distal end of the sheath, as shown **in** **FIGS. 3A-3C****.** It is further understood that any values of dᵣ disclosed herein are also applicable to the sheath configurations, as shown in **FIG. 18** and **19****.**

During transcatheter aortic valve replacement (TAVR) procedures, a sheath as disclosed herein is used to provide access to the vasculature with no trauma to the patient, acting to maintain hemostasis and facilitate the delivery of interventional devices, as well as wire and catheter exchanges. In order for the sheath to be as minimally invasive as possible, it must have a low profile, or low outer diameter (OD), upon entry. However, the sheath must expand to a larger diameter once inside the body in order to allow passage of catheters larger than the initial diameter of the sheath. The force to advance these devices through the sheath is commonly referred to as push force. With larger devices, such as a crimped valve on a delivery system (DS), as well as challenging vessel anatomies, such as small, tortuous, or constricted vessels, the push force during the procedure is of great importance. High push force can cause procedural delays, physician dissatisfaction, and even inability to complete the procedure.

An important function of the sheath is to have clinically acceptable push force to advance the delivery system and valve throughout the sheath for all patient anatomies. In some aspects, lubrication is used to aid in this reduction of push force. Lubrication is placed in between layers that slide over one another, reducing the frictional forces that are needed to be overcome to expand the sheath, thus making the DS easier to pass through the sheath. Recent studies have shown that lubrication is needed in order to bring push forces down to acceptable levels.

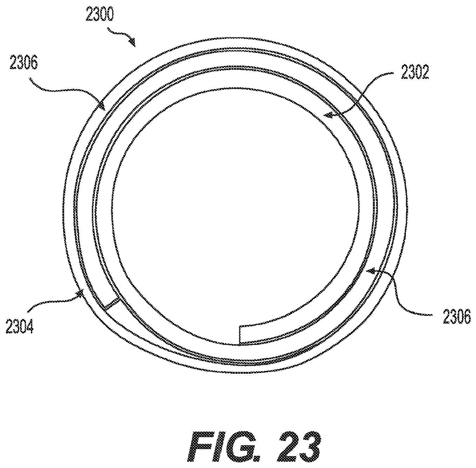

Still, further, the sheath configurations, as disclosed herein, can comprise a lubricant. One exemplary aspect of the sheath **2300** is shown in **FIG. 23****.** In this configuration, for example, a lubricant **2306** can be disposed between an inner liner **2302** and an outer layer **2304.**

For sheath having a configuration as shown in **FIG. 18** and **19****,** a lubricant can be disposed between any portions and segments in any amounts and any combinations. In certain exemplary and unlimiting aspects, the lubricant can be disposed between the first and the second segment, or between the second and the third segment, or between the first and the third segment, or any combination thereof. In yet other aspects, the lubricant can be disposed such that it is positioned on an innermost surface of the sheath, or an outermost surface of the sheath, or a combination thereof.

In still further aspects, the lubricant is disposed along a whole circumference of the inner liner, or it can be disposed between at least a portion of the overlying portion of the sheet and at least a portion of the sliding portions of the sheet.

Still, further, the lubricant can be disposed along at least a portion of the inner surface of the sheet or at least a portion of the outer surface of the sheet or a combination thereof.

Any known in the art lubricants can be utilized. In yet further aspects, the lubricant can comprise a PTFE-based lubricant or a silicone-based lubricant. In certain and unlimiting aspects, the lubricants can comprise Christo Lube supplied by ECL, or MED10/6670 or PRO-3499 supplied by Nusil, or PRO-3595 also supplied by Nusil. In still further aspects, it is understood that the amount of the first and/or second lubricant can be easily determined by one of ordinary skill in the art.

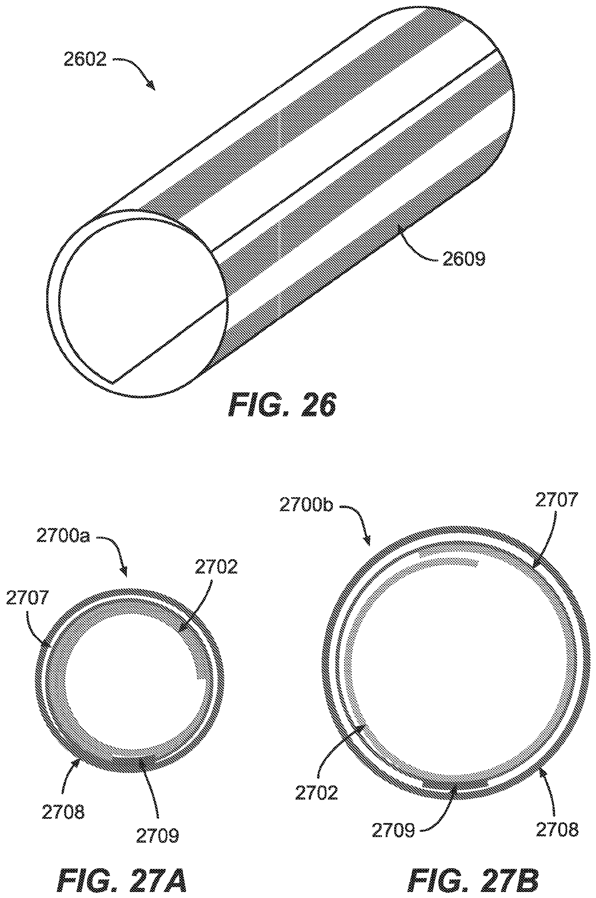

In still certain aspects, the lubricant can be disposed in a predetermined pattern, as shown, for example, in **FIG. 26****.** In such aspects, the lubricant is disposed in a pattern **2609,** for example, on the inner liner **2602.** It is understood that the pattern **2609** is only exemplary, and any desired pattern for any specific application can be applied.

The lubricant can be applied in any manner. For example, it can be applied manually. Lubrication is brushed by hand onto the sheath, making it hard to precisely control how much lubrication is added to the sheath and the exact locations where the lubrication is applied onto the sheath.

Lubricants, as disclosed herein, therefore, can also be applied by pad-printing or spraying, resulting in the material being applied in a precisely controlled and repeatable manner, suitable for large-scale manufacturing. Detailed methods of lubricant application are discussed below.

It is understood, however, that in aspects where the lubricant is applied by pad-printing, the lubricant has a viscosity prior to its application from about 600 to about 1,200 cP, including exemplary values of about 650 cP, about 700 cP, about 750 cP, about 800 cP, about 850 cP, about 900 cP, about 950 cP, about 1,000 cP, about 1,050 cP, about 1,100 cP, and about 1150 cP.

While in aspects where the lubricant is sprayed, the lubricant can have a viscosity equal to or less than about 600 cP, or about 550 cP, about 500 cP, about 450 cP, about 400 cP, about 350 cP, or equal to or less than about 350 cP.

In still further aspects, when the lubricant is cured prior to positioning the outer layer on the inner liner of the sheath.

The lubricants can also form a film. When a film of the lubricant is formed, such a film can have a thickness of equal to or less than about 20 µm, about 15 µm, about 10 µm, about 5 µm, about 1 µm, or even equal to or less than about 0.5 µm.

In still further aspects, the inner liner of any of the configurations disclosed herein can comprise a polyolefin, polyamide, fluoropolymer, copolymers thereof, or blends thereof. In still further aspects, the polyolefin can comprise a high-density polyethylene, polypropylene, or blends thereof.

In still further aspects, the sheet can comprise one or more layers. In yet further aspects, the sheet can have a multilayer structure. In some aspects, if one or more layers are present, each layer can comprise the same or different polymer. In still further aspects, the sheet can have a predetermined thickness, wherein the predetermined thickness can be defined by one of ordinary skill in the art depending on the specific application. In certain aspects, the predetermined thickness of the inner liner can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the predetermined thickness of the sheet forming the inner liner of any of the disclosed herein configurations can be varied depending on the desired amount of radial expansion, as well as the strength required.

In certain aspects, the inner liner of any of the sheath configurations described herein can comprise a compound material. For example, the polymer layer of the sheet used to form the inner liner can comprise a compound material comprising a polyolefin and a lubricious filler. It is understood that any of the polyolefins mentioned above can be used. In some exemplary aspects, the polyolefin used in the compound material is high-density polyethylene. In yet other aspects, the lubricious filler can be any filler that can improve the lubricity of the polymer layer and decrease its overall friction coefficient of the liner. In some exemplary and unlimiting aspects, the lubricious filler can comprise any additive that is known to reduce friction and behave as a lubricant. In such exemplary and unlimiting aspects, the lubricious filler can comprise one or more of graphene, reduced graphene oxide, carbon black, boron nitride, silicones, talc, polytetrafluorethylene (PTFE), fluorinated ethylene propylene, and the like. In still further aspects, the lubricious filler comprises a PTFE filler. In yet further aspects, the PTFE filler is a powder.

In yet further aspects, the lubricious filler can be present in any amount. In some exemplary and unlimiting aspects, the lubricious filler can be present in an amount from about 5 wt % to about 20 wt% of a total weight of the compound material used to make the polymer layer of the inner liner. In yet further aspects, the lubricious filler can be present in an exemplary amount of about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, or about 20 wt%.

In still further aspects, the sheet comprising such compound materials is lubricious and can have a coefficient of friction less than about 0.5, less than about 0.4, less than about 0.3, less than about 0.2, less than about 0.1, or less than about 0.05, or even less than about 0.01. It is further understood that the sheet can have a coefficient of friction having any value between any two foregoing values.

It is further understood that when the polymer layer of the sheet used to form the inner liner comprises the disclosed herein compound material, the sheath can be substantially free of a separately disposed lubricant. For example, the lubricant as disclosed above that is applied between the overlapping portions of the inner liner or between an outermost surface of the inner liner and an innermost surface of the outer layer may not be needed if the inner liner itself comprises a lubricious filler. However, disclosed herein are also aspects of the sheath where the inner liner comprises a lubricious compound in its composition, and a separate lubricant as disclosed above is still applied between various portions of the sheath. In such exemplary aspects, this additional lubricant applied manually, pad-printed, or sprayed can be applied between some of the portions of the inner liner and outer layer or all the portions of the inner liner and outer layer as disclosed above. It is also understood that this additional lubricant can be applied in any desirable pattern. It can also be applied along all the length of the sheath or only some portions of the sheath. The lubricant can also be applied in different patterns in different portions of the sheath. Yet, in other aspects, the lubricant can be applied in the same pattern along various portions of the sheath.

In still further aspects, the inner surface of the sheet can be at least partially ribbed. In such exemplary aspects, the inner surface of the sheet can be at least partially ribbed prior to rolling the sheet into the spiral configuration to form the inner liner.

In some aspects, the sheath described herein that comprises the lubricious material in the inner liner can exhibit a push force needed to move the prosthetic device through the sheath that is comparable or even smaller than a push force of a substantially identical reference sheath rolled in a spiral configuration, wherein an inner liner of the substantially identical reference sheath comprises a polymer layer substantially free of a lubricious filler and comprises an amount of a lubricant disposed between overlapping portions of the spiral configuration and/or an outermost surface of the inner liner. In other words, in some aspects when the performances of any of the disclosed herein, sheath configurations are compared, in such exemplary and unlimiting aspects, the sheath having the lubricious material in the inner liner and no additional lubricant present can demonstrate similar or even better performance than the similar sheath without lubricious material present in the inner liner but having an additional lubricant dispersed between various portions of the sheath.

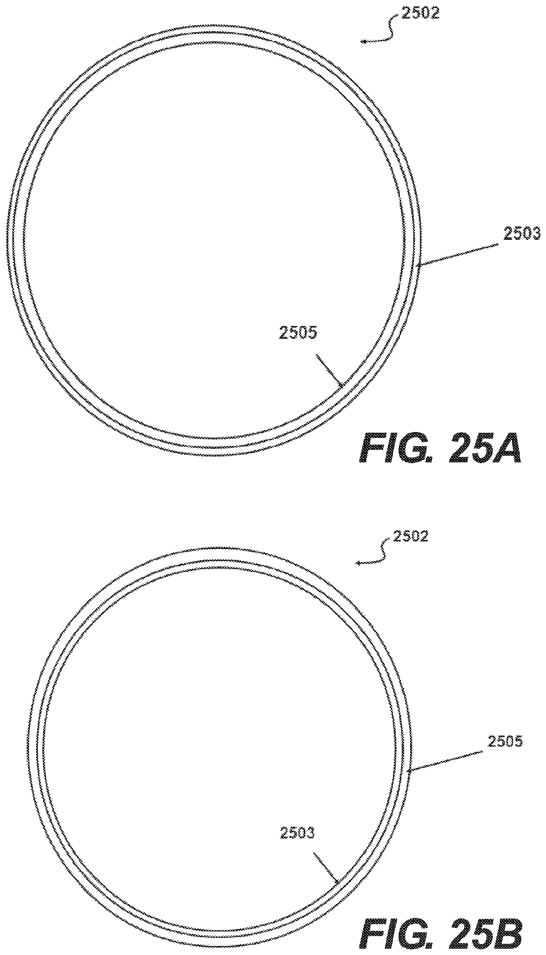

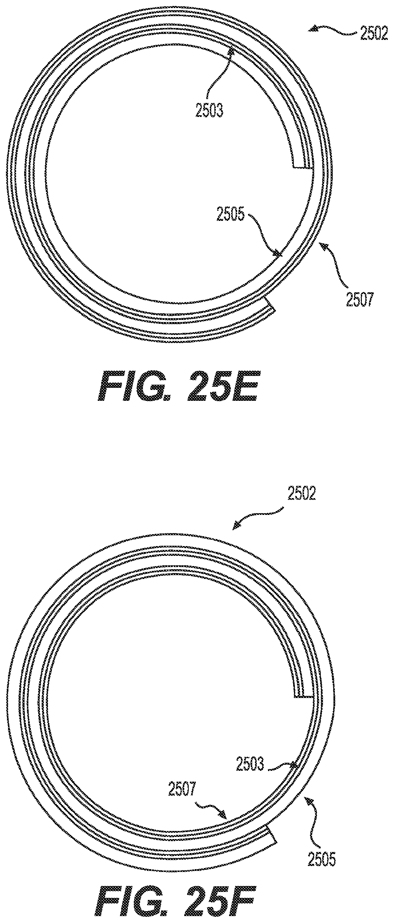

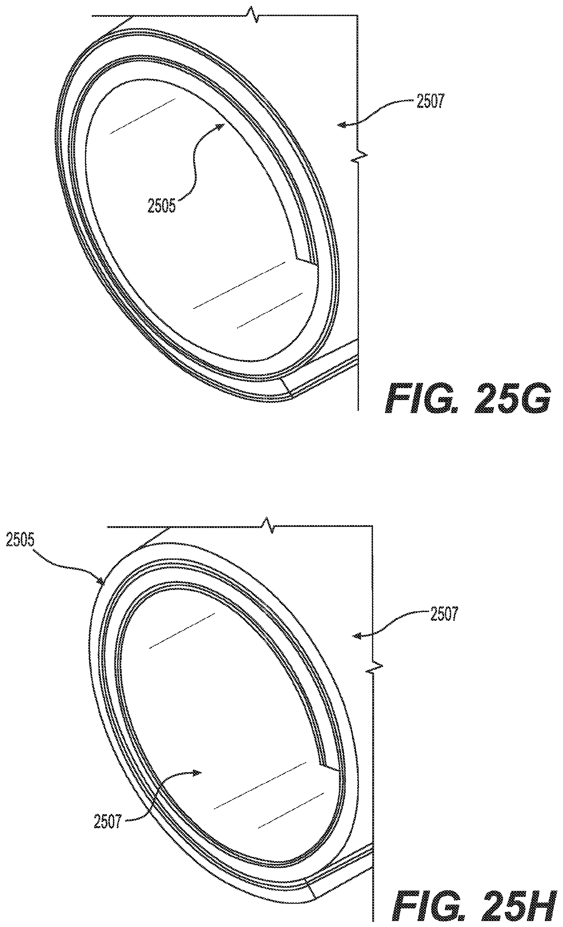

In still further aspects and as described herein, the sheath can also comprise a tie layer. In such aspects, the tie layer can be disposed at the inner surface of the inner liner or the outer surface of the inner liner, referring to **FIGS. 25A-B,** for example. **FIGS. 25A-B** show an exemplary coextruded tubing that can be used to form the sheet that is then rolled into the spiral configuration. The specifics of the methods of forming the inner liner are discussed in more detail below. Here, **FIGS. 25A-B**show the coextruded tubing **2502** comprising a polymer layer **2505** and a tie layer **2503.** **FIG. 25A** shows the tie layer **2503** is coextruded with the polymer layer **2505** such that the tie layer is positioned on an outer surface of the polymer layer. It is understood that the outer surface of the polymer layer will define at least a portion of the outer surface of the inner liner when it is in the spiral configuration. **FIG. 25B** shows a tie layer **2503** being coextruded with the polymer layer **2505** such that the tie layer **2503** is positioned on an inner surface of the polymer layer. It is understood that the inner surface of the polymer layer will define at least a portion of the inner surface of the inner liner when it is in the spiral configuration.

It is understood that the polymer layer **2505** can be any polymer layer described above and used to make the sheet. In certain aspects, the polymer layer can be high-density polyethylene.

In yet further aspects, the tie layer **2503** can comprise any material suitable for the desired application. It is understood that the tie layer can have adhesive or bonding properties. In certain aspects, the tie layer can comprise a polyurethane material such as Tecoflex, or polymer, copolymer, or terpolymer such as maleic anhydride modified polyolefin, for example, and without limitation, Orevac^{®} (commercially available from Arkema), ethylene acrylic acid copolymers, such as DOW Chemical Primacor^{®}, ethylene acrylate copolymers such as Lotryl^{®} (commercially available from Arkema), ethylene glycidyl methacrylate copolymer, ethylene acrylic esters glycidyl methacrylate terpolymer such as Lotader^{®} (commercially available from Arkema), ethylene acrylic esters maleic anhydride terpolymers such as Lotader^{®} or Orevac^{®} (commercially available from Arkema).

In certain aspects, a total thickness of the sheet having the polymer layer and the tie layer can be from about 0.002 inches to about 0.025 inches, including exemplary values of about 0.003, about 0.004, about 0.005, about 0.006, about 0.007, about 0.008, about 0.009, about 0.01, about 0.015, and about 0.02 inches. It is further understood that the total thickness of the sheet forming the inner liner of any of the disclosed herein configurations can be varied depending on the desired amount of radial expansion, as well as the strength required.

In yet further aspects, the tie layer can have a thickness from about 0.001" to about 0.003", including exemplary values of about 0.0011", about 0.0012", about 0.0013", about 0.0014", about 0.0015", about 0.0016". about 0.0017", about 0.0018", 0.0019", about 0.0020", about 0.0021", about 0.0022", about 0.0023", about 0.0024", about 0.0025", about 0.0026", about 0.0027", about 0.0028", and about 0.0029".

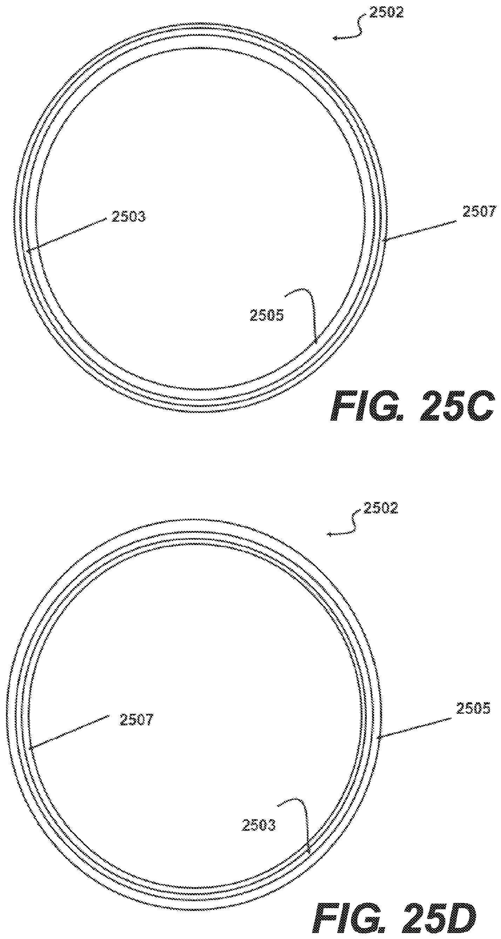

In still further aspects, any of the disclosed herein sheath configurations can have at least one lubricious liner, as, for example, shown in **FIGS. 25C-D.**

**FIGS. 25C-D** show an exemplary coextruded tubing comprising the tie layer **2503** and the polymer layer **2505,** and the disclosed above lubricious liner **2507.** This tubing can be used to form the sheet that is then rolled into the spiral configuration. The specifics of the methods of forming the inner liner are discussed in more detail below. As can be seen herein, the lubricous liner **2507** is disposed on the tie layer **2503.** It is understood that in one configuration, the tie layer and the lubricious liner are disposed on the outer surface of the polymer layer **(****FIG. 25C****)** or on the inner surface of the polymer layer **(****FIG. 25D****).** In yet further aspects, the lubricious liner is bonded to the polymer layer of the sheet with the tie layer.

**FIGS. 25E-H** show various configurations of sheet **2502** in the spiral configuration when the sheet comprises the polymer layer **2505,** the tie layer **2503,** and the lubricious liner **2507.**

The lubricious liner can comprise any material that can reduce the coefficient of friction of the sheath. In some exemplary and unlimiting aspects, the lubricious liner can comprise PTFE, polyether block amide, silicone-based liners, perfluoro alkoxy alkane-based liner, e-PTFE, ethylene tetrafluoroethylene, and the like. In yet further aspects, the lubricious liner comprises PTFE.

In yet further aspect, the total thickness of the sheath can be any thickness, as disclosed above.

Yet in other aspects, the at least one lubricious liner has a thickness from about 0.001" to about 0.005", including exemplary values of about 0.0011", about 0.0012", about 0.0013", about 0.0014", about 0.0015", about 0.0016", about 0.0017", about 0.0018", 0.0019", about 0.0020", about 0.0021", about 0.0022", about 0.0023", about 0.0024", about 0.0025", about 0.0026", about 0.0027", about 0.0028", about 0.0029", about 0.0030" about 0.0031", about 0.0032", about 0.0033", about 0.0034", about 0.0035", about 0.0036", about 0.0037", about 0.0038". 0.0039", about 0.0040", about 0.0041", about 0.0042", about 0.0043", about 0.0044". about 0.0045", about 0.0046", about 0.0047", about 0.0048", and about 0.0049".

In still further aspects, the lubricious liner can be further ribbed. It is also understood that aspects comprising an additional lubricant added separately from the lubricious liner are also disclosed. In such aspects, an additional lubricant can be disposed by any of the disclosed herein methods. It can be manually disposed, pad-printed, or sprayed. It is further understood that when this additional lubricant is present, it can be disposed in any of the disclosed herein predetermined patterns, along a portion of the sheath length or along a whole length of the sheath. In yet other aspects, the additional lubricant is not present when the lubricant layer, as described herein, is present.

In still further aspects, the outer layer of any one of the sheath configurations can comprise a styrene-based elastomer, polyurethane, latex, copolymers thereof, blends thereof, or co-extrudates thereof. In certain and unlimiting aspects, the polymer can comprise polyether block ester copolymer, polyesters, polyvinyl chloride, thermoset silicone, polyisoprene rubbers, polyolefin, other medical grade polymers, or combinations thereof.

In still further aspects, the outer layer can comprise one or more layers. In some aspects, at least one layer comprises the styrene-based elastomer. In yet other aspects, at least one layer can comprise polyurethane. While in other aspects, the at least one layer comprises a blend of the styrene-based elastomer and polyurethane.

It is understood that the hardness of each layer of the disclosed sheath can also be varied depending on the particular application and desired properties of the sheath. In some aspects, the layer of the outer layer has a Shore A durometer from 20A to 50A, including exemplary values of about 25A, about 30A, about 35A, about 40A, and about 45 A.

In yet further aspects, the polymers layer of the outer layer can have a Shore hardness of less than 90 Durometer, less than 80 Durometer, less than 70 Durometer, less than 60 Durometer, less than 50 Durometer, less than 40 Durometer, less than 30 Durometer, or less than 20 Durometer. In yet further exemplary aspects, the polymers layer of the outer layer can have a Shore hardness from about 25 Durometer to about 75 Durometer, including exemplary values of about 30 Durometer, about 35 Durometer, about 40 Durometer, about 45 Durometer, about 50 Durometer, about 55 Durometer, about 60 Durometer, about 65 Durometer, and about 70 Durometer.

The sheath as shown in any of the preceding configurations can also comprise an outer layer comprising a first polymer layer. wherein the first polymer layer comprises a first compound composition comprising from greater than 0 wt% to less than 100 wt% of a polymer comprising a polyether block amide, a polyurethane, or a combination thereof; less than about 65 % of an inorganic filler based on a total weight of the first compound composition; and up to about 20 % of a solid lubricant filler based on a total weight of the first compound composition. It is understood, however, that there are also aspects where the disclosed sheaths can comprise additional components. These exemplary aspects are disclosed herein, as shown below in detail.

In certain aspects, the outer layer comprises a first polymer layer. In such exemplary aspects, the first polymer layer can comprise a first compound composition comprising from greater than 0 wt% to less than 100 wt%, including exemplary values of about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof.

In still further aspects, the first compound composition can comprise from greater than about 35 wt% to less than about 80 wt%, including exemplary values of about 40 wt%, about 45 wt%, about 50 wt%, about 55 wt%, about 60 wt%, about 65 wt%, about 70 wt%, and about 75 wt% of a polymer comprising a polyether block amide, a polyurethane, or any combination thereof.

In certain aspects, the polymer in the first compound composition comprises a polyether block amide. In such exemplary aspects, the polyether block amide can comprise PEBAX^{®} from Arkema. In yet further aspects, the polymer can comprise polyurethane, for example, NEUSoft^{®}. While in still further aspects, the polymer can compromise a combination of the polyether block amide, such as, for example, PEBAX^{®} and polyurethane. It is further understood that if the mixture of the polymers is present, such a mixture can comprise each component in any amount relative to another component to provide the desired polymer falling within the disclosed above range.

In still further aspects, the first compound composition can comprise less than about 65 wt% of an inorganic filler based on a total weight of the first compound composition, including exemplary values of less than about 60 wt%, less than about 55 wt%, less than about 50 wt%, less than about 45 wt%, less than about 40 wt%, less than about 35 wt%, less than about 30 wt%, less than about 25 wt%, less than about 20 wt%, less than about 15 wt%, less than about 10 wt%, less than about 5 wt%, and less than about 1 wt% of the inorganic filler.

In yet further aspects, the inorganic filler can be present in an amount of at least about 1 wt%, at least about 2 wt%, at least about 5 wt%, at least about 10 wt%, at least about 15 wt%, at least about 20 wt%, at least about 25 wt%, at least about 30 wt%, at least about 35 wt%, at least about 40 wt%, at least about 45 wt%, at least about 50 wt%, or at least about 55 wt%.

In still further aspects, the inorganic filler can comprise any inorganic materials that can be used as a filler and are acceptable for the desired application. In certain exemplary and unlimiting aspects, the inorganic filler can comprise bismuth oxychloride, barium sulfate, bismuth subcarbonate, calcium carbonate, aluminum trihydrate, barite, kaolin clay, limestone, or any combination thereof. Again, it is understood that the inorganic filler can comprise a combination of the various fillers. In such exemplary aspects, an amount of each filler in the combination can be in any range to provide a final combination that falls within the disclosed above range.

In still further aspects, the first compound composition can comprise up to about 20 wt% of a solid lubricant filler based on a total weight of the first compound composition, including exemplary values of about 0.01 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, about 19 wt%, and about 19.9 wt%. In yet further aspects, the solid lubricant filler can be present up to about 20 wt%, up to about 15 wt%, or up to about 10 wt% based on a total weight of the first compound composition.

In still further aspects, the solid lubricant filler can comprise any additive that is known to reduce friction and behave as a lubricant. In such exemplary and unlimiting aspects, the solid lubricant filler can comprise one or more of graphene, reduced graphene oxide, carbon black, boron nitride, silicones, talc, polytetrafluorethylene (PTFE), fluorinated ethylene propylene, and the like. In still further aspects, the solid lubricant comprises a PTFE filler. In yet further aspects, the PTFE filler is a powder.

In still further aspects, the first compound composition can further comprise at least one tackiness reducing compound. Any compounds known in the art as capable of reducing the tackiness of the polymer composition can be considered and used for the purpose of this disclosure. In yet further exemplary and unlimiting aspects, the at least one tackiness reducing compound comprises ProPell^{™} from Foster Corporation

In certain aspects, the at least one tackiness reducing compound is present in an amount from 0 wt% to about 20 wt%, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the first compound composition. In still further aspects, the at least one tackiness reducing compound is present in any amount having a value between any two foregoing values. For example, and without limitation, the at least one tackiness reducing compound can be present in an amount from about 1 wt% to about 5 wt%, or from about 5 wt% to about 10 wt% based on a total weight of the first compound composition.

In still further aspects and as disclosed herein, the polymer in the first polymer layer composition has a substantially same durometer along a total length of the outer layer. It is understood, however, the durometer of the polymer in the first polymer layer composition of the outer layer can also be varied along the length of the outer layer. For example, and without limitation, disclosed herein are aspects where a durometer of the polymer in the first polymer layer composition at a proximal end of the outer layer is different from a durometer of the polymer in the first polymer layer composition at a distal end of the outer layer.

In still further aspects, the polymer in the first polymer layer composition has a Shore D from about 20D to about 72D, including exemplary values of about 25D, about 30D, about 35D, about 40D, about 45D, about 50D, about 55D, about 60D, about 65D, and about 70D. In still further aspects, the polymer in the first polymer layer composition has a Shore D from about 20D to about 35D. In still further aspects, the polymer in the first polymer layer composition has a Shore D of about 30D. Yet, in still further aspects, the polymer in the first polymer layer composition has a Shore D of about 25D.

It is understood that the outer layer, as disclosed herein, can comprise aspects where only one polymer layer is present. Yet, in other aspects, two or more polymer layers can be present in the outer layer. In such exemplary aspects, the outer layer can comprise a second polymer layer comprising a second compound composition comprising from greater than 0 wt% to 100 wt% of a second polymer comprising polyether block amide, a polyurethane, or a composition thereof. Similar to the first compound composition, the second polymer can be present in any amount that falls within the disclosed range. For example, the second polymer can be present in the second compound composition from greater than 0 wt%, about 0.01 wt%, about 1 wt%, about 5 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, and about 99.9 wt% of a polymer comprising a polyether block amide, a polyurethane, or any combination thereof. In yet further aspects, the second polymer can be present in the second compound composition from greater than about 95 wt% to less than about 99wt%, including exemplary values of about 95.5 wt %, about 96 wt%, 96.5 wt %, about 97 wt%, about 97.5 wt %, about 98 wt%, and about 98.5 wt%.

In yet further aspects, the second compound composition can further comprise up to 20 wt% of a tackiness reducing additive, including exemplary values of about 0.01 wt%, about 0.05 wt%, about 0.1 wt%, about 0.5 wt%, about 1 wt%, about 2 wt%, about 3 wt%, about 4 wt%, about 5 wt%, about 6 wt%, about 7 wt%, about 8 wt%, about 9 wt%, about 10 wt%, about 11 wt%, about 12 wt%, about 13 wt%, about 14 wt%, about 15 wt%, about 16 wt%, about 17 wt%, about 18 wt%, and about 19 wt% based on a total weight of the second compound composition. In still further aspects, the at least one tackiness reducing compound is present in any amount having a value between any two foregoing values. For example, and without limitation, the at least one tackiness reducing compound can be present in an amount from about 1 wt% to about 5 wt%, or from about 5 wt% to about 10 wt% based on a total weight of the second compound composition. In still further aspects and as disclosed herein, the second compound composition can be substantially free of a solid lubricant filler.

It is further understood that in certain aspects, the first polymer in the first compound composition can be the same as the second polymer in the second compound composition. Yet, in other aspects, the first polymer in the first compound composition is different from the second polymer in the second compound composition. In yet further aspects, the second polymer layer composition comprises PEBAX^{®}. While in further aspects, the second polymer layer composition can comprise polyurethane, for example, NEUSoft^{®} from PolyOne.

In still further aspects, the second polymer has a Shore D from about 20D to about 35D. Yet, in further aspects, the second polymer has a Shore D of about 25D or about 35D.

In still further aspects, the second compound composition can be substantially free of an inorganic filler. While in certain aspects, the inorganic filler can be present in the second compound composition in any amount from greater than 0 wt% to less than 100 wt%, including exemplary values of about 0.01 wt%. about 0.05 wt%, about 0.1 wt%, about 0.5 wt%, about 1 wt%, about 5 wt%, about 10 wt%, about 20 wt%, about 30 wt%, about 40 wt%, about 50 wt%, about 60 wt%, about 70 wt%, about 80 wt%, about 90 wt%, and about 95 wt%. In the aspects where the inorganic filler is present in the second compound composition, such inorganic filler can comprise any filler disclosed above.

In still further aspects, and as disclosed herein, the outer layer has a predetermined thickness, and wherein at least about 10%, at least about 20%, at least about 30%, at least about 40%, at least about 50%, at least about 60%, at least about 70%, at least about 80%, at least about 90%, or 100% of the predetermined thickness comprises the first and/or the second compound composition comprising the first and/or the second polymer having a Shore D equal to or lower than about 30D.

In still further aspects, the predetermined thickness of the outer layer can vary along a length of the sheath. While in other aspects, the predetermined thickness of the outer layer is the same along a length of the sheath. Yet, in further aspects, the predetermined thickness of the outer layer is greater at the proximal end. In still further aspects, the predetermined thickness of the outer layer is up to 0.006", for example, and without limitation from about 0.001" to about 0.006", including exemplary values of about 0.0015", about 0.002", about 0.0025", about 0.003", about 0.0035", about 0.004", about 0.0045", about 0.005", about 0.0055", and about 0.006".

In still further aspects, the first polymer layer and the second polymer layer can have the same thickness. While in other aspects, the first polymer layer and the second polymer layer have different thicknesses. For example, in some aspects, the first polymer layer has a thickness of about .001" to about 0.003", including exemplary values of about 0.0011", about 0.0012", about 0.0013", about 0.0014", about 0.0015", about 0.0016", about 0.0017", about 0.0018", about 0.0019", about 0.0020", about 0.0021", about 0.0022", about 0.0023", about 0.0024", about 0.0025", about 0.0026", about 0.0027", about 0.0028", and about 0.0029". Yet still, in further aspects, the second polymer layer can have a thickness of about 0.002" to about 0.004", including exemplary values of about 0.0011", about 0.0012", about 0.0013", about 0.0014", about 0.0015", about 0.0016", about 0.0017", about 0.0018", about 0.0019", about 0.0020", about 0.0021", about 0.0022", about 0.0023", about 0.0024", about 0.0025", about 0.0026", about 0.0027", about 0.0028", about 0.0029", 0.0030", about 0.0031", about 0.0032", about 0.0033", about 0.0034", about 0.0035", about 0.0036", about 0.0037", about 0.0038", and 0.0039".

In still further aspects, the predetermined thickness of the outer layer is greater at the proximal end. While in other aspects, the predetermined thickness of the outer layer is smaller at the distal end as compared to the predetermined thickness of the outer layer at the proximal end.

In still further aspects where two or more layers are present in the outer layer, the first polymer layer can define the inner surface of the outer layer, while the second polymer layer can define the outer surface of the outer layer. However, there are also aspects where the first polymer layer defines the outer surface of the outer layer, while the second polymer layer defines the inner surface of the outer layer. It is also understood that other aspects are also enclosed, where one or more additional polymer layers are disposed between the first polymer layer and the second polymer layer.

In still further aspects, the outer layer is extruded as a tube that can then be slide on the inner liner of the sheath. In the aspects where the first and the second polymer layers are present, such polymer layers can be co-extruded. In still further aspects, the first polymer layer can be substantially bonded to the second polymer layer. In such exemplary aspects, the first polymer layer substantially does not delaminate from the second polymer layer. It is understood that in some aspects, the bonding can be physical or chemical or any other type known in the art.

In still further aspects, any sheath that comprises the disclosed herein outer layer can exhibit an insertion force of less than about 55 N, less than about 50 N, less than about 45 N, less than about 40 N, less than about 35 N, or less than about 35 N when a medical device is pushed through the sheath.

In still further aspects, the outer layer can also exhibit a friction force of less than about 10 N, or less than about 9 N, or less than about 8 N, or less than about 7 N, or less than about 6 N, or even less than about 5 N, in the dry state against a substrate surface comprising one or more of polytetrafluoroethylene, fluorinated ethylene propylene, or high density polyethylene having a diameter of about 0.300".

In still further aspects, the outer layer extruded as a tube can exhibit a hoop force at 10 mm extension (about 85% strain) of less than about 10 N, or less than about 9 N., or less than about 8 N, or less than about 7 N, or less than about 6 N, or even less than about 5 N. In such exemplary aspects, the extruded tube that will form the outer layer of the sheath can have a diameter of about 0.290" (7.4 mm) and wall thickness as disclosed herein. In aspects where the outer layer has a diameter of about 0.290" (7.4 mm) and a total wall thickness of about 0.0045", with a sample length of about 0.25" (6.4 mm), a hoop direction forces at 10 mm extension can be less than about 8 N. It is understood that in some exemplary and unlimiting aspects, a low force at 10 mm extension is desired for low sheath expansion force.

In still further aspects, the outer layer can exhibit an elongation at break of ranging from about 650 % and about 800 %, including exemplary values of about 680 %, about 700 %, about 710 %, about 750 %, and about 780 %. It is understood that in some exemplary and unlimiting aspects, a high elongation is preferable for expansion to a larger diameter before the outer layer breaks.

In certain aspects, the outer layer extends along a portion of the length of the sheath. In such exemplary aspects, the outer layer can be positioned at the proximal end of the sheath, or in the middle of the sheath, or at the distal portion of the sheath. While in other aspects, the outer layer extends along the whole length of the sheath. In such exemplary aspects, the outer layer can be positioned at the proximal end of the sheath and extend to the distal end of the sheath.

In still further aspects, the outer layer of any one of the disclosed herein sheath configurations can comprise one or more polymer layers. In some aspects, a first polymer layer can be the first polymer layer disclosed above. Yet, in other aspects, the outer layer can also comprise a second polymer layer, wherein the second polymer layer can be any second polymer layer disclosed above. In some exemplary and unlimiting aspects, the second polymer layer can comprise polyurethane. In some exemplary and unlimiting aspects, the first polymer layer can comprise PEBAX alone or in combination with the inorganic filler and the solid lubricant filler, as disclosed above. In yet other exemplary and unlimiting aspects, the second polymer layer can comprise polyurethane, such as Neusoft.

In certain aspects, the first polymer layer, as disclosed above, and the second polymer can be coextruded to form a bump tubing. It is understood that a bump tubing or tapered tubing are commonly used in various applications.

It is understood that in some aspects, the bump tubing or the tapered tubing can be especially useful for certain catheter applications. Neurovascular and microcatheters usually depend on a larger proximal diameter to increase the pushability of the device, while a smaller distal end provides improved performance and deliverability.

In some aspects disclosed herein, the bump tubing that forms the outer layer of the sheath can have a predetermined length that is substantially similar to a length of the sheath. Yet, in other aspects, the bump tubing that forms the outer layer of the sheath can have a predetermined length that is shorter than a length of the sheath.

In certain aspects, the first polymer layer can define an inner surface of the outer layer (bump tubing). In such aspects, the second polymer layer will define an outer surface of the outer layer.

Yet, in other aspects, it can define an outer surface of the outer layer (bump tubing). In such aspects, the second polymer layer will define an inner surface of the outer layer.

In still further aspects, where the outer layer has the described above configuration, the first polymer layer can have a thickness from about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095". It is understood that the thickness of the first polymer layer in the outer layer can be uniform along the length of the sheath. Yet, in other aspects, the thickness of the first polymer layer in the outer layer can vary along the length of the sheath. In some aspects, the thickness of the first polymer layer is greater at the proximal end of the sheath as compared to a thickness of the first polymer layer along other portions of the sheath. Yet, in other aspects, the thickness of the first polymer layer can be smaller at the distal end of the sheath as compared to a thickness of the first polymer layer at the proximal end of the sheath.

In some aspects, the second polymer layer, if present in the outer layer, can have a thickness from about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095". It is understood that the thickness of the second polymer layer in the outer layer can be uniform along the length of the sheath. Yet, in other aspects, the thickness of the outer polymer layer in the outer layer can vary along the length of the sheath. In some aspects, the thickness of the second polymer layer is greater at the proximal end of the sheath as compared to a thickness of the second polymer layer along other portions of the sheath. Yet, in other aspects, the thickness of the second polymer layer can be smaller at the distal end of the sheath as compared to a thickness of the second polymer layer at the proximal end of the sheath.

In still further aspects, the first polymer layer can have a Shore D from about 20D to about 72D, including exemplary values of about 25D, about 30D, about 35D, about 40D, about 45D, about 50D, about 55D, about 60D, about 65D, and about 70D. In still further aspects, the polymer in the first polymer layer composition has a Shore D from about 20D to about 35D. In still further aspects, the polymer in the first polymer layer composition has a Shore D of about 30D. Yet, in still further aspects, the polymer in the first polymer layer composition has a Shore D of about 25D.

Yet, in other aspects, the second polymer layer can have a Shore A from about 30A to about 80A, including exemplary values of about 40A, about 45A, about 50A, about 55A, about 60A, about 65A, about 70A, and about 75A.

In still further aspects, and as disclosed herein, the outer layer has a total predetermined thickness, and wherein at least about 10%, at least about 20%, at least about 30%, at least about 40%, at least about 50%, at least about 60%, at least about 70%, at least about 80%, at least about 90%, or 100% of the total predetermined thickness comprises the first compound composition comprising the first polymer having a Shore D from about 20D to about 35D. In still further aspects, the total predetermined thickness of the outer layer is up to about 0.02", or up to about 0.015", or up to about 0.01", or up to about 0.009", or up to about 0.008", or up to about 0.007", or up to about 0.006'.

In yet further aspects, the total predetermined thickness of the outer layer can be uniform along the length of the sheath, or it can vary along the length of the sheath. In some exemplary and unlimiting aspects, the total predetermined thickness of the outer layer is greater at the proximal end of the sheath. Yet, in other aspects, the total predetermined thickness of the outer layer is smaller at the distal end of the sheath as compared to the total predetermined thickness of the outer layer at the proximal end of the sheath.

Also disclosed herein are sheath aspects, where the outer layer has one or more layers and when these layers are formed separately. For example, the first and second polymer layers, as described above, are formed separately instead of being coextruded. In such aspects, the outer layer is formed by disposing one of the polymer layers on another.

In some exemplary and unlimiting aspects, the second polymer layer can be at least partially disposed over the first polymer layer. Also disclosed are aspects where the first polymer layer at least partially overlies the second polymer layer.

In certain aspects, when the two polymers are formed separately and disposed over each other, each of the polymer layers can have a different length.

In some exemplary and unlimiting aspects, the first polymer layer can have a length that is shorter than the length of the second polymer layer. In some aspects, the first polymer layer can be disposed on the inner liner at the proximal end of the sheath and have a length from about 5 cm to about 15 cm, including exemplary values of about 6 cm, about 7 cm, about 8 cm, about 9 cm, about 10 cm, about 11 cm, about 12 cm, about 13 cm, and about 14 cm. In a still further aspect, the second polymer layer is then disposed on the first polymer layer. In such aspects, the second polymer layer can have any length that suits the desired application. In certain aspects, the second polymer layer can have a length that is substantially identical to the length of the sheath.

It is understood, however, that an opposite configuration of the outer layer is also disclosed. In such an aspect, the second polymer layer can be first disposed on the inner liner and have a length that is shorter than the length of the sheath. Further, the second polymer layer can be disposed on the second polymer layer. In such an exemplary aspect, the first polymer layer can have any length. In some aspects, the length of the first polymer layer can be substantially identical to the length of the sheath.

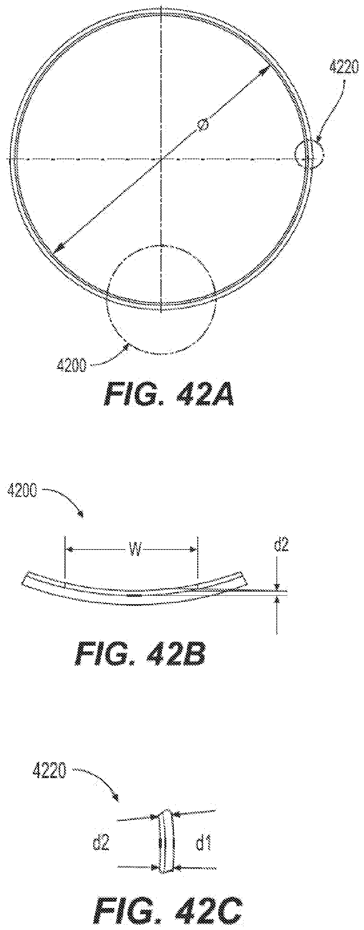

Still further, the first polymer layer, as disclosed in these aspects, can have a thickness that is uniform along the length of the first polymer layer, or it can vary along the length of the first polymer layer. For example, the thickness of the first polymer layer (d2, for example, as shown in **FIG. 42B****, 4200)** can be any thickness, as disclosed above. In some aspects, the thickness can be anywhere from about 0.001" to about 0.006", including exemplary values of about 0.002", about 0.0025", about 0.003", about 0.0035", about 0.004", about 0.0045", about 0.005", and about 0.0055".

Still further, the second polymer layer, as disclosed in these aspects, can have a thickness that is uniform along the length of the second polymer layer, or it can vary along the length of the second polymer layer. The thickness of the second polymer layer can be any thickness, as disclosed above. In some aspects, the thickness d1 of the second polymer can be, for example, as shown in **FIG. 42C****, 4200** anywhere from about 0.001" to about 0.010", including exemplary values of about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009', and about 0.0095".

In still further aspects, the overall thickness of the outer layer can be anywhere from about 0.001" to about 0.015", including exemplary values of about 0.0015", about 0.002", about 0.0025", about 0.003", about 0.0035", about 0.004", about 0.0045", about 0.005", about 0.0055", about 0.006", 0.0065", about 0.007", about 0.0075", about 0.008", about 0.0085", about 0.009", 0.0095", about 0.01", about 0.0105", about 0.011", about 0.01105", about 0.012", about 0.01205", about 0.013", about 0.01305", about 0.014", and about 0.01405.

In still further aspects, the outer layer disclosed herein can comprise at least two polymer layers. In still further aspects, the outer layer disclosed herein can comprise at least one intermediate reinforcement layer disposed between the first polymer layer and the second polymer layer. In still further aspects, the at least one intermediate reinforcement layer is a polymer layer.

In some aspects, the at least one intermediate layer can extend along the whole circumference of the outer layer. In yet further aspects where the first polymer layer forms the inner surface of the outer layer and the second polymer layer forms the outer surface of the outer layer, the intermediate layer is disposed between the outer surface of the first polymer and the inner surface of the second polymer layer. Yet in other aspects, and as disclosed above, if the second polymer layer forms the inner surface of the outer layer and the first polymer layer forms the outer surface of the outer layer, the intermediate layer is disposed between the outer surface of the second polymer layer and the inner surface of the first polymer layer. In still further aspects, the intermediate reinforcement layer can bond the first and second polymer layers and can also assist bonding of the outer layer as a whole to an inner member of the sheath.

In still further aspects, the at least one intermediate layer has a finite width that is smaller than the circumference of the outer layer. In such aspects, the at least one intermediate layer can be inserted as a strip between the first and the second polymer layers. In some exemplary and unlimiting aspects, if the outer layer has a distal outer diameter of about 0.200", the strip can have a width (w, for example, as shown in **FIG. 42B****, 4200)** from about 0.010" to about 0.150", including exemplary values of about 0.03", about 0.035", about 0.04", about 0.045", about 0.05", about 0.055", about 0.06", about 0.065", about 0.07", about 0.075", about 0.08", about 0.085", about 0.09", about 0.095", about 0.10", about 0.105", about 0.110", about 0.115", about 0.120", about 0.125", about 0.130", about 0.135", about 0.140", and about 0.145". It is understood that the widths shown above are exemplary, and if the distal outer diameter of the outer layer has a size different from 0.200", the strip width can be adjusted in the same or a different ratio.

In still further aspects, the at least one intermediate layer has a finite width that is smaller than the circumference of the outer layer. In such aspects, the at least one intermediate layer can be inserted as a strip between the first and the second polymer layers. In some exemplary and unlimiting aspects, if the outer layer has a distal outer diameter of about 0.200", the strip can have a width from about 5% to about 50% of the circumference of the outer layer. In still further aspects, the total combined width of the strips is about 5%, about 15%, about 20%, about 25%, about 30%, about 35%, about 40%, about 45%, or about 50% of the circumference of the outer layer. It is understood that the widths shown above are exemplary, and if the distal outer diameter of the elongated sheath has a size different from 0.200", the strip width can be adjusted in the same or a different ratio.

In still further aspects, the outer layer can comprise two or more intermediate layers. In such aspects, the two or more intermediate layers can be disposed, as individual strips, circumferentially between the first and the second polymer layers at a predetermined distance from each other. In aspects where the two or more intermediate layers are disposed between the first and the second polymer layers of the outer layer, a total combined width of all the strips is about 5% to about 50% of the circumference of the outer layer. In still further aspects, the total combined width of the strips is about 5%, about 15%, about 20%, about 25%, about 30%, about 35%, about 40%, about 45%, or about 50% of the circumference of the outer layer.

In still further aspects, the at least one intermediate layer is configured to provide an axial reinforcement to the outer layer and, as a result, to the sheath where the outer layer can be used. In such exemplary aspects, the at least one intermediate layer can be disposed along the length of the outer layer or along a portion of the length of the outer layer.

In some aspects, the portion of the length of the outer layer where the at least one intermediate layer is disposed is positioned at the distal end and/or proximal end of the outer layer. In yet other aspects, the at least one intermediate layer can also be positioned anywhere along the length of the outer layer.

It is further understood that in the aspects where the intermediate layer is present as one or more strips disposed circumferentially along the length of the sheath, the width of the strip can be the same along the length, or it can vary along the length. In aspects where the strips' width varies along the length of the outer layer, such a strip can have any of the disclosed above width values.

In still further aspects, the first polymer layer used in this exemplary outer layer can be any of the first polymer layers described above. In still further exemplary and unlimiting aspects, the first polymer layer forms the inner surface of the outer layer and comprises a first compound composition comprising from greater than 0 wt% to less than 100 wt% of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition.

Any of the disclosed above inorganic fillers and solid lubricant fillers can be present in any amount as disclosed. For example, the inorganic filler can comprise bismuth oxychloride, barium sulfate, bismuth subcarbonate, calcium carbonate, aluminum trihydrate, barite, kaolin clay, limestone, or any combination thereof. In yet other aspects, the inorganic filler can be present in at least 10 wt %. In still further aspects, the inorganic filler can be present in an amount of less than about 50 wt % based on a total weight of the first compound composition.

In yet further aspects, the solid lubricant filler can comprise a PTFE filler.

The first compound can also comprise any of the disclosed above additives. For example, the compound can comprise at least one tackiness reducing compound in an amount from about 1 wt% to about 20 wt%.

In still further exemplary aspects, the polymer present in the first compound can have Shore D from about 20D to about 35D, including exemplary values of about 22D, about 25D, about 27D, about 30D, and about 32D.

In yet further aspects, a durometer of the polymer in the first polymer layer composition at a proximal end of the outer layer can be different from a durometer of the polymer in the first polymer layer composition at a distal end of the outer layer.

In still further aspects, the polymer in the first compound can comprise polyether block amide, for example, PEBAX^{®}. While in other aspects, the polymer in the first compound can comprise polyurethane. In still further aspects, the first compound can also comprise polyamide.

In still further aspects, the thickness of the first polymer layer can be from about 1 mil to about 5 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, and about 4.9 mils.

In still further aspects, the second polymer layer can comprise any of the disclosed above polymers. In some aspects, the second polymer layer can comprise a second compound composition comprising from greater than 0 wt% to 100 wt% of a second polymer comprising polyether block amide, a polyurethane, or a composition thereof. In still further aspects, the second polymer layer can comprise a polyamide. In yet in some other aspects, the second compound can also comprise any of the fillers or additives disclosed above. While in some aspects, the second compound does not comprise the solid lubricant fillers disclosed herein. While in still further aspects, the second compound can comprise a tackiness reducing additive described in this disclosure. In some aspects, the second polymer can be a polyurethane. In still further aspects, the polyurethane is a thermoplastic polyurethane.

While in still further aspects, the second polymer can be a blend comprising a polyurethane with a styrene block copolymer. In still further aspects, the blend can further comprise additional polymers and copolymers. For example, ether-based polymers can be present in the blend. In some exemplary and unlimiting aspects, the second polymer can be chosen from commercially available polymers sold under the trade name of Neusoft^{™}. In still further aspects, the second polymer can have a Shore A durometer from about 20A to about 75A, including exemplary values of about 25A, about 30A, about 35A, about 40A, about 45A, about 50A, about 55A, about 60A, about 65 A, and about 70A. In yet further aspects, the second polymer can have a Shore A durometer of less than 60A. In some exemplary aspects, the second polymer can be Neusoft^{™} 597-50A.

In still further aspects, the thickness of the second polymer layer can be from about 1 mil to about 6 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, about 4.9 mils, about 5.1 mils, about 5.2 mils, about 5.3 mils, about 5.4 mils, about 5.5 mils, about 5.6 mils, about 5.7 mils, about 5.8 mils, and about 5.9 mils. In still further aspects, the thickness of the at least one intermediate reinforcement layer can be anywhere from about 1 mil to about 6 mils, including exemplary values of about 1.1 mils, about 1.2 mils, about 1.3 mils, about 1.4 mils, about 1.5 mils, about 1.6 mils, about 1.7 mils, about 1.8 mils, about 1.9 mils, about 2.0 mils, 2.1 mils, about 2.2 mils, about 2.3 mils, about 2.4 mils, about 2.5 mils, about 2.6 mils, about 2.7 mils, about 2.8 mils, about 2.9 mils, about 3.0 mils, about 3.1 mils, about 3.2 mils, about 3.3 mils, about 3.4 mils, about 3.5 mils, about 3.6 mils, about 3.7 mils, about 3.8 mils, about 3.9 mils, about 4.1 mils, about 4.2 mils, about 4.3 mils, about 4.4 mils, about 4.5 mils, about 4.6 mils, about 4.7 mils, about 4.8 mils, about 4.9 mils, about 5.1 mils, about 5.2 mils, about 5.3 mils, about 5.4 mils, about 5.5 mils, about 5.6 mils, about 5.7 mils, about 5.8 mils, and about 5.9 mils.

In still further aspects, the at least one intermediate layer can comprise any of the polymers disclosed herein. In some aspects, the at least one intermediate layer can comprise the first compound disclosed above. Yet, in other aspects, the at least one intermediate layer can comprise the second compound disclosed above. While in still further aspects, the at least one intermediate layer can comprise the first compound. Yet, in still further aspects, the at least one intermediate layer can comprise any polymers that are known in the art and suitable for the desired application. In some aspects, the at least one intermediate layer can comprise polyether block amide, polyurethane, or a combination thereof. While in still further aspects, the at least one intermediate layer is a polyether block amide, for example, PEBAX^{®}. While in still further aspects, the intermediate layer is a polyurethane. In such exemplary aspects, the at least one intermediate layer does not comprise a solid lubricant filler, such as a PTFE. In yet other aspects, the at least one intermediate layer does not comprise an inorganic filler. In still further aspects, the at least one intermediate layer can comprise a polymer comprising PEBAX^{®} or polyurethane having a Shore D (or Shore A) durometer from about 45D (85A) to about 90D, including exemplary values of about 50D, about 55D, about 60D, about 65D, about 70D, about 72D, about 75D, about 80D, and about 85D.

In yet further aspects, the at least one intermediate reinforcement layer can comprise a polyolefin. In still further aspects, the at least one intermediate reinforcement layer can comprise a polyethylene, a polypropylene, a graft modified polyethylene, or polypropylene. In yet further aspects, the at least one intermediate reinforcement layer can comprise the grafted low-density polyethylene (LDPE), grafted medium density polyethylene, grafted ultra-low-density polyethylene (ULDPE) grafted high density polyethylene (HDPE), grafted heterogeneously branched linear low-density polyethylene (LLDPE), grafted homogeneously branched linear ethylene polymers and substantially linear ethylene polymers, grafted polypropylene, or ethylene-vinyl acetate (EVA), or any combination thereof. In such exemplary aspects, a maleic anhydride or an acrylic acid can be used to graft the disclosed above polymers. In still further aspects, the at least one intermediate reinforcement layer can comprise a maleic anhydride or an acrylic acid grafted low-density polyethylene. In yet further aspects, the at least one intermediate reinforcement layer can comprise a maleic anhydride or an acrylic acid grafted polypropylene. In still further aspects, the at least one intermediate reinforcement layer can comprise a maleic anhydride or an acrylic acid grafted ethylene vinyl acetate. In still further aspects, the at least one intermediate reinforcement layer can comprise a maleic anhydride grafted polyolefin sold under a trademark of OREVAC^{®}.

In still further aspects, any of the disclosed above at least one intermediate reinforcement layer can thermally bond the outer layer to the variable inner liner of the sheath. In still further aspects, the intermediate reinforcement layer can be extruded to be positioned between the first polymer layer and the second polymer layer. In still further aspects, the at least one intermediate reinforcement layer can be fused with the first and second polymer layers by at least one of heat or compression.

In still further aspects, the outer layer as disclosed herein comprising the at least one intermediate reinforcement layer can exhibit an expansion force of less than about 50 N, less than about 49N, less than about 48N, less than about 47N, less than about 46N, less than about 45N, less than about 44N, less than about 43N, less than about 42N, less than about 41N, or even less than about 40N. It is further understood, however, that the expansion force is dependent on the size of the medical device passing through the sheath. The exemplary values shown above are suitable for a medical heart implant of about 26 mm. It is understood that the force values are not limited to the values disclosed above and are adjusted depending on the device size.

In still further aspects, the outer layer as disclosed herein that comprises the at least one intermediate reinforcement layer can exhibit a burst pressure greater than about 4.5 psi, greater than about 5 psi, greater than about 5.5 psi, greater than about 6 psi, greater than about 6.5 psi, greater than about 7 psi, greater than about 7.5 psi, greater than about 8 psi, greater than about 8.5 psi, greater than about 9 psi, greater than about 9.5 psi, greater than about 10 psi, greater than about 10.5 psi, greater than about 11 psi, greater than about 11.5 psi, about 12 psi, greater than about 12.5 psi, greater than about 13 psi, greater than about 13.5 psi, greater than about 14 psi, greater than about 14.5 psi, or greater than about 15 psi.

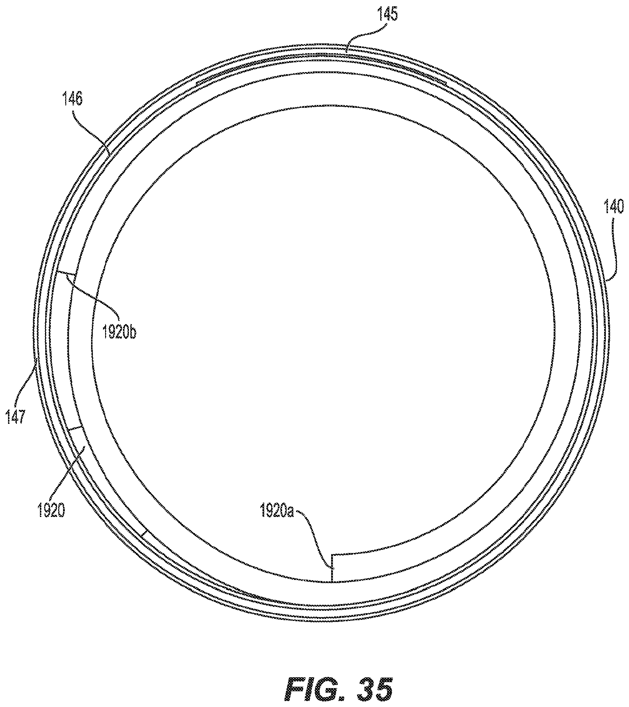

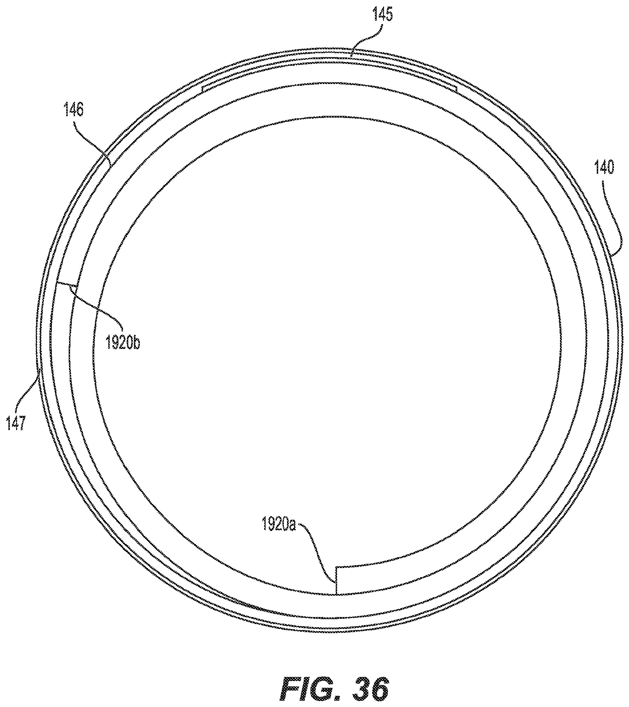

As illustrated in **FIG. 35-36****,** the outer layer **140** can include one or more axial reinforcing members **145** that extend longitudinally along all or a portion of the outer layer **140.** The reinforcing member **145** helps to prevent axial bunching of the outer layer **140** during insertion into the patient's vasculature while not sacrificing the low radial expansion force of the outer layer **140.**

As illustrated in **FIG. 10****,** the sheath **100** can include a tapered segment adjacent the flared end portion **114** at the proximal end of the sheath **100.** Referred to as a strain relief section, the tapered segment and the flared end portion **114** help ease the transition between the smaller diameter portion of the sheath **100** and the housing **101.** When the outer layer **140** is present, its thickness and/or composition can be adjusted to improve the performance of the strain relief section and to reduce the push force, as disclosed above.

For example, in certain aspects, the outer layer **140** can be bonded at the proximal end and/or distal end of the inner liner. At the proximal and distal ends, the outer layer **140** can be bonded to the inner liner around the full circumference of the outer layer.

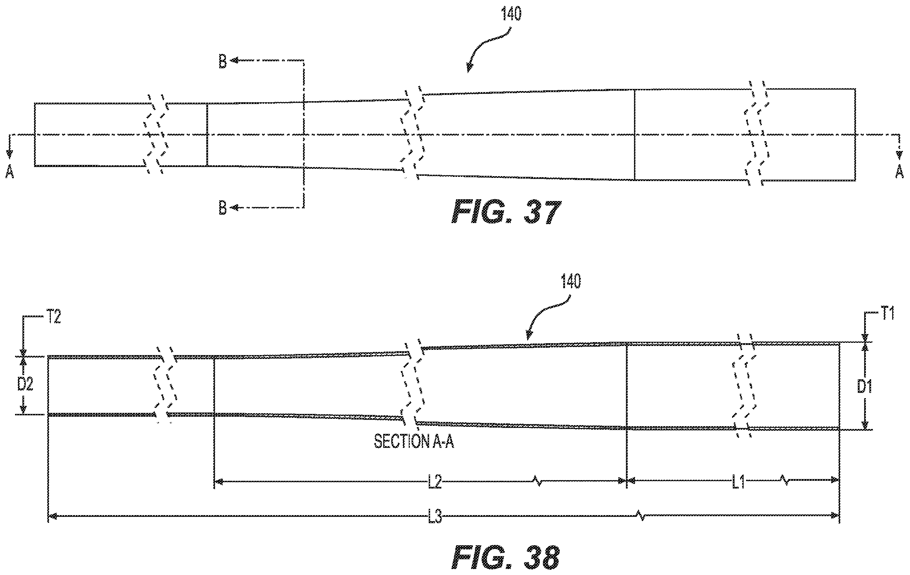

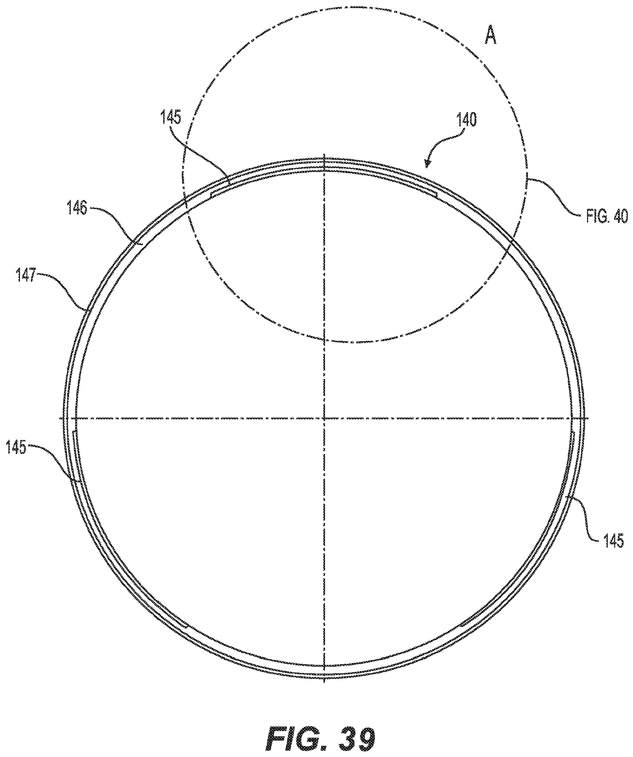

As disclosed herein, the outer layer can have the same diameter across the length of the sheath, or it can have a varied diameter across the length of the sheath. **FIG. 37** is an elevation view of the outer layer **140** showing a tapered segment adjacent the flared end portion at the proximal end of the sheath. **FIG. 38** is a cross-section view of an exemplary elongated tube taken along section line A-A of **FIG. 37****.** **FIG. 39** is a cross section view of the outer layer **140** taken along section B-B in **FIG. 37****.** As described above, the tapered portion is referred to as a strain relief section, and the tapered segment and the flared proximal end help ease the transition between the smaller diameter portion of the sheath **100** and the housing **101.** The length of the proximal end (L1) can range from 1.600 inches to 2.400 inches. In some aspects, the length of the proximal end is about 2.000 inches. The length of the tapered segment (L2) can range from 2.000 inches to 3.000 inches. In some aspects, the length of the tapered segment (L2) is about 2.500 inches. The overall length of the outer layer **140**/sheath 100 (L3) can range from 17.600 inches to 26.400 inches. In some aspects, the overall length of the outer layer **140**/sheath **100** (L3) is about 22.000 inches.

As provided in **FIG. 38****,** the diameter of the outer layer **140** at the proximal end is greater than the diameter of the outer layer **140** at the distal end. This allows the outer layer **140** to be slid over the inner liner **108** without having to be expanded. For example, the diameter of the outer liner **140** at the proximal end (D1) can range from 0.264 inches to 0.396 inches. In some examples, the diameter of the outer liner **140** at the proximal end (D1) is about 0.330 inches. The diameter of the outer layer **140** at the distal end (D2) can range from 0.176 inches to 0.264 inches. In some examples, the diameter of the outer layer at the distal end (D2) is about 0.220 inches.

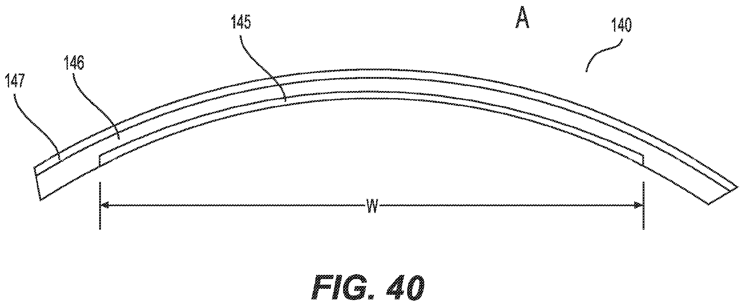

In still further aspects, the outer layer **140** can comprise two or more reinforcing members **145.** In such aspects, the two or more reinforcing members **145** can be disposed, as individual strips, disposed circumferentially in the first polymer layer, in the second polymer layer, or between the first and second layers at a predetermined distance from each other. **FIG. 39** is a detailed view of the outer layer **140** taken along section lines B-B in **FIG. 37****.** As provided in **FIG. 39****,** the outer layer **140** includes three reinforcing members **145.** In some examples, the outer layer **140** includes only one reinforcing member **145 (****FIG. 40****).** In other examples, the outer layer includes up to eight reinforcing members **145.** When more than one reinforcing member **145** is used, the reinforcing members are spaced evenly around the circumference of the outer layer **140.** As further illustrated in **FIG. 39****,** the reinforcing member **145** can have a rectilinear shape (e.g., rectangular) in the cross section. However, any other regular or irregular shape is contemplated. For example, reinforcement members can also be round in the cross section.

In further aspects, the reinforcing member **145** has a finite width that is smaller than the circumference of the outer layer **140.** The total combined width (w) of the reinforcing members **145** can range from 5% to 50% of the circumference of the outer layer **140.** In still further aspects, the total combined width of the strips is about 5%, about 15%, about 20%, about 25%, about 30%, about 35%, about 40%, about 45%, or about 50% of the circumference of the outer layer.

**FIG. 40** includes a partial view of the outer layer **140 of** **FIG. 39****.** As provided in **FIG. 40****,** the circumferential width of the reinforcing members **145** can range from 0.010 inches to 0.150 inches. In some examples, the distal end of the outer layer **140** has a diameter of 0.200 inches, and the circumferential width of the reinforcing members **145** can range from 0.010 inches to 0.150 inches. In some exemplary and unlimiting aspects, the diameter of the outer layer at the distal end is about 0.200", the reinforcing member can have a width from about 0.010" to about 0.150", including exemplary values of about 0.03", about 0.035", about 0.04", about 0.045", about 0.05", about 0.055", about 0.06", about 0.065", about 0.07", about 0.075", about 0.08", about 0.085", about 0.09", about 0.095", about 0.10", about 0.105", about 0.110", about 0.115", about 0.120", about 0.125", about 0.130", about 0.135", about 0.140", and about 0.145". It is understood that the widths shown above are exemplary, and if the distal outer diameter of the elongated sheath has a size different from 0.200", the strip width can be adjusted in the same or a different ratio. In yet still, further aspects, as described above, the width of the reinforcing member can be measured as a percentage of the outer layer circumference.

It is further understood that in the aspects where the reinforcing member **145** is present as one or more strips disposed circumferentially along the length of the outer layer **140,** the width of the reinforcing member **145** can be the same along the length, or it can vary along the length. In aspects where the reinforcing member **145** width varies along the length of the outer layer **140,** such a reinforcing member **145** can have any of the disclosed above width values.

In still further aspects, at least one reinforcing member **145** is configured to provide an axial reinforcement to the outer layer **140.** In such exemplary aspects, the at least one reinforcing member **145** can be disposed along the length of the outer layer **140** or along a portion of the length of the outer layer **140.** In some aspects, the portion of the length of the outer layer **140** where the at least reinforcing member **145** is disposed is positioned at the distal end and/or proximal end of the outer layer **140.** In yet other aspects, the reinforcing member **145** can also be positioned anywhere along the length of the outer layer **140.**

As described above, and as illustrated in **FIG. 39****,** the outer layer of the sheath **140** includes a two-layer construction comprising a first polymer layer **146** (disposed abut the inner liner of the sheath) and a second polymer layer **147** (disposed further from the inner liner of the sheath and forming an outer layer of the outer layer of the sheath). However, it is further understood that the outer layer can have both a two-layer and three-layer construction depending on the location. In some aspects, the layer formed by the second polymer can provide abrasion resistance (for example, between the sheath and a calcific lesion) and better resistance to the hydrophilic coating process, while the layer formed by the first polymer comprises a more lubricious material (for example, to prevent sticking of the outer layer against the inner liner of the sheath during expansion) and provides higher pressure resistance or ballooning resistance and hemostasis. In some aspects, the layer formed by the first polymer **146** forms the inner surface of the outer layer **140,** and the layer formed by the second polymer **147** forms the outer surface of the outer layer of the sheath, the reinforcing members **145** are disposed between the outer surface of the first polymer layer **146** and the inner surface of the second polymer layer **147.**

In some examples, the first polymer layer **146** can be composed of Pebax or polyurethane, having Shore 25D to 35D. In some examples, the first polymer layer **146** includes a PTFE powder, an optional inorganic filler, and an optional tackiness reducing additive to lower friction when inner member **108** of the sheath expanding by sliding against the outer layer **140.** In some examples, the second polymer layer **147** of the outer layer **140** is composed of polyurethane or polyurethane/Styrene Block Copolymer (SBC) having Shore A durometer lower than about 60, e.g., Neusoft 597-50A having Shore A hardness of about 55A. In certain examples, the first polymer layer **146** is constructed from Polyether Block Amide, such as Pebax having a Shore D durometer less than about 35.

As provided in **FIG. 39** and **40****,** the reinforcing members **145** are at least partially embedded in the first polymer layer **146.** In some examples, the thickness of the reinforcing member **145** is less than the thickness of the first polymer layer **146.** For example, as illustrated in **FIG. 40****,** the reinforcing members **145** have a thickness ranging from 0.0005 inches to 0.00155 inches. In some examples, the reinforcing members **145** have a thickness of about 0.001 inches. In an example configuration, the reinforcing members **145** have a thickness of 0.001 inches, and the first polymer layer has a thickness of 0.00154 inches. In another example, not shown, the reinforcing member **145** has a thickness corresponding to the thickness of the first polymer layer **146.** In a further example, the reinforcing member **145** has a thickness greater than the thickness of the first polymer layer **146.** In some examples, the first polymer layer **146** and the reinforcing member **145** are co-extruded. Similarly, the first polymer layer **146,** reinforcing member **145,** and the second polymer layer **147** are co-extruded with the reinforcing member **145** positioned between the first and second polymer layers **146, 147.** In other examples, the first polymer layer **146** is provided over the reinforcing member **145,** and the two components are bonded or fused together by at least one of heat or compression.

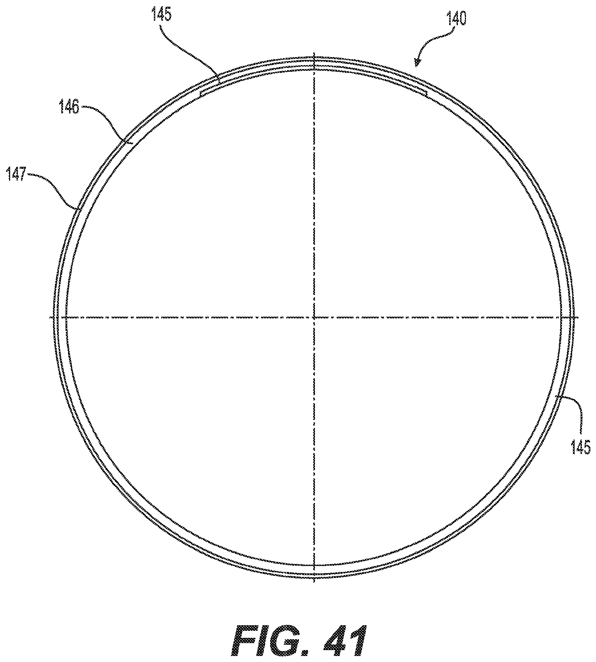

**FIG. 41** shows is a section view of another exemplary outer layer in a rest (unexpanded) configuration, including one reinforcing member, taken along section lines B-B of **35.**

As described above, the reinforcing members **145** are constructed from a stiffer material than the main body portion of the outer layer **140** (a first polymer layer **146,** a second polymer layer **147)** and also a material having a low coefficient of friction (e.g., high density polyethylene). In some examples, the reinforcing members **145** are constructed from a polymer compatible with the first and second polymer layers, including, for example, high durometer Pebax or polyurethane. The reinforcing member **145** can also be constructed from a material having a Shore D durometer ranging from 45D to 76D.

Additional examples of the sheath that can be used with the disclosed herein outer layer can be found in US application No. 63/021,945.

An additional exemplary two-layer construction is shown in **FIGS. 42A-42C****.** In such a configuration, for example, the reinforcement member can be a part of the inner layer, where the reinforcement member has the substantially identical thickness as the inner layer (shown in 4200 of **FIGS. 42A and 42B****).**

In some exemplary and unlimiting aspects, if the two-polymer layers of the outer layer are formed separately, a tie layer can be disposed between the two. It is further understood that any of the disclosed above tie layers can be utilized.

As discussed in detail above, an important function of the sheath is to have clinically acceptable push force for all patient anatomies.

To decrease the push force, various types of lubrication can be used. Some of the various lubrication types and methods are disclosed above. As disclosed, any of the disclosed lubricants (lubricious materials, lubricious fillers, lubricious liners) can reduce the frictional force between various layers of the sheath that slide over one another, making it easier for the delivery system to open the sheath. However, lubrication between the inner liner and the outer layer can cause the outer layer to easily slide over the sheath shaft. When large portions of the outer layer slide together, the outer layer can bunch up in one spot, thereby raising the outer diameter (OD), called bunching. This can lead to trouble inserting or retrieving the sheath and a more traumatic interaction with the vessel.

In certain aspects, to avoid this problem, the outer layer can be bonded to the inner liner of an expandable sheath to prevent movement of the outer layer along the sheath shaft.

In certain aspects, the bonding of the inner liner and the outer layer can occur anywhere. In yet further aspects, such bonding can occur on an area of the sheath cross section where minimal stretching of the outer layer during expansion is set to occur. The bonding is also done in a location where no lubrication is applied.

Thus, disclosed herein are aspects where at least a portion of an innermost surface of the outer layer is bonded to at least a portion of an outermost surface of the inner liner. Such exemplary aspects are also depicted in **FIGS. 27A and 27B****.** It is understood that such a bond can be optionally present in any one of the disclosed above sheath configurations. The bonding can be done by any method known in the art. In some aspects, the bonding is done by laser welding, compression bonding, and/or selective ultrasonic welding.

In certain aspects, the bonding of the outer layer to the inner liner can occur on a section of the sheath cross section where the outer layer is not expected to stretch much or move relative to the inner liner during expansion. In yet further exemplary and unlimiting aspects, the portion of the inner member directly next to the end of its' outside layer meets this criterion. As shown in **FIGS. 27A-27B****,** the bonding **2790** is done between the inner liner **2702** and the outer layer **2708** at a portion that is not expected to stretch much or move relative to the inner liner. In such aspects, lubrication **2707** is not applied in this location on the inner liner to ensure a good bond between the two components. At this location, the outer layer can be bonded in place along the length of the sheath shaft to prevent longitudinal movement over the inner member. In yet other aspects, the outer layer can be bonded to the inner liner at a portion of the sheath. Since the bond as disclosed herein does not hinder the inner liner movement relative to the outer layer or the stretching of the outer layer itself, the force required to expand the sheath is not adversely affected by the bond.

In still further aspects, it is understood that the bond location can be important to minimize push forces. Yet, in other aspects, the bond covers a relatively small portion of the sheath circumference. In yet other aspects, the methods of making the bond need to be precisely controlled, and repeatability of the process needs to be ensured. The specific methods of making the bond are described in detail below.

In still further aspects, two or more portions of the inner liner and the outer layer can be bonded together. In such exemplary aspects, the bond can be formed in a predetermined pattern. In yet further aspects, the bonding pattern can be aligned to the lubricant pattern. In such aspects, the bonding can be done at any portion of the sheath where the lubricant is not present.

It is understood that when the sheath is used to deliver a prosthetic device into a patient's vessel, hemostasis can be compromised if the blood from the patient's arteriotomy penetrates in between the inner liner and the outer layer. In such a scenario, the outer layer is the only sheath element that resists blood pressure and maintains hemostasis.

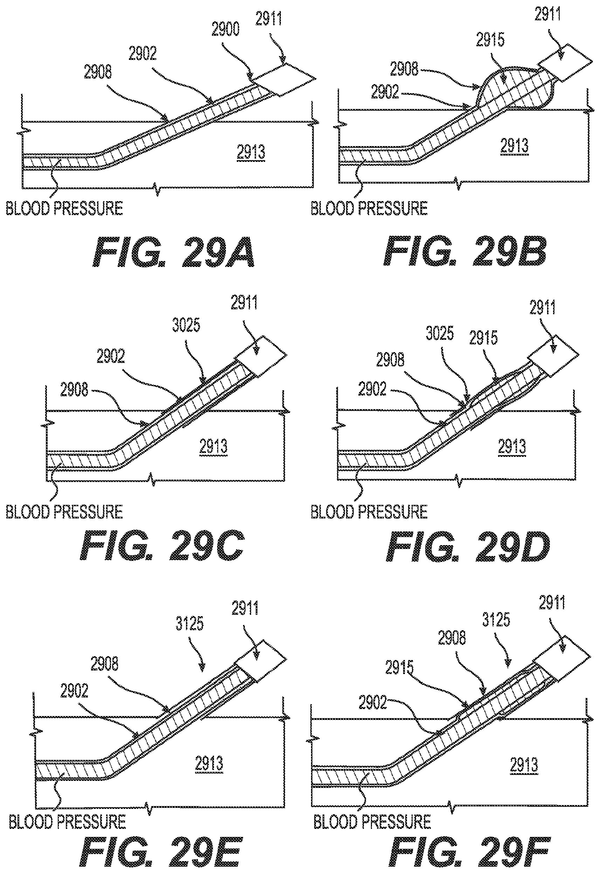

The outer layer is usually formed from the materials that, on the one hand, allow easy expansion of the inner liner and, on the other hand, can apply inward force on the inner liner to contract it to the original unexpanded configuration. However, high enough blood pressure may cause the outer layer to "balloon" and, at some point, even burst and compromise hemostasis. The schematic representation of such phenomena is shown in **FIGS. 29A-B.** An exemplary sheath **2900** having an inner liner **2902** and outer layer **2908** is connected to a hub **2911** and is inserted into the patient anatomy **2913.** When hemostasis is not maintained, there is a possibility that the outer layer will "balloon" **2915** and undesirably affect the patient.

For the portion of the sheath that is under the skin level of the patient, the potential ballooning can be contained by the tissue surrounding the sheath and resists the blood pressure, but for the portion of the sheath that is outside the patient, this undesired phenomenon can still occur. The portion of the sheath that stays outside the patient's body varies and depends on the patient's size and anatomy, as well as on the physician's preferences.

Making the outer layer material stiff enough such that it will resist the blood pressure and will not balloon may have an undesired tradeoff of increasing the force required to expand the inner liner and thus increasing the force required to advance a delivery system through the sheath.

The aspects described herein address this issue and help prevent excessive outer layer ballooning on the portion of the sheath that stays outside the patient's body while having a minimal effect on the force to expand the sheath.

The aspects described herein are aimed to reinforce the outer layer of the sheath along its proximal section without having a significant impact on its ability to expand while the delivery system is being pushed through the sheath. It is understood that in some aspects described herein, the reinforced portion of the outer layer can fully remain outside of the patient arteriotomy. Yet, in other aspects, at least a portion of the reinforced portion can be inserted in the patient's vessel. Some exemplary schematics of the disclosed aspects are shown in **FIGS. 29C-D,** wherein the reinforcing layer **3025** is disposed on the outer layer **2908** and substantially prevents the "ballooning" effect. As shown in **FIG. 29D****,** the reinforcing portion of the sheath can be long enough that at least a portion of this reinforcing portion is inserted into the patient's anatomy **2913.**

In such aspects, reinforcing the outer layer at the proximal portion of the sheath can be achieved by disposing a reinforcing jacket with a proximal end and a distal end on at least a portion of the outer layer. The reinforcing jacket, as disclosed herein, can comprise an elastomeric material and a reinforcing element. This reinforcing jacket is then positioned on the proximal portion of the outer layer. In such aspects, the end of the reinforcing jacket is substantially seamlessly bonded to at least a portion of the outer surface of the outer layer. This smooth transition between the outer layer and the reinforcing jacket allows the sheath to be inserted into the patient's body.

In still further aspects, the proximal end of the reinforcing jacket can be bonded to the proximal end of the outer layer. However, also disclosed are the aspect where the proximal end of the reinforcing jacket is not bonded to the proximal end of the outer layer.

In certain aspects, the reinforcing jacket can have a length from about 5 to about 15 cm, including exemplary values of about 6 cm, about 7 cm, about 8 cm, about 9 cm, about 10 cm, about 11 cm, about 12 cm, about 13 cm, and about 14 cm.

In still further aspects, the elastomeric material present in the reinforcing jacket can be any elastomer known in the art. In some aspects, the elastomeric material comprises a polyether block amide, styrene-based elastomer, polyurethane, latex, copolymers thereof, blends thereof, or co-extrudates thereof. In still further aspects, the elastomer can include silicon-based elastomers.

In still further aspects, the elastomeric material can have a Shore hardness of about 10A to about 80A, including exemplary values of about 20A, about 25A, about 30A, about 35A, about 40A, about 45A, about 50A, about 55A, about 60A, about 65A, about 70A, and about 75A.

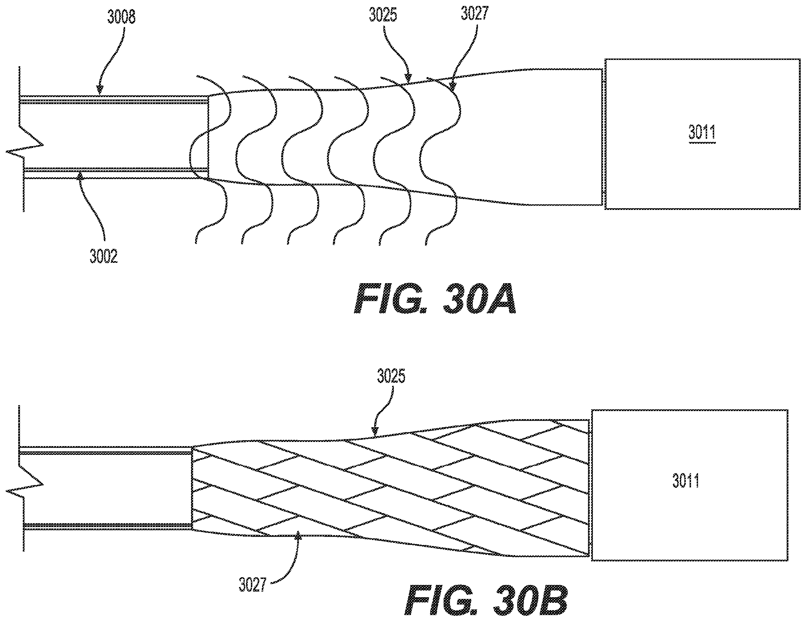

**In** still further aspects, the reinforcing jacket comprises a reinforcing element. Some exemplary schematics of various reinforcing jackets are shown in **FIGS. 30A-B.** The reinforcing jacket **3025** is seamlessly bonded at the distal end of the reinforcing jacket to the outer layer **3008** that is disposed on the inner liner **3002.** The proximal end of the reinforcing jacket may or may not be bonded to the inner liner and/or hub **3011.** The reinforcing filament **3027** is disposed within the elastomer. The reinforcing element that has a minimal impact on the resistance of the outer layer to expand up to a certain diameter in which its resistance increases dramatically.

When a delivery system is pushed through the sheath, it requires only a limited amount of expansion to accommodate the OD of the crimped valve. This limited expansion will not engage the reinforcing element, so the impact on the force to expand the sheath up to this point will be minimal and only be derived from the low durometer elastomer. In case a ballooning phenomenon starts, the diameter of the outer layer and reinforcing layer will increase only until the reinforcing element come into effect and prevents excessive ballooning.

In certain aspects, the reinforcing element can comprise a plurality of filaments arranged in a braid configuration. In such aspects, the plurality of filaments can be disposed in a plurality of circumferential rows within the reinforcing jacket, wherein each of plurality has a sinusoidal form or any irregular form, or any combination thereof. In certain aspects, the braid or coil can be an expandable braid or coil.

In yet further aspects, the plurality of filaments can comprise stainless steel, nitinol, a polymer material, or a composite material. In certain unlimiting aspects, the filaments can comprise Nitinol and/or other shape memory alloys. In yet other unlimiting aspects, the filaments can comprise polyester or nylon. In yet some other exemplary aspects, the filaments can comprise spectra fiber, polyethylene fiber, aramid fiber, or combinations thereof.

Some exemplary aspects of the braid or coil configurations are shown in **FIGS. 4A-4D****.** In certain aspects, the braid or coil can be a generally thin, hollow, substantially cylindrical tube comprising an arrangement, pattern, structure, or configuration of filaments or struts, however other geometries can also be used. Suitable filaments can be round, having a diameter less than about 0.015", less than about 0.01", less than about 0.008", less than about 0.005", less than about 0.002", less than about 0.001", less than about 0.0008", or less than about 0.0005". In yet other aspects, suitable filaments can be round and having a diameter ranging from about 0.0005" inches thick to about 0.015" thick, including exemplary values of about 0.0006", about 0.0007", about 0.0008", about 0.0009", about 0.001", about 0.002", about 0.003", about 0.004", about 0.005", about 0.006", about 0.007", about 0.008", about 0.009", about 0.01", about 0.012", about 0.013", and about 0.014". In yet other aspects, the suitable filaments can be flat filaments having a height of less than about 0.006", less than about 0.005", less than about 0.004", less than about 0.003", less than about 0.001", less than about 0.0009", less than about 0.0008", less than about 0.0007", less than about 0.0006",and about 0.0005". In yet other aspects, the flat filaments can have a width from greater than about 0.003" to about 0.015", including exemplary values of about 0.004", about 0.005", about 0.006", about 0.007", about 0.008", about 0.009", about 0.01", about 0.012", about 0.013", and about 0.014". However, other geometries and sizes are also suitable for certain aspects.

In yet further aspects, the braid can have a per-inch crosses (PIC) count of less than 50, less than 40, less than 30, less than 20. or less than 10. In yet other aspects, the braid can have the PIC count from 10 to 2, including exemplary values of 9, 8, 7, 6, 5, 4, and 3. In still further aspects, the PIC can vary along the longitudinal axis of the lumen. In yet other aspects, the braid pattern can vary along the longitudinal axis of the lumen. In the aspects where the braid or coil comprises filament that is nitinol, the nitinol is a heat-set at the expanded diameter *dₑ.* In yet further aspects, where the filament comprises stainless steel or nitinol, the filament is configured to be atraumatic, at least at the distal end of the sheath. **FIGS. 4A-4D** illustrate partial elevation views of various structures for the braid or coil **28.** It is understood that the structure of the braid or coil **28** can vary from section to section, changing along the length of the sheath. It is further understood that the structures shown in **FIGS. 4A-4D** are not necessarily drawn to scale and show just exemplary and unlimiting aspects. It is further understood that the braid or coil is configured to provide the torquability of the sheath during the insertion of the prosthetic device.

Yet, in other aspects, the reinforcing element can comprise wires disposed in a plurality of circumferential rows embedded within the elastomeric material. In such aspects, the wires can have any shape that is configured to expand or contract. For example, the wires can have a sinusoidal or waveform. The phase and amplitude of the wire can vary depending on the desired application. Also, it is understood that the frequency and the total number of the circumferential rows present in the elastomeric material can also vary depending on the desired application. The expansion of the wires will allow the outer layer of the sheath to continue to expand until the reinforcing element present in the elastomeric material is tightened. In this configuration, the reinforcing jacket stops further expansion and thus maintains hemostasis.

In still further aspects, a tie layer can be disposed between the reinforcing jacket and the outer layer of the sheath.

An additional configuration of the reinforcing jacket is also disclosed. In this aspect, the outer layer does not extend to the proximal end of the sheath and wherein at least a portion of the inner liner at the proximal end of the sheath is substantially free of the outer layer. This proximal portion of the sheath that is not covered by the outer layer can be any length. In some aspects, this proximal portion is from about 5 to about 15 cm, including exemplary values of about 6 cm, about 7 cm, about 8 cm, about 9 cm, about 10 cm, about 11 cm, about 12 cm, about 13 cm, and about 14 cm.

A proximal portion of such an outer layer can be bonded to the inner liner to ensure that no spacing between the inner liner and the outer layer is present when the sheath is inserted into the patient's body. This bond can also prevent the outer layer from sliding off when the sheath is inserted into the patient's body. In such aspect, the reinforcing jacket having a proximal end and a distal end is positioned then over the at least a portion of the outer surface of the inner liner at the proximal end of the sheath that is substantially free of the outer layer. It is understood that in such configuration, the proximal end of the reinforcing jacket is abutting the proximal end of the sheath and is at least partially bonded to at least a portion of a proximal end of the outer surface of the inner liner.

Again, the reinforcing jacket can have a length substantially similar to a length of the proximal portion of the sheath that does not have an outer layer. In some exemplary and unlimiting aspects, the length of the reinforcing jacket can be from about 5 to about 15 cm, including exemplary values of about 6 cm, about 7 cm, about 8 cm, about 9 cm, about 10 cm, about 11 cm, about 12 cm, about 13 cm, and about 14 cm.

In still further aspects, the distal end of the reinforcing jacket is abutting or at least partially overlays a proximal portion of the outer layer. It is further understood that the distal end of the reinforcing jacket is seamlessly bonded to the at least a portion of the proximal portion of the outer surface of the outer layer. It is understood that the reinforcing jacket, as described in this aspect, can comprise all components of the reinforcing jacket disclosed above.

In still further aspects also disclosed the sheath having any one of the disclosed above configurations and comprising a ballooning guard. Similar to the reinforcing jacket disclosed above, the balloon guard is configured to prevent excessive outer layer ballooning on the portion of the sheath that stays outside the patient's body while accommodating the different insertion depths of the sheath and not affecting the force to expand the sheath. The ballooning guard is configured to stay outside of the patient's body and not to be inserted into the patient's anatomy.

In the aspects disclosed herein, the ballooning guard has a proximal end and a distal end and is disposed over at least a portion of the outer layer and wherein the ballooning guard is configured to remain outside of a subject's blood vessel and to substantially maintain hemostasis.

The ballooning guard, as described in the current disclosure, can be collapsible. In yet other aspects, the ballooning guard can be configured such that its length can be adjusted based on the insertion depth of the sheath.

In some aspects, the inner diameter of the ballooning guard can be big enough or have minimal resistance to expansion up to a certain diameter, such that it is not affecting the force to expand the sheath nor the force to advance the delivery system through the sheath. Once the ballooning guard reaches a certain diameter, the force to expand the ballooning guard increases significantly, such that it can resist the blood pressure and stop the ballooning. It is understood that while the ballooning guard may not prevent the initiation of the ballooning, it can contain it such that the outer layer will not over expand, burst, and compromise hemostasis. Some exemplary schematics of the balloon guard are shown in **FIGS. 29E-29F****.** The ballooning guard **3125** is disposed over the outer layer **2908** such that while the blood **2915** maybe can enter into the portion between the outer layer **2908** and the inner layer **2902,** it does not cause a burst of the sheath and helps maintain hemostasis.

In still further aspects, the proximal end of the ballooning guard is connected to a most proximal portion of the outer layer and/or a hub of the sheath. While the distal end of the ballooning guard radially circumscribes at least a portion of the outer layer and wherein the distal end is not bonded to the outer layer. It is understood that the ballooning guards, as described herein, seal against a subject's skin and are not inserted into the subject's anatomy **2913.**

In still further aspects, the ballooning guard can comprise a braided or coiled sleeve comprising a plurality of filaments. Any of the disclosed above plurality of filaments can be utilized. In some aspects, where the braided or coiled sleeve is present, at least a portion of the plurality of the filaments at the distal end of the ballooning guard are coupled to each other to allow shortening of the braided or coiled sleeve and sealing against the outer layer and against the skin of the patient.

In still further aspects, the braided or coiled sleeve can also comprise a polymer. Any of the disclosed above elastomeric polymers can be used. In certain aspects, the braid or coil can be embedded with the polymer. It is understood that the braid or coil material can be any material known in the art. In certain aspects, the braid or coil material can comprise metal or metal alloys. In certain aspects, any known in the art metals or metal alloys used in medical devices can be utilized to make a braid or coil. In some aspects, the braid or coil can be made of a memory shape material. In still further aspects, the braid or coil can be any braid or coil as disclosed herein.

In yet other aspects, the braid material can comprise a polymer. It is understood that any known in the art polymers can be used to form a braid. In such exemplary and unlimiting aspects, the polymer can comprise any known polyolefin, any known polyamide, or any known polyester. In still further aspects, the braid material can comprise a fabric. In still further aspects, the braided sleeve can comprise a fabric. Yet, still, in further aspects, the ballooning guard can comprise the e-PTFE tubing, the ballooning guard comprises the e-PTFE tubing, corrugated tubing, or any polymeric tubing having a shape that is configured to be compressed. In certain aspects, the ballooning guard can comprise the e-PTFE tubing. In such exemplary and unlimiting aspects, the e-PTFE tubing can be compressed without it losing its shape. While in still further aspects, the ballooning guard can comprise a corrugated tubing. The corrugated tubing allows the guard to be compressed and to adjust its length.

In such exemplary and unlimiting aspects, the ballooning guard can be made of any polymeric material that can have a shape that allows this material to be compressed. For example, a tubing can be formed from any polymer known in the art. The wall of this tubing can be cut in a pattern that allows it to be compressed without losing the initial shape.

Additionally, some aspects of any of the sheath configurations disclosed herein can include an exterior hydrophilic coating on the outer surface of the outer layer. Such a hydrophilic coating can facilitate insertion of any of the sheaths disclosed herein into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other coatings (e.g., PTFE, polyethylene, polyvinylidene fluoride), are also suitable for use with the sheath.

Also, soft tip **102,** as shown in **FIG. 11****,** can be utilized with any of the disclosed herein sheath configurations. In certain aspects, the tip can comprise low-density polyethylene (LDPE) and can be configured to minimize trauma or damage to the patient's vessels as the sheath is navigated through the vasculature. For example, in some aspects, the soft tip portion **102** can be slightly tapered to facilitate passage through the vessels. The soft tip portion **102** can be secured to the distal end **104** of the sheath **100,** such as by thermally bonding the soft tip portion **102** to the inner and outer layers of the sheath **100.** Such a soft tip portion **102** can be provided with a lower hardness than the other portions of the sheath **100.** In some aspects, the soft tip **102** can have a Shore hardness from about 25 A to about 40 A, including exemplary values of about 28 A, about 30 A, about 32 A, about 35 A, and about 38 A. It is further understood that Shore hardness can have any value between any two foregoing values. In yet other aspects, the soft tip **102** can have a Shore hardness from about 25 D to about 40 D, including exemplary values of about 28 D, about 30 D, about 32 D, about 35 D, and about 38 D. The tip portion **102** is configured to be radially expandable to allow a prosthetic device to pass through the distal opening of the sheath **100.**

As shown in **FIG. 11****,** the sheath **100** can optionally include at least one radiopaque filler or marker, such as a discontinuous or C-shaped band **112** positioned near the distal end **104** of the sheath **100.** The marker **112** can be associated with the inner liner and/or outer layer **108, 110** of the sheath **100.** Such a radiopaque tip marker can comprise materials such as those suitable for the radiopaque filler, platinum, iridium, platinum/iridium alloys, stainless steel, other biocompatible metals, or combinations thereof. Suitable materials for use as a radiopaque filler or marker include, for example, barium sulfite, bismuth trioxide, titanium dioxide, bismuth subcarbonate, or combinations thereof. The radiopaque filler can be mixed with or embedded in the layer of the elastomeric polymer used to form the outer layer and can comprise from about 5% to about 45% by weight of the outer layer, including exemplary values of about 10%, about 15%, about 20%, about 25%, about 30%, about 35%, and about 40% by weight of the outer polymeric tubular layer. The more or less radiopaque material can be used in some aspects, depending on the particular application.

The disclosed herein sheath can be configured such that it locally expands at a particular location corresponding to the location of the medical device along the length of the lumen and then locally contracts once the medical device has passed that particular location. Thus, a bulge may be visible, traveling longitudinally along the length of the sheath as a medical device is introduced through the sheath, representing continuous local expansion and contraction as the device travels the length of the sheath. In some aspects, each segment of the sheath can locally contract after removal of any radial outward (insertion) force such that it regains the original resting diameter of lumen *dᵣ.*

In some aspects, each segment of the sheath can locally contract after removal of any radial outward force such that it at least partially returns to the original resting diameter of lumen *dᵣ.*

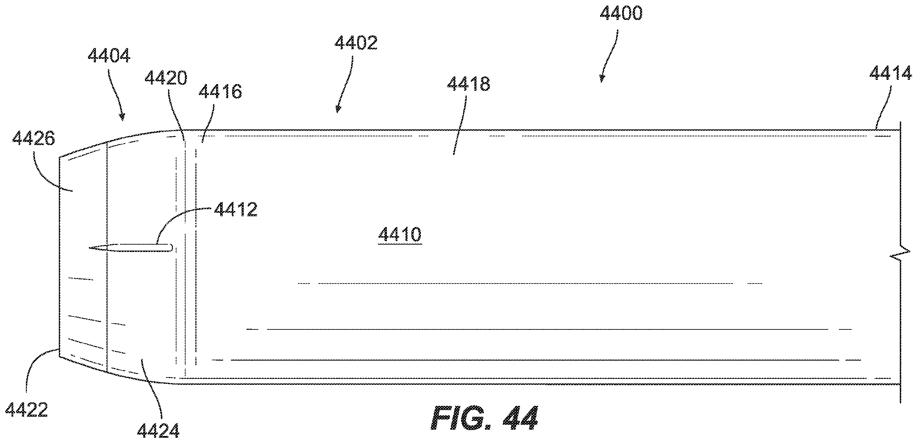

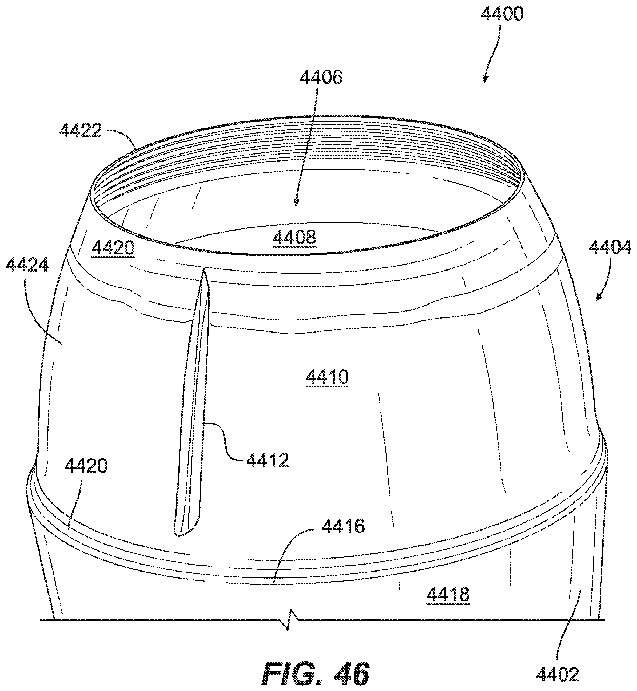

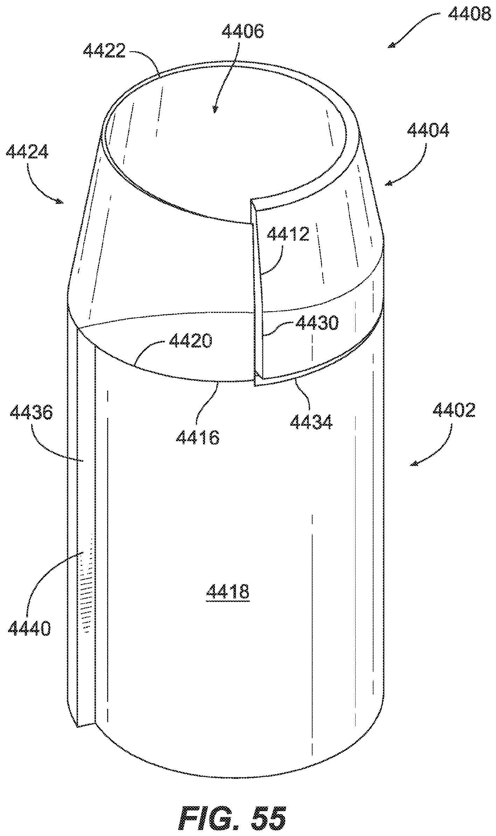

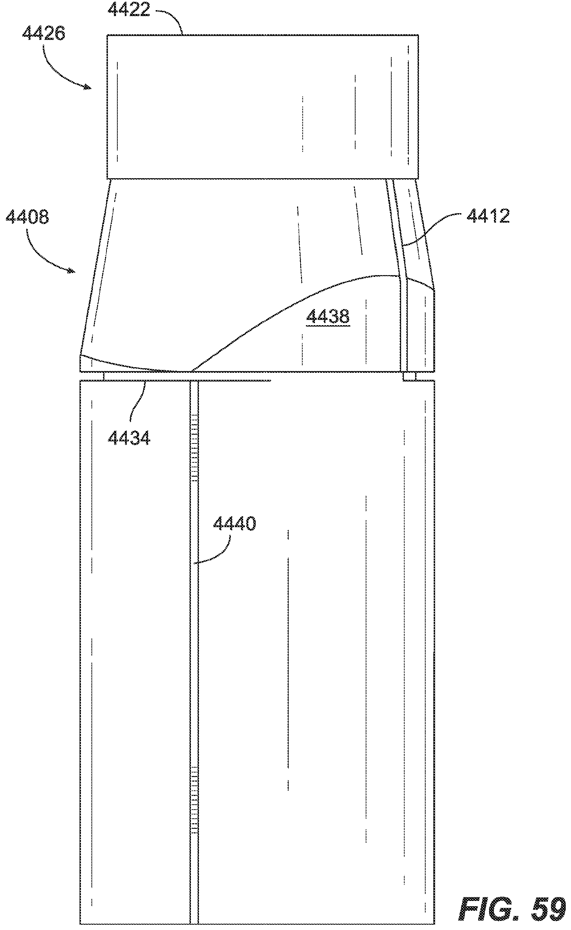

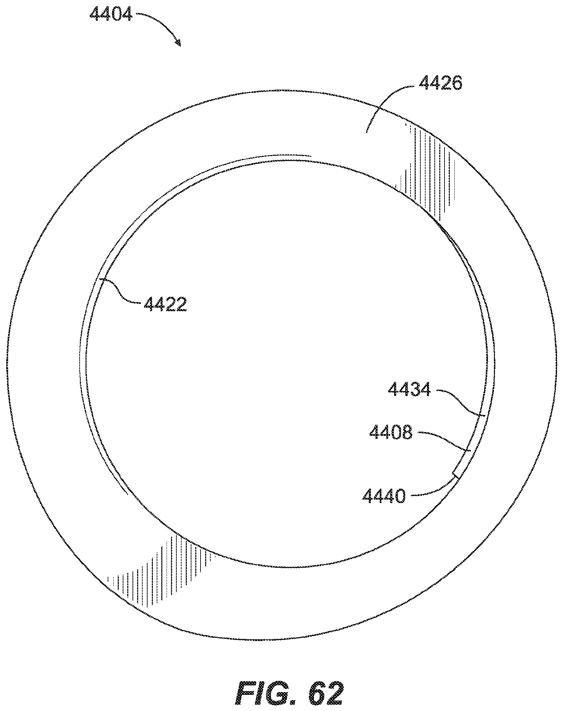

**FIGS. 44-66** disclose examples of expandable sheaths 4400 according to the invention and methods of making the same that include a proximal section 4402 and a distal tip section 4404 that can be permanently split to facilitate retrieval of medical devices. The sheath 4400 defines a lumen 4406 extending therethrough for passage of the medical devices, such as those described hereinabove. The proximal section 4402, for example, may include a scroll or helical type expandable inner liner layer 4408 that has a slit therealong, allowing its edges to overlap. The inner liner layer 4408, under bias from a surrounding elastomeric jacket or outer layer 4410, locally expands and collapses as the medical device is passed therethrough. The tip section 4404 extends from a distal end 4416 of the proximal section 4402 and defines a slit 4412 that extends most of the length of the tip section except, in some cases, a distal end 4422 of the tip section. The slit 4412 also extends through most or all of the layers of the tip section 4404. In this manner, the portion of the lumen 4402 defined through the tip section 4404 can be expanded by passage of the medical device therethrough.

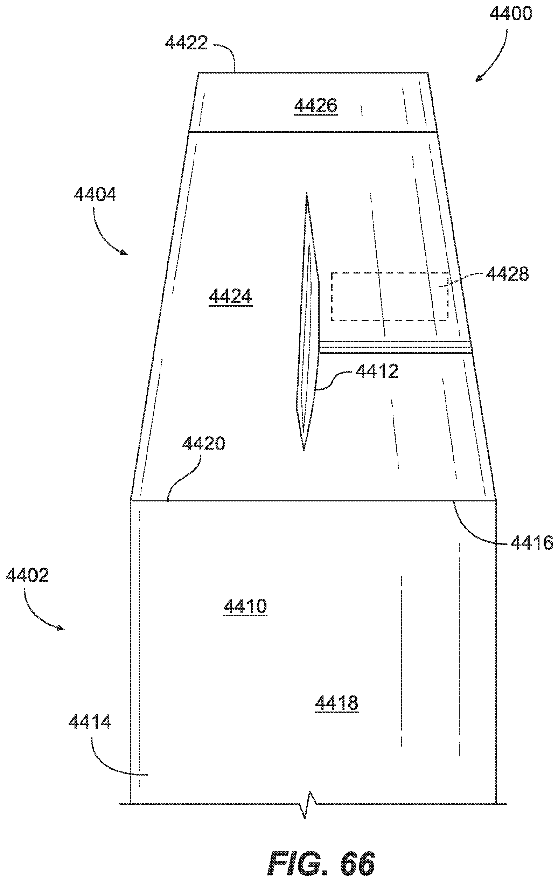

Advantageously, the slit 4412 in the tip section 4404 presents a low push force of less than about 25 Newtons (5.6 pounds-force). or in another aspect, less than about 15 Newtons (3.4 pounds-force), for opening and remains open to facilitate, when necessary, partial or full retrieval of the medical device. The elastomeric outer layer 4410 extends over all or part of the tip section and contributes to smoothing the surface, maintaining hemostasis and the atraumatic nature of the tip section 4404. Also, the expandable sheath 4400 may include a radiopaque marker 4428 embedded in the tip section 4404 for improved positioning of the expandable sheath, as shown in **FIG. 66****.**

In **FIGS. 44-46****,** for example, the proximal section 4402 includes an elongated body 4418 that extends between a proximal end 4414 and the distal end 4416. The elongated body 4418 defines a proximal portion of the lumen 4406 extending through the expandable sheath 4400. The tip section 4404 extends distally from the distal end 4416 of the proximal section 4402. The tip section 4404 includes a proximal end 4420, the distal end 4422, and a generally tapering tip body 4424 that extends between the proximal end 4420 and the distal end 4422. The tip body 4424 defines a distal portion of the lumen 4406 that extends through the expandable sheath 4400.

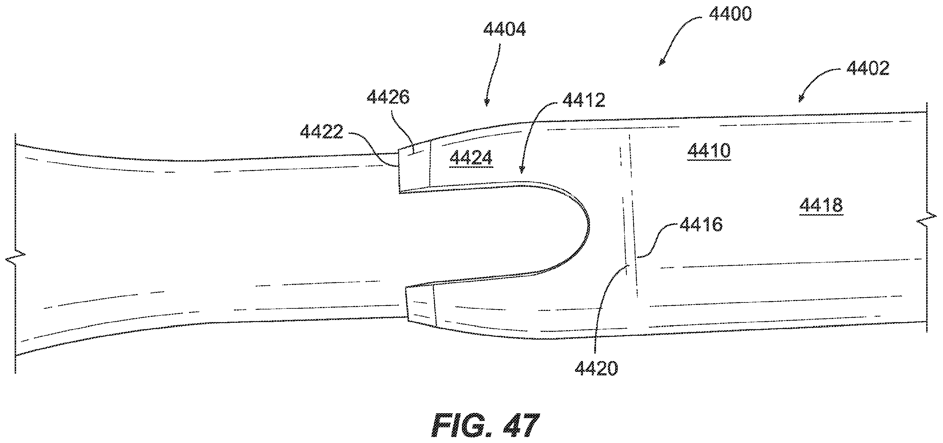

The tip section 4404 includes at least the inner liner layer 4408 defining the slit 4412 and some relatively softer structure that extends and/or coats the inner liner layer to make the tip section atraumatic such as the outer (jacket) layer 4410 and/or an intermediate layer 4426. Generally, in some aspects, the outer layer 4410 prevents blood loss through the inner liner layer 4408 and extends over the proximal section 4402 to bias the scrolling inner liner layer 4408 into a compressed configuration as described better hereinabove. In other aspects, the intermediate layer 4426 can be used to bond the inner liner layer 4408 to the outer layer 4410. (The intermediate layer 4426 may be the only thing holding the tip section 4404 together.) The intermediate layer 4426 can also mediate blood loss through the inner liner layer 4408. The intermediate layer 4426 can include its own sub-layers, such as two or three layers, including a tie layer. Unlike scored layers, most or all of the layers of the tip section 4404 may be slit fully through (and lightly sealed over with thermal reflowing) for reduced push forces and a cleaner, more well-defined expanded or split condition, as shown in **FIGS. 47-48****.**

The atraumatic tip section 4404 can be used in any catheter where a lubricious liner, such as an HDPE (high density polyethylene) inner liner layer 4408, and an elastomeric outer jacket, for example, a jacket comprised of copolyamide such as poly ether block amide (Pebax) outer layer 4410, benefits from thermal bonding with good adhesion by intermediate layer 4426. In some instances, the intermediate layer, since it extends the furthest distally with as a single layer, is the atraumatic part of the tip.

The inner liner layer 4408 can be an extension of the same layer in the proximal section 4402 that performs the "scroll" function of having free edges, defined by a spiraling elongate slit that expands and contracts during passage of the medical device. The inner liner layer 4408 can have a generally stiffer composition than the other layers and can, for example, be comprised of a polyethylene such as a high-density polyethylene (HDPE) or low-density polyethylene (LDPE) and other relatively stiff and/or lubricious polymers described herein.

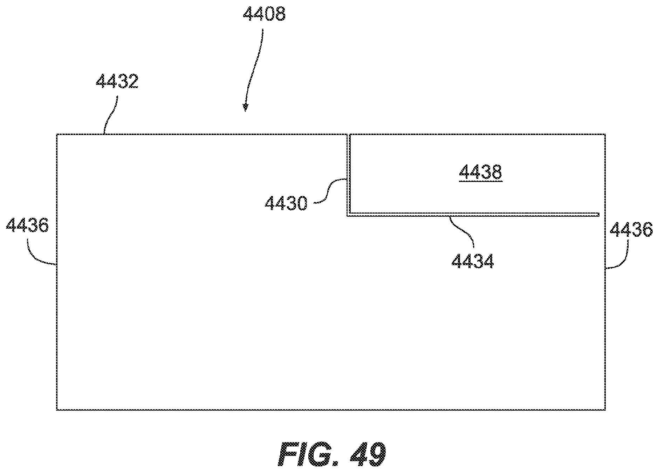

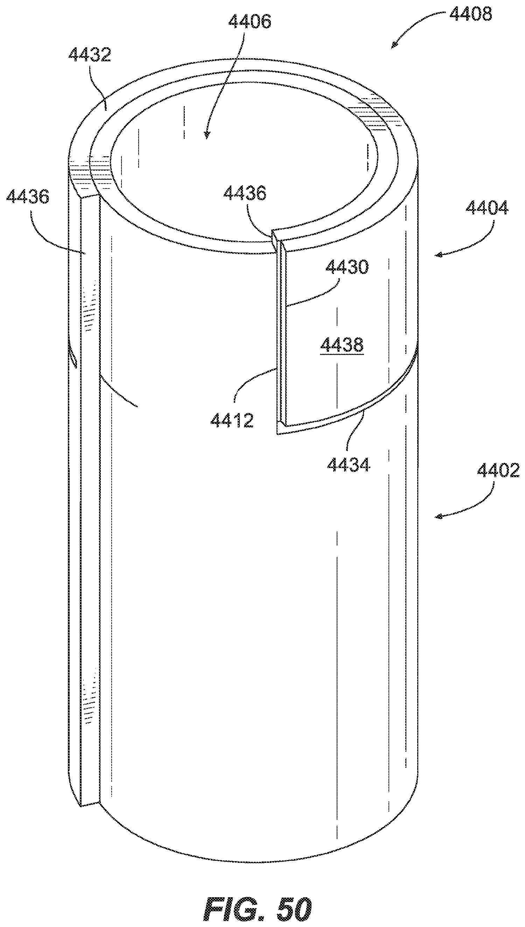

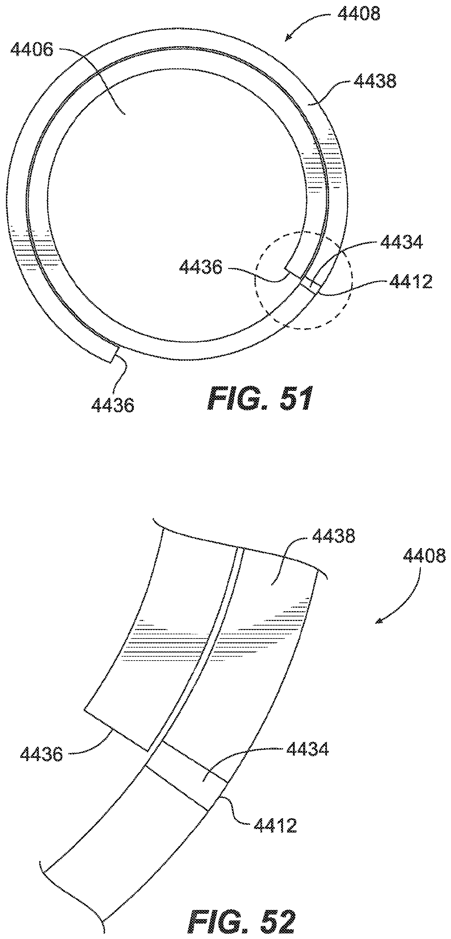

The inner liner layer 4408 at the tip section 4404 can be manufactured by modifying the distal end of the scrolled inner liner layer of the proximal section 4402. For example, as shown in **FIG. 49****,** the manufacturer cuts the inner liner layer 4408 with two distinct cuts. A first cut 4444 extends proximally from a distal edge 4432 of the inner liner layer 4408. A second cut 4434 extends from the proximal end of the first cut 4444 perpendicularly to one of a pair of lateral edges 4436 of the inner liner layer 4408. These two cuts form a flap 4438 that is then re-rolled into the scrolled configuration, as shown in **FIGS. 50-52****.** The cuts 4444, 4434 are positioned to define a portion of the slit 12 through the inner liner layer 4408. In particular, first cut 4444 is positioned to align with the lateral edge 4436 opposite the flap 4438 on the radially inner part of the scrolled configuration. Thus, the inner liner layer 4408, even in the collapsed condition, does not block the slit 12 from extending into the lumen 4406, as shown in **FIGS. 51 and 52****.**

The flap 4438 can have different sizes and shapes depending on the size and shape of the expandable sheath 4400, which in turn in related to the size, shape, and nature of the medical device being passed therethrough. However, for stent mounted heart valves and similar devices first cut 4444 may extend proximally about .157 inches (4 mm) and be positioned about .5 inches (12.7 mm) inward from the lateral edge 36. The second cut 4434 may extend therefrom .485 inches (12.3 mm) toward the lateral edge leaving about 0.015 inches (.38 mm) uncut and connecting the flap to the rest of the inner liner layer 4408. Thus, the flap has the rectangular dimensions of about 4 mm and 12 mm. The width of the inner liner layer 4408 between the lateral edges 4436 is about 1.068 inches (27.1 mm), and thus, the flap is about 40% to 50% of the width of the inner liner layer. The first cut 4444 generally will extend about 4 mm to 6 mm, or about 2/3 of the eventual length of the tip section 4404 once it is completely formed.

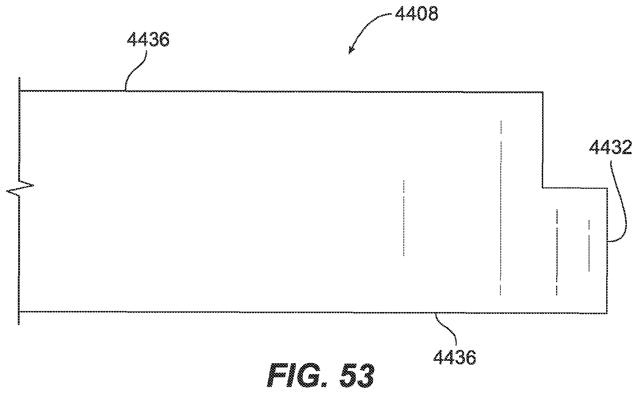

In some instances, additional or excess portions of the inner liner layer 4410 may be trimmed away as part of forming the tip section 4404. For example, an additional, small (2 mm to 3 mm or about 2.5 mm) rectangular piece can be removed from the distal edge 4432, as shown in **FIG. 53****,** to facilitate conical or tapered shaping. As described hereinabove for other aspects, the inner liner layer 4410, including in the proximal section 4402, may include lubricants to facilitate relative motion of the layers during expansion and contraction. Generally, though, the tip section 4404 will not include lubricant because it will be reflowed to form an integral structure.

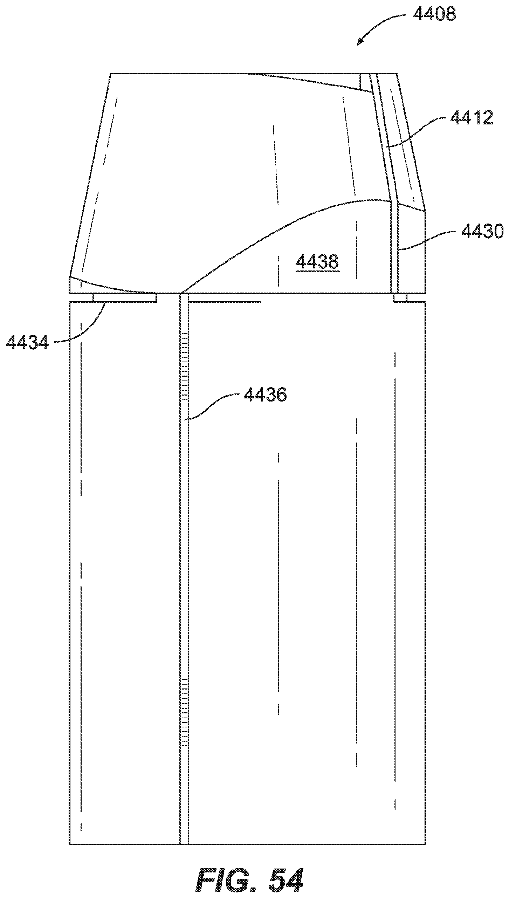

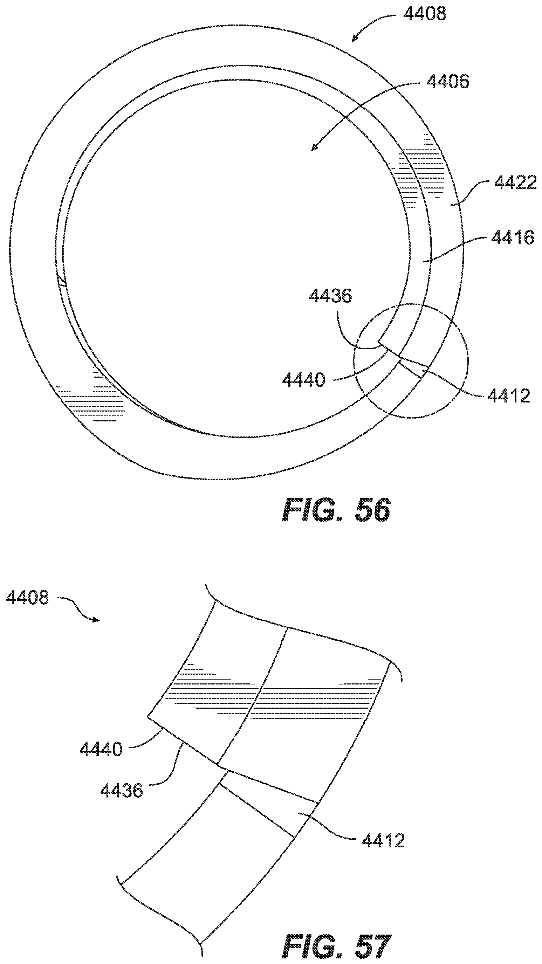

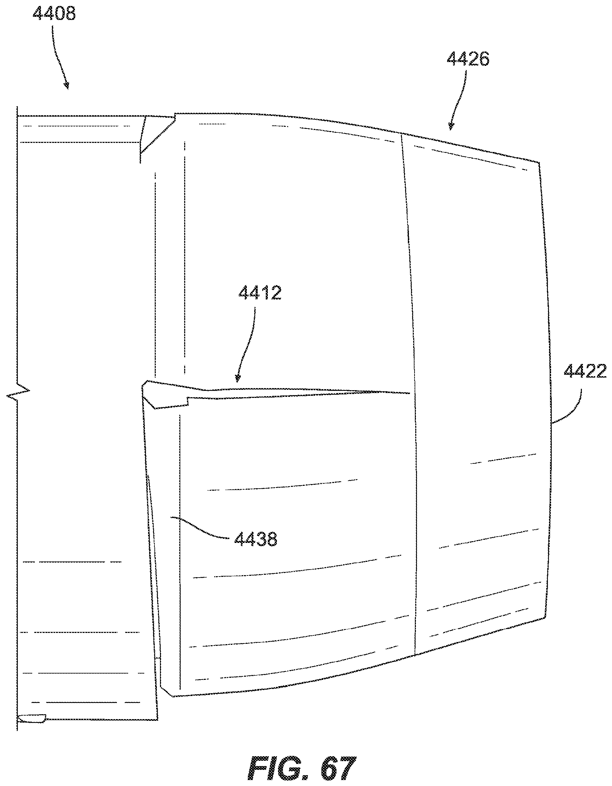

As shown in **FIGS. 54-57****,** the inner liner layer 4408 configuration of **FIGS. 50-****52** can be mounted on a mandrel or heated die and reflowed (with the application of heat) into a tapering conical shape (as shown in **FIG. 67****)** and then trimmed to present a relatively flat distal end. In this intermediate configuration shown in **FIG. 54****,** the tip section 4404 extends about 4 mm beyond the distal end 4416 of proximal section 4402. Also, the slit 4412 extends the whole length of the tapered conical shape. Also notable is that the proximal section 4402 still maintains the scroll configuration with an elongate edge 4440 for free expansion and contraction as described hereinabove. The two slits 4412 and 4440 are offset from each other circumferentially by about 44 degrees to 120 degrees or, in some aspects, 70 degrees to 120 degrees. The larger range reflects, for example, aspects of the sheath with greater overlaps of the rolled inner liner. Greater overlaps facilitate passage of larger medical devices, such as larger heart valves. However, as shown in **FIG. 56****,** at the portion of the overlapping inner liner layer 4408 at the distal tip section 4404, the scrolled inner liner layer has been reflowed to form an integral wall structure. In particular, the wall structure is thicker where the reflowed inner liner layer 4408 had an overlapping configuration in **FIGS. 50-52** and is thinner at the end of the flap 4438, where there was only a single layer of thickness. The wall thickness of the distal end of the inner liner layer 4408 is reduced at its thinnest point by heating to less than .005 inches (.13 mm) for about a circumferential length of about .080 inches (2 mm).

As shown in a plan view of **FIGS. 56-57****,** the slit 4412 has narrowed somewhat due to reflowing, and the adjacent overlapping layers of the inner liner layer 4408 have reflowed together to form a single wall thickness. However, the overlapping scroll configuration at the proximal section 4402 remains visible as the inner liner layer 4408 has not been reflowed proximal to the tip section 4404. In this manner, the inner liner layer 4408's ability to expand and contract in the proximal section 4402 of the expandable sheath 4400 is preserved.

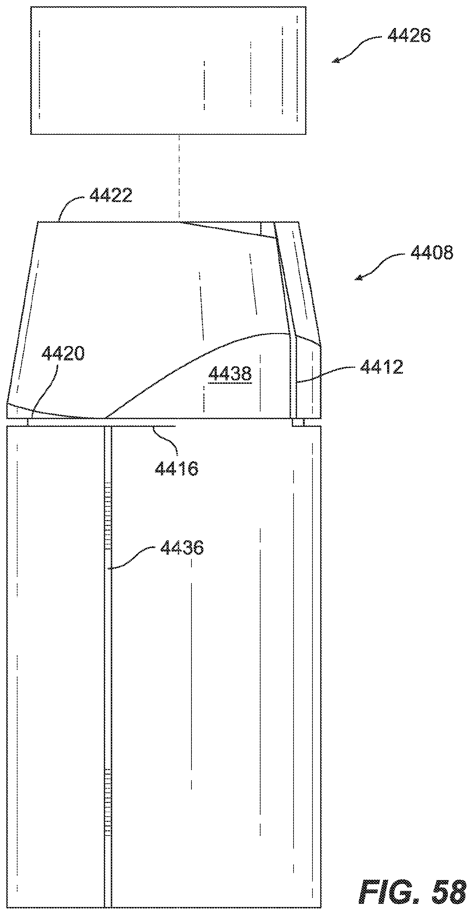

As shown in **FIGS. 58-62** **and** **67,** in one aspect, the next step in manufacturing the tip section 4404 is to apply the intermediate layer 4426 to the shaped and trimmed inner liner layer 4408 of **FIGS. 54-57****.** The intermediate layer 4426 is formed of a relatively soft thermoplastic that facilitates connection of the inner liner layer 4408 and the outer layer 4410 and is atraumatic. (In other aspects, no intermediate layer 4426 is necessary, and the layers 4408, 4410 can be coupled to each other via reflowing, adhesives, mechanical fasteners, etc.) The intermediate layer 4426 also reflows at a lower temperature and gives a larger bonding area for the elastomeric outer layer 4410, improving bond strength. Another advantage is that the intermediate layer 4426 can give the tip section 4404 a more lubricious inner diameter for easier deployment and retrieval of medical devices. The intermediate layer 4426 may also impart some structural stiffness to the distal end 4422 and avoid deformation such as ovalizing or "fish mouthing" at the distal end.

In one aspect, the intermediate layer 4426 extends distally by about 0.040 inches (or 2 mm to 3 mm) beyond the inner liner layer 4408 and the outer layer 4410. In other aspects, the intermediate layer 4426 extends up to about 0.080 inches distally of the layers 4408, 4410. The intermediate layer 4426 can be less stiff than the inner liner layer 4408, such that the intermediate layer 4426 is more easily bendable due to axial forces than the inner liner layer 4408.

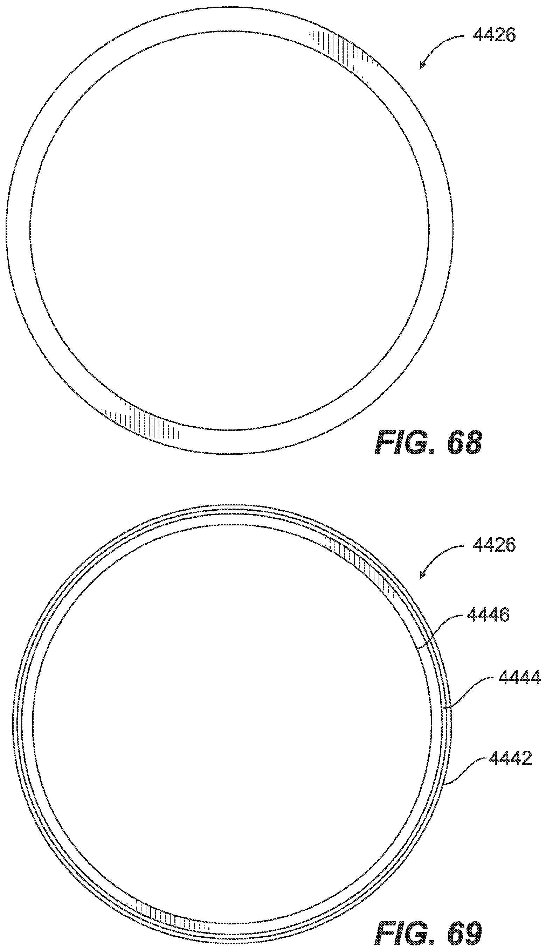

The intermediate layer 4426 can be a cylindrical single layer, such as a tie layer of about .012 inches (.3 mm) and an internal diameter of about .199 inches (5 mm), as shown in **FIG. 68****.** The intermediate layer 4426 can have several sub-layers, such as a bi-layer or a tri-layer, as shown in **FIG. 69****.** For sub-layers, the tube that is formed into the intermediate layer 4426 can be co-extruded. Materials for the intermediate layer 4426 and its (optional) sublayers include thin polymer tubing or film of thermally bondable tie layer like Orevac 18440M (Maleic Anhydride modified LLDPE), Orevac 9318, or Orevac 9444 (Maleic Anhydride modified EVA). These can be an intermediate sub-layer in a tri-layer or an outer layer in a bi-layer configuration.

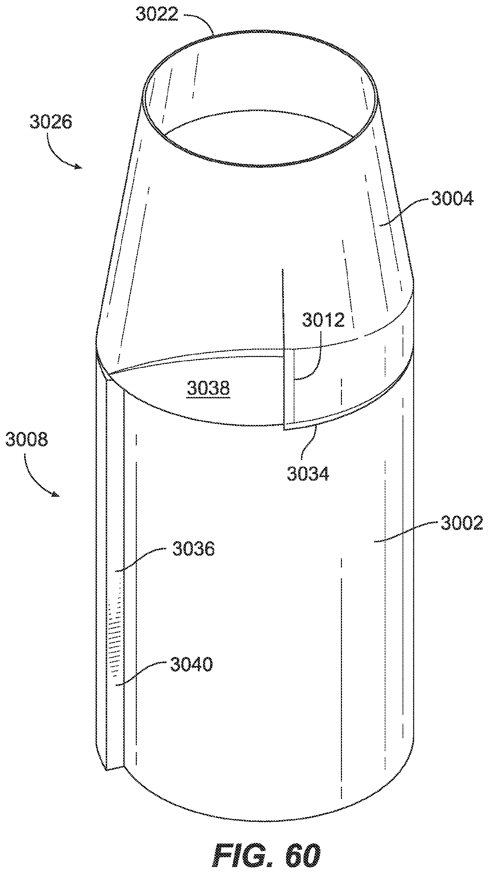

Referring again to **FIGS. 58-59****,** the intermediate layer 4426 is placed over the distal end of the conically shaped inner liner layer 4408. Then, the intermediate layer 4426 is heated and flows downward to coat the shaped inner liner layer 4408, forming the structure shown in **FIGS. 60-62** **and** **67.** Also, or alternatively, the intermediate layer 4426 may be heat shrunk over the inner liner layer 4408. Advantageously, reflowing the intermediate layer 4426 does not require use of a mandrel - it flows over the already - shaped surface of the inner liner layer 4408.

In another aspect, as shown in **FIG. 67****,** the intermediate layer 4426 does not entirely melt down and over the inner liner layer 4408. Instead, it extends beyond the inner liner layer 4408. For example, the thermally bondable tip tubing is cut to about .250 inches (6.35 mm) long, and about .160 inch (4 mm) of the thermally bondable tip tubing is overlapped to the .160 inches (4 mm) long thinned HDPE inner liner layer 4408 leaving several millimeters of the intermediate layer 4426 extending beyond the inner liner layer 4408. This extension can then be trimmed to 1 mm to 2 mm of tip beyond the inner liner layer 4408. Also, the now extended length of the tip section 4404 can be again cut or scored to refresh and extend the slit 4412 through the intermediate layer 4408 and extend it (if necessary) to about within .040 inches (1 mm) or .080 inches (2 mm) of the distal end 4402 of the tip section 4404. (Notably, this additional 1 mm to 2 mm remains uncovered by the outer layer 4410 when such an outer layer is employed.)

In other aspects, the intermediate layer 4426 can include two sublayers. For example, a thermally bondable tie sublayer can be formed by co-extrusion with an HDPE or LDPE inner sublayer. In one example, the inner sublayer can have an inner diameter of about .199 inches and a thickness of .010 +/- .001 inches and be comprised of an LDPE or an HDPE. The outer sublayer is .002 +/- .0005 inches and is comprised of a tie layer material.

In another aspect, the intermediate layer 4426 can include two sub-layers, such as an Orevac 18440M outer sub-layer to be bonded to the elastomeric outer layer 4410 and an LDPE inner sub-layer to be bonded to the inner liner layer 4408. The Orevac layer can have a thickness of about .002 inches, while the LDPE layer can have a larger thickness, such as .010 inches. Bonding the intermediate layer, for example, could be at 450 degrees Fahrenheit for about 1 minute with FEP heat shrink tubing.

In yet another aspect, the outer sub-layer may include Pebax 45D (Shore D) and an Orevac inner sub-layer. The Orevac inner sub-layer can have about a .200 inches inner diameter and a .006 inches thickness. The Pebax 45D outer sub-layer can also have about a .006 inches wall thickness. The outer sub-layer can be thermally bonded to the outer elastomeric layer 4410, such as an outer layer of Pebax 25D. The inner sub-layer can be thermally bonded to the inner liner layer 4408, such as an inner layer of HDPE.

As shown in **FIG. 69****,** in another aspect, the intermediate layer 4426 can include three sublayers, an outer sublayer 4442, an intermediate sublayer 4444, and an inner sublayer 4446. The inner sublayer can have a diameter that is larger than the intermediate and outer sublayers 4444 and 4442. For example, the inner sublayer 4446 can have a thickness of about .006 to .008 inches, the intermediate sublayer 4444 can have a thickness of about .002 to .003 inches, and the outer sublayer 4442 can have a thickness of about .002 to .003 inches. The inner sublayer 4446 may be comprised of a lubricious or relatively stiff polymer such as an HDPE or LDPE. The intermediate sublayer 4444 can be comprised of a tie layer material. The outer sublayer 4442 can be comprised of about Pebax 25D to 45D.

In yet another aspect, the inner sublayer 4446 can have a thickness of about .005 inches with an inside diameter of .200 inches, the intermediate sublayer 4444 can have a thickness of about .002 inches and an outer sublayer 4442 a thickness of about .005 inches. The inner sublayer 4446 can comprise, for example, an LDPE. The intermediate sublayer 4444 can comprise Orevac and the outer sublayer 42 can comprise Pebax 45D. Although the various layers disclosed herein for the intermediate layer 4426 can be mixed in to form different combinations of thicknesses and materials, the LDPE of the inner sublayer 4446 bonded well to an HDPE inner liner layer 4408 and the Pebax 45D outer sublayer 4442 bonded well to a Pebax 25D outer layer 4410. In addition, with the LDPE inner sublayer 4446 the tip section 4404 was easily removed from a stainless-steel shaping mandrel.

In yet another aspect, the intermediate layer 4408 includes an LDPE inner sublayer 4446 with an inner diameter of .200 inches and a wall thickness of .006 inches, an Orevac intermediate sublayer 4444 with a wall thickness of .003 inches, and a Pebax 25D outer sublayer 4442 with a wall thickness of .003 inches. Notably, for this aspect, the inner sublayer had double the thickness of the other sublayers.

In another aspect, the intermediate layer 4426 can be formed in a multi-layer coextrusion of and HDPE or LDPE inner sublayer 4446, an intermediate thermally bondable tie sublayer 4444, and Pebax 25D or 35D outer sublayer 4442.

The tie sublayer between the Pebax and LDPE sublayers may be Orevac. Other tie layers, alone and in combination, can also be used, such as functionalized olefin, for example, a maleic anhydride grafted ethylene vinyl acetate, polyolefin modified with acrylic acid, and other polar functional groups are possible.

As an additional aspect, the tip section 4404 of the expandable sheath 4400 may also include the radiopaque marker 4428. As shown in **FIG. 66****,** the tip section 4404 supports the marker 4428 very close to the tip - within the 6 mm length of the marker in aspects for delivering stent-mounted heart valves, for example. Having the marker in the tip section 4404 facilitates more accurate positioning of the medical device.

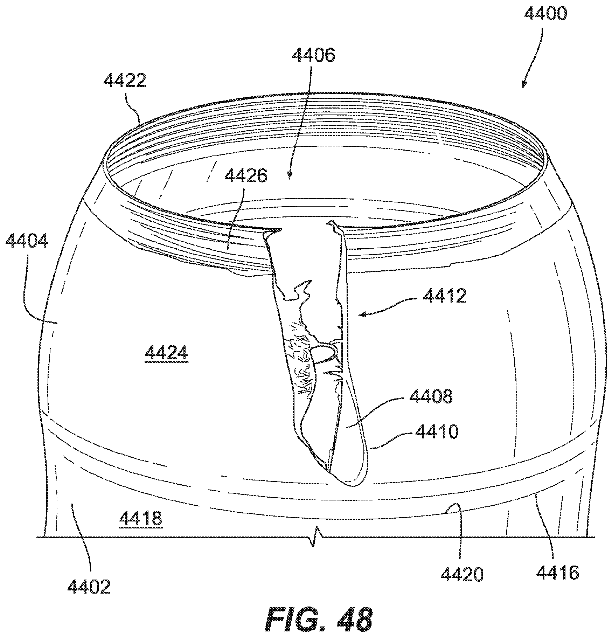

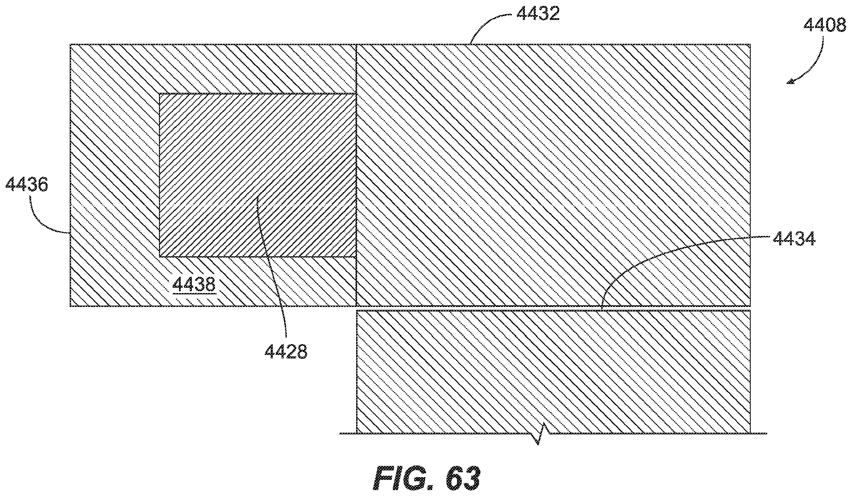

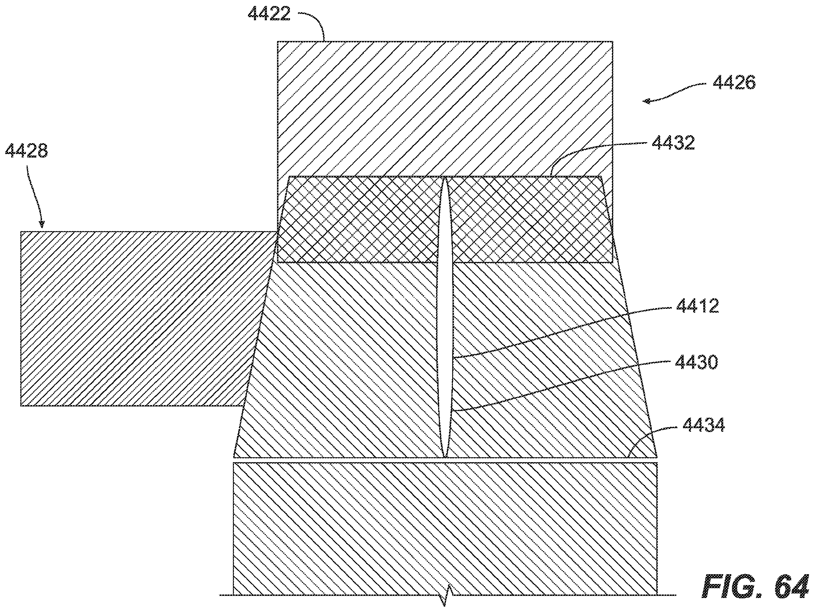

**FIGS. 63-65** schematically illustrate one example of incorporating the radiopaque marker 4428 into the tip section 4404. In particular, the marker may be incorporated into the inner liner layer 4410 under the flap 4438. As shown in **FIG. 63****,** when the inner layer is cut to the shape of **FIG. 48****,** the marker may be placed before the flap 4438 is folded back over the inner winding of the inner liner layer 4410. Then, as shown in **FIGS. 54-57****,** for example, the reflowing process and/or the heat shrinking process (with the intermediate layer 4426) can encapsulate the radiopaque marker 4428 into the inner liner layer. This also contributes to the thickened wall portion of the inner liner layer 4408 shown in **FIG. 56****.** Advantageously, the additional steps of heat shrinking the intermediate layer 4426 and the outer layer 4410 can additionally secure the radiopaque marker 4428. **FIGS. 64-65** show, in another aspect, formation of the distal end of the tip section 4408 by heat shrinking and trimming the intermediate layer 4426 to form another 1-2 mm of tip and application of the outer jacket layer 4410. The radiopaque marker is represented in **FIGS. 63-64** as not yet being folded into the inner liner layer 4408 to illustrate its relative positioning during the assembly process.

In another aspect, the radiopaque marker can be bonded between the intermediate layer 4426 and the inner liner layer 4408 or between the outer layer 4410 and the intermediate layer 4426. In any case, having the radiopaque marker in the tip section 4404 facilitates positioning of the expandable sheath 4400 by allowing more precise visualization.

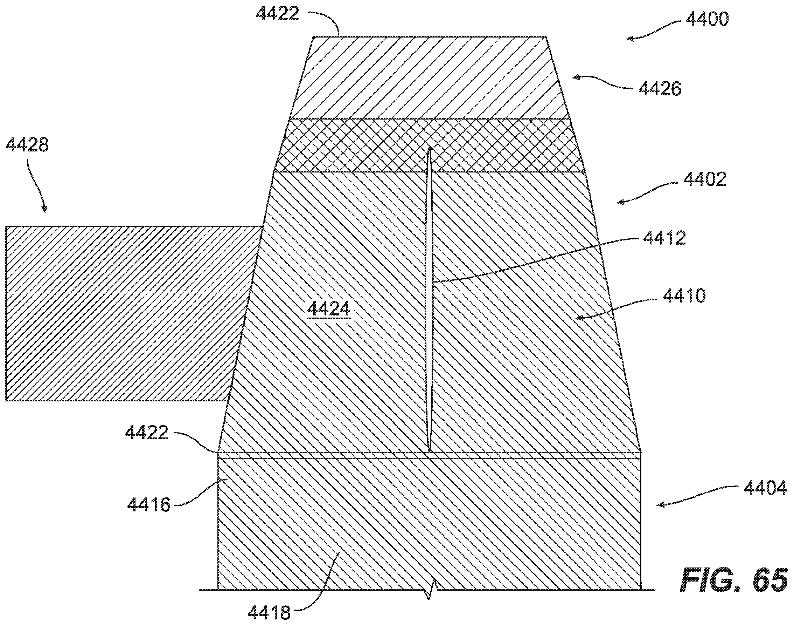

Generally, the radiopaque marker 4428 has a rectangular shape and is bent into a C-shape as it is folded into the tip section 4404. In one aspect, as shown in **FIG. 63****,** the radiopaque marker has an axial length of about 2.5 mm and is axially centered under the flap 4438, leaving, for example, .75 mm on either side for a 4 mm or 1.25 mm on either side for a 5 mm length of inner liner layer 4408 in a tip section 4404. As shown in **FIG. 64****,** the intermediate layer 4426 overlaps the trimmed and shaped inner liner layer 4408 about 1-2 mm and extends distally about 2-3 mm. As shown in **FIG. 65****,** the outer layer 4410 is applied, covering up about 1 mm of the intermediate layer 4426, and the distal portion of the intermediate layer is trimmed to about 1-2 mm.

As shown in **FIGS. 44-46****,** in one aspect, the outer elastomeric layer 4410 extends over the inner liner layer 4408 and intermediate layer 4426 of both the proximal section 4402 and the distal section 4404 of the expandable sheath 4400. For example, the outer layer 4410 is slid over the entire length of the rolled inner liner layer 4408 and tip section leaving about .040 inches to .080 inches (1-2 mm) of uncovered tip at the distal end of the sheath 4400, as shown in **FIG. 65****.** Generally, the thickness of the outer layer 4410 is about .005 inches. The outer layer may also have a taper to adapt it to the shape of the proximation and tip sections 4402, 4404. The taper can be formed due to heat shrinking with an FEP HS tubing over a tapered mandrel, for example.

The outer elastomeric layer 4410 can be a single layer, such as a polyurethane layer, or include multiple sublayers. For example, the elastomeric layer can include two sublayers, such as an inner layer of low durometer PEBA, such as Pebax from Arkema, having a Shore D durometer less than about 25-35. In another aspect, the outer layer 4410 can have a Shore D durometer from 35 to 65. And the outer layer can include a low durometer Polyurethane having a Shore A durometer of less than about 65 to 75. One example is Neusoft 597-50 from NEU Specialty Engineered Materials.

In another aspect, the outer elastomeric layer 4410 can be Neusoft 55A slid over the entire length of the rolled inner liner layer 4408 leaving the distal tip of the intermediate layer 4426 exposed. The layers are then heat shrunk for bonding. In one aspect, the outer layer 4410 may be bonded directly to the inner liner layer 4408, such as when the outer layer is comprised of the Neusoft, and the inner liner layer is comprised of HDPE. Also, outer layer 4410 can be pulled over the intermediate layer 4426 at the tip section 4404, and FEP shrink tubing slid over the outer layer to thermally bond the outer layer to the intermediate layer.

In another aspect, the elastomeric layer 4410 can be comprised of a Pebax 25D and be slid over the intermediate layer 4426 to facilitate heat bonding of the layers of the tip section. Such as described hereinabove, an optional lubricant such as Nusil may be applied to the outer surface of the inner liner layer 4408 to facilitate expansion and contraction of the inner liner layer 4408. However, generally, the tip section 4404 will not have lubricant because of the heat bonding of the layers.

In another aspect, the elastomeric layer 4410 can include a radiopaque filler, such as Barium Sulfate, for one layer or multiple sublayers.

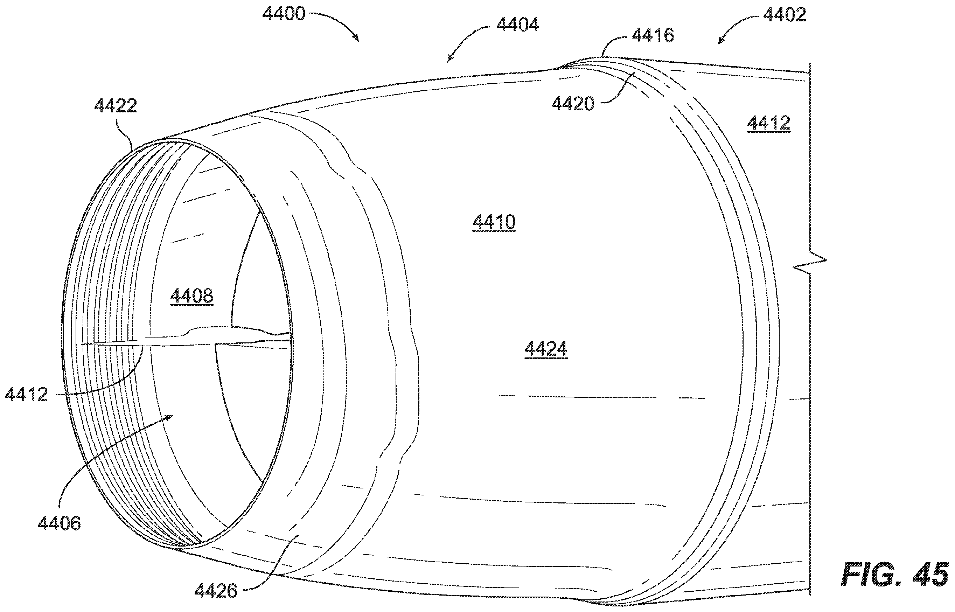

The slit 4412, as mentioned above for expandable sheath 4403 for cardiovascular applications, such as delivery of stent-mounted heart valves, has a length of about 3.8 mm to 4 mm and stops short 1-3 mm, in one aspect 2 mm, from the distal end of the tip section 4404. Thus, the slit 4412 is about 60% to 80% the length of the tip section 4404. (And the un-slit length is 20% to 40% of the length of the tip section 4404.) As shown in **FIGS. 45-46** and **65-66,** for example, the slit is linear in shape and can have a gap or can be partially sealed over by reflowing at various steps of construction, depending on how the manufacturer wants to modulate the push force needed to tear open the tip section 4404. In another aspect, the outer layer 4410 and intermediate layer 4426 can be split together in a single step, such as at the end of the manufacturing process.

Although the illustrated aspects only have a single linear slit 4412, the shape and number of slit(s) can be varied depending on how much or little push force is desired. Generally, more slits will reduce the push force. Slits may also have shapes that are wider at some points and narrower at others, for instance, if push force needed to be less at the proximal end 4420, then the slit could be wider or could bifurcate into two slits. Or two shorter slits could be used. Also, push forces could be higher by reducing the width or length of the slits, such as tapering or reflowing the distal end of the slit 4412. The slits also do not need to be linear and could be spiral, bifurcated, include a series of enlarged gaps, etc. Overall, however, the objective is to reduce the force for the medical device to pass through the end of the expandable sheath 4400 and/or facilitate retrieval by tearing open the tip section 4404.

The tip section 4401 is permanently changed during expansion by elongation of the slit 4412 through tearing open the distal end 4422 through the intermediate layer 4426. For example, the tip section 4404 can go from a tapered shape with a smaller first diameter to a more expanded diameter after passage of the medical device. The first inner diameter may be about 4.7 mm or 0.184 inches, for example. The expanded second inner diameter may be from about 5.5 mm or .216 inches to about 9.0 mm or 0.354 inches, for example, depending, in cases where the medical device is a heart valve, on the crimped valve outer diameter mounted on the valve delivery system and deployed valve outer diameter.

As shown in **FIG. 47****,** the slit 4412 could have a U-shape with the rounded portion being on the proximal end and then the arms extending distally - with those arms separating during expansion of the tip section 4404. Notably, the U-shape has a relatively smooth and clean periphery to it to facilitate deployment and retrieval of the medical device. **FIG. 48** shows how the removal of the expansion force of the medical device allows the slit 4412 to retract somewhat due to the elasticity of the layers of the tip section 4404. However, the tip section remains substantially more receptive to retrieval due to the tearing and also some residual flaring open of the distal end 4422. **FIG. 48** also illustrates how, in one aspect, the layers may retract by different amounts, such as the inner liner layer 4408 retracting more than the outer layer 4410 or vice versa.

A method of delivering a medical device includes placing at least the distal end of the expandable sheath 4400 into a patient's blood vessel. As described above, the radiopaque marker 4428 is adjacent the distal end 4422 of the tip section 4404. The marker allows a medical technician to closely approximate the location of the distal end 4422 by looking at the location of the radiopaque marker 4428. The radiopaque marker is viewed by a medical technician through a device such as an x-ray device. The radiopaque marker is axially advanced and aligned at the desired location in the blood vessel, such as at a calcified aortic valve. A medical device is advanced distally through the sheath 4400, expanding the lumen 4406 to accommodate the size of the medical device.

As the medical device passes through the tip section 4404, the tip is radially expanded by enlarging the slit 4412 and tearing the distal-most end of the tip section, as shown in **FIG. 47****,** for example. Once the medical device has passed through the distal end 4416 of the tip section 4404, and the slit 4412 has been axially extended through the distal end 4422 of the tip section 4404, the diameter at the distal end of the sheath 4400 retracts partially, such as shown in **FIG. 48****,** into a diameter less than the expanded configuration but greater than the unexpanded configuration. If necessary, the medical device, such as a prosthetic heart valve, can be withdrawn partially or fully through the still somewhat expanded (and generally looser or more compliant) tip section 4404. The medical device can also be redeployed again by expanding the tip section 4404 to its largest diameter.

In certain aspects, a distal tip section 4404 of an expandable sheath 4400 can be manufactured according to the methods described below. The distal tip section 4404 disclosed herein can be combined with any of the other aspects of the sheaths disclosed herein. In some aspects, an elongated sheet as shown in **FIG. 47** of an inner liner layer material (as described above) is rolled helically or spirally to form an elongated tube that will ultimately become the inner liner layer 4408, as shown in **FIGS. 50-52****.** The first cut 4444 is made to extend proximally from a distal edge 4432 of the inner liner layer 4408. The second cut 4434 is made to extend from the proximal end of the first cut 4444 perpendicularly to one of a pair of lateral edges 4436 of the inner liner layer 4408.

The flap 4438 formed by the first and second cuts extends outwardly when the sheet is rolled into the elongated tubular inner liner layer 4408, as shown in **FIG. 63****.** In one aspect, the flap 4438 is retained by the remaining uncut portion between the second cut 4434 and the adjacent lateral edge 4436 forming a tab. Thus, the flap is an extension of the sheet that is rolled into the inner liner layer 4408. The radiopaque marker 4428 can be positioned between the extended flap 4438 and the underlying layers prior to subsequent heat processing steps, thereby encapsulating the radiopaque marker 4428. In some examples, the radiopaque marker is pre-formed in a C-shape, but in other examples, the radiopaque marker is any other shape that is suitable to be positioned between the flap 4438 and the underlying layers. The flap composition itself could also include a radiopaque compound. Note that the cuts 4444 and 4434 can also be made after rolling the sheet into the inner liner layer 4408.

As it is wrapped around, the end of the flap 4438 is aligned with the interior longitudinally extending end of the rolled inner liner layer 4408 and will ultimately become a part of the slit 4412. The flap 4438 is wound around the rolled elongated tube of the inner liner layer 4408 at a slight angle such that the (partially assembled) tip section 4404 tapers toward the distal end 4422 of the sheath 4400. The distal end 4422 of the flap 4438 is trimmed perpendicular to the longitudinal axis. The distal tip section 4404 is wrapped in heat shrink separately from the proximal section 4402 of the expandable sheath 4400. The heat shrink wrap forms and sets the tapered shape, such as by thinning the wall structure of the inner liner layer 4408.

The proximal section 4402 and the tip section 4404 of the rolled elongated inner liner layer 4408 are placed on a mandrel. Then, during a first reflow step, heat is applied to the distal tip section 4404, melting the flap 4438 to the remaining inner liner layers 4408 of the tip section. The area of the former first cut 4444 is visible even after melting and is reopened to form part of the slit 4412, as shown in **FIGS. 50-52****.** The tab between the end of the second cut 4434 and adjacent lateral edge 4436 is cut to free the proximal section 4402 of the inner liner layer 4408 to helically or spirally expand and collapse, as shown in **FIG. 63****.**

In one aspect, as mentioned above, prior to the first reflow step, the radiopaque marker 4428 can be positioned between the flap 4438 and the uncut portion of the rolled inner liner layer 4408. The flap is reflowed over the radiopaque marker 4428, encapsulating the radiopaque marker within the inner liner layer 4408, as shown in **FIGS. 63** **and** **66****.** Some thinning and tapering of the materials may also occur in this (and other heating and reflowing steps), as shown, for example, in **FIG. 56****.**

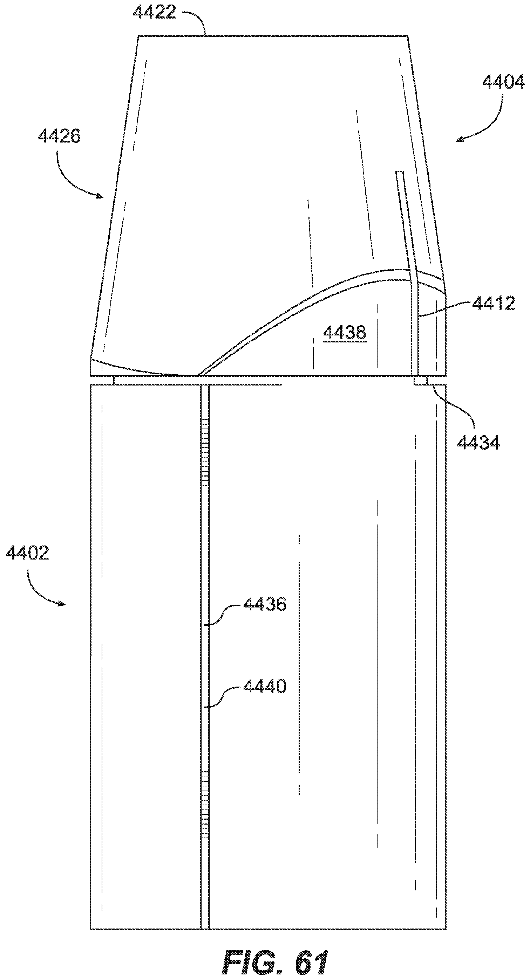

Next, as shown in **FIG. 64****,** the intermediate layer 4426 is positioned at a distal end of the distal tip section 4404 of inner liner layer 4408 such that the intermediate layer 4426 overlaps a portion of the inner liner layer 4408. In a second reflow step, the intermediate layer 4426 is reflowed (melted) with the inner liner layer 4408 such that the intermediate layer extends proximally over the outer surface of the inner liner layer. As shown in FIG. 61, some thinning of the soft intermediate layer 4426 (and other layers) may also in this step.

As shown in **FIG. 65****,** the outer layer 4410 is then positioned partially over the inner liner layer 4408 and a portion of the intermediate layer 4426 such that a portion of the intermediate layer extends distal of the outer layer 4410. The outer layer is heat treated to couple with the intermediate layer 4426 by a third reflowing/heat processing step. As such, the intermediate layer 4408 couples the outer layer 4410 to the inner liner layer 4408. In some aspects, the intermediate layer can be sandwiched between the inner liner layer 4408 and the outer layer 4410 prior to the second reflowing step, such that the inner liner layer 4408, the intermediate layer 4426, and the outer layer 4410 are coupled to each other simultaneously during the second reflowing step.

Once the outer layer 4410 is bonded to the distal tip section 4404, the slit 4412 is cut through the inner liner layer 4408, the outer layer 4410, and at least a portion of the intermediate layer 4426 of the tip section. The slit 4412 stops short of the distal end of the intermediate layer 4426, leaving the distal end 4422 of the distal tip section 4404 intact and circumferentially continuous, as shown in **FIG. 65****.**

Advantages of the tip section 4404 disclosed hereinabove include ease of manufacture, lower force to split the tip with no stretching, and a cleaner split, there is no distal or proximal shoulder formed by the layers, the marker 4428 is embedded in a secure location near the distal-most end of the sheath 4400, the spit tip has some dynamic recovery after device retrial, the intermediate layer 4426 bonds the outer layer (jacket) well to the inner liner layer and is atraumatic to the body lumen. Also, the tip section 4404 avoids premature opening during sheath insertion, and the alignment of the edge of the inner liner layer 4408 with the slit 4412 avoids delamination. The outer edge of the scrolled inner liner layer 4408 of the proximal section 4402 is flush behind the tip section 4404, eliminating any distal or proximal facing edge.

### METHODS (not claimed)

The aspects of the present disclosure also relate to a method of making a sheath having a proximal and a distal end and comprising: forming a variable diameter inner liner by rolling a sheet having a first edge and a second edge and wherein the sheet is defined by an inner surface and an outer surface in a spiral configuration such that at least a portion of the inner surface of the sheet overlays at least a portion of the outer surface of the sheet thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis; forming an outer layer having an inner surface and an outer surface and extending about at least a portion of the variable diameter inner liner such that the inner surface of the outer layer is positioned adjacent to the outer surface of the inner liner, wherein the outer layer comprises: a braid; and a layer of an elastomeric polymer having a predetermined thickness and having an inner surface and outer surface; wherein the variable diameter inner liner is configured to expand from a predetermined rest diameter *dᵣ* to an expanded diameter *dₑ* by sliding the first edge of the sheet along at least a portion of the inner surface and sliding the second edge of the sheet along the at least a portion of outer surface, during application of a radial outward force by passage of a medical device through the lumen of the inner liner.

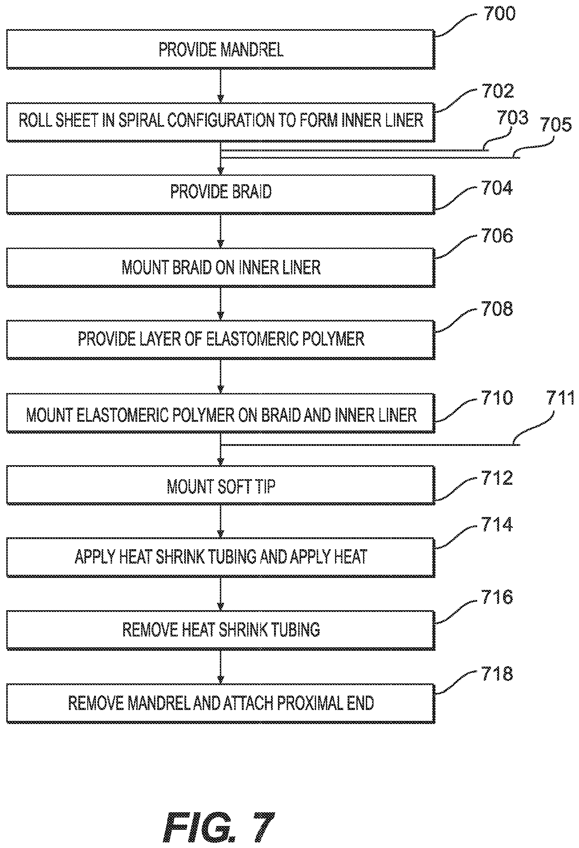

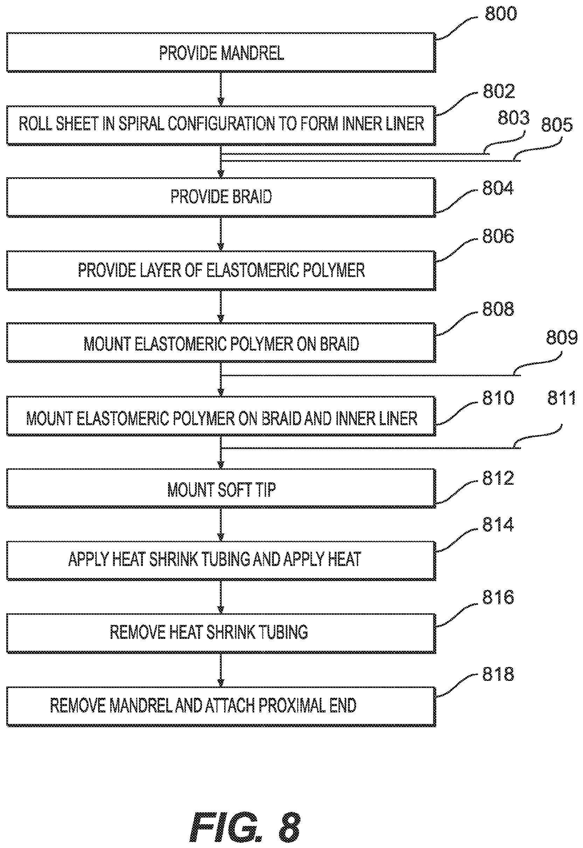

Various methods can be used to produce the sheaths discussed above and below throughout the present disclosure. For example, **FIGS. 7** and **8** exemplify block diagrams of exemplary methods of producing the sheath in various aspects. The various methods steps are also depicted in **FIG. 9A-9K** and **FIG. 31****.** In certain aspects, and as shown in **FIG. 9A****,** the inner liner can be formed from an extruded tube **903** having an inner surface and outer surface and having any thickness that is described above. This extruded tube can be cut **905** along the length to form a sheet. In certain aspects, the inner surface and/or outer surface of the tube can be surface treated, such as, for example, by plasma etching, chemical etching, or other suitable methods of surface treatment. In some exemplary aspects, where the outer surface of the inner liner is treated, the treatment can provide for better bonding with the outer layer when formed. In yet other aspects, the inner surface of the inner liner can be ribbed. In such exemplary aspects, the ribbed surface facilitates a reduction of contact points with the prosthetic device and can reduce friction. In still further aspects, the initial extruded tube **903** can be produced by co-extrusion with multiple layers of the same or different polymers as described herein. It is understood that one of ordinary skills in the art can choose the composition of the inner liner depending on the desired application. In certain aspects, the decision to use a specific material for the inner liner can be dependent on the desired stiffness, wall thickness, and lubricious optimization. In yet other aspects, and as disclosed above, the tube can also be cut to form a longitudinal slit **911** (shown in **FIG. 31****),** such that when the formed sheet is wound, it forms a helical scroll configuration as shown in **FIG. 32****.**

In still further aspects, the elongate single lumen tubing used to form the inner liner has an inner surface and an outer surface, wherein the at least a portion of an outer surface of this elongated single lumen tubing comprises a first plurality of protrusions and/or wherein at least a portion of the inner surface of the elongated single lumen tubing comprises a second plurality of protrusions. When the spiral configuration of the inner member is formed, this tubing is longitudinally cut at at least a portion of its circumference to form a sheet having a first longitudinal edge and an opposite second longitudinal edge. In such aspects, the cut is performed such that at least a portion of the first plurality of protrusions is disposed abutting the first longitudinal edge of the sheet and/or wherein the at least a portion of the second plurality of protrusions is disposed abutting the second longitudinal edge of the sheet. In still further exemplary aspects, when the sheet is rolled into a spiral configuration, at least a portion of the inner surface of the sheet overlays at least a portion of the outer surface of the sheet, thereby forming an overlapping portion wherein that the at least a portion of the first and/or second plurality of protrusions is disposed within the overlapping portion thereby reducing a contact area between the inner surface and the outer surface of the sheet within the overlapping portion. In still further aspects, the tubing having the first plurality of protrusions disposed on its outer surface and/or the second plurality of protrusions disposed on its inner surface can be made by any methods known in the art. In some aspects, the tubing is formed by coextrusion, when the first and/or second plurality of the protrusions are coextruded together with the remaining portions of the sheath. In still further aspects, the first and/or second plurality of protrusions can be formed by molding using a mold having the desired shapes.

It is understood that also disclosed herein are aspects where the second plurality of protrusions are formed on the inner surface of the tubing in addition or instead to the first plurality of the protrusions on the outer surface. In such aspects, for example, if the second plurality of protrusions is formed on the inner surface, the tubing is cut such that the second plurality of protrusions are abutting the second longitudinal edge, such that when the sheath is in the scroll configuration, the second plurality of protrusions are also disposed in the overlapping portion. It is further understood that if the second plurality of protrusions are present on the inner surface of the sheet, such a second plurality of protrusions can comprise any of the disclosed above protrusions. Similarly, in the aspects where the second plurality of protrusions are disposed on the inner surface, such a second plurality of protrusions can be disposed at at least a portion of the length of the inner liner or along the full length of the inner liner. Similar to aspects, where the first plurality of protrusions is present on the outer surface, when the second plurality of protrusions is present on the inner surface, they can be outside of the overlapping portion or even along a full circumference of the inner liner when in scroll configuration.

In still further aspects, the elongate single lumen tubing used to form the inner liner has an inner surface and an outer surface, wherein the at least a portion of an outer surface of the elongated single lumen tubing comprises a plurality of bonding sites at least partially embedded within a wall of the elongated single lumen tubing. When the spiral configuration of the inner member is formed, this tubing is longitudinally cut at at least a portion of its circumference to form a sheet having a first longitudinal edge and an opposite second longitudinal edge. In still further exemplary aspects, when the sheet is rolled into a spiral configuration, at least a portion of the inner surface of the sheet overlays at least a portion of the outer surface of the sheet, thereby forming an overlapping portion. In such aspects, the outer surface of the sheet in the overlapping portion is substantially free of the plurality of bonding sites, while at least a portion of the outer surface of the sheet outside of the overlapping portion comprises the plurality of bonding sites.

In still further aspects, the tubing having the plurality of bonding sites can be made by any methods known in the art. In some aspects, the tubing is formed by coextrusion, when the plurality of bonding sites are coextruded together with the remaining portions of the sheet. In still further aspects, the plurality of bonding sites can be formed by molding using a mold having the desired shapes.

In yet other aspects, also disclosed are methods when one or more bonding sites are positioned on the inner surface of the outer layer. It is understood that such bonding sites can also be made by any methods known in the art and suitable for the desired application. In certain aspects, such exemplary bonding sites can be formed by coextrusion or molding.

In still further aspects, one or more mandrels can be provided (step **700** or **800** in **FIG. 7** and **8****,** respectively). The mandrel can be provided with an exterior coating, such as a Teflon^{®} coating, and the mandrel's diameter can be predetermined based on the desired rest diameter *dᵣ* of the resulting sheath. As shown in **FIG. 9B****,** the sheet formed by cutting **905** the extruded tube **903** can be rolled in a spiral configuration (step **702** and **802** in **FIGS. 7** and **8** respectively) around the mandrel **901** to form the inner liner **902** such that at least a portion of the inner surface of the sheet overlays at least a portion of the outer surface of the sheet thereby forming an overlying portion **902c** and wherein the first edge (not shown) of the sheet is slidable along at least a portion the inner surface of the sheet and the second edge **902b** is slidable along at least a portion of the outer surface of the sheet. When the slit is formed similar to 911 of **FIG. 31****,** the steps of rolling the sheet around the mandrel are similar, but to provide a helical configuration and not spiral.

In still further exemplary aspects, in steps **705** and **805 (****FIGS. 7** and **8** respectively), an amount of a first lubricant **910 (****FIGS. 9C-9E****)** can be optionally applied on the outer surface of the inner liner. The presence of this lubricant material can reduce the friction between the inner liner and the outer layer of the final sheath. In yet other aspects, in steps **703** and **803,** an amount of a second lubricant **908** can be applied between the overlaying and sliding portions of the inner liner to further improve slidability and decrease friction. **(****FIG. 9D** depicts the inner liner with the two optional lubricants present with the mandrel hidden from the view). In still further aspects, it is understood that the inner liner formed with the use of mandrel can have any rest diameter, as described above. In certain aspects, the rest diameter dᵣ is substantially uniform along the longitudinal axis of the lumen. While in the other aspects, the rest diameter *dᵣ* varies along the longitudinal axis of the lumen and wherein the rest diameter *dᵣ* at the proximal end that is larger than the rest diameter *dᵣ* at the distal end. It is also understood that described herein are aspects where the lubricant material between the inner liner and the outer layer is not present.

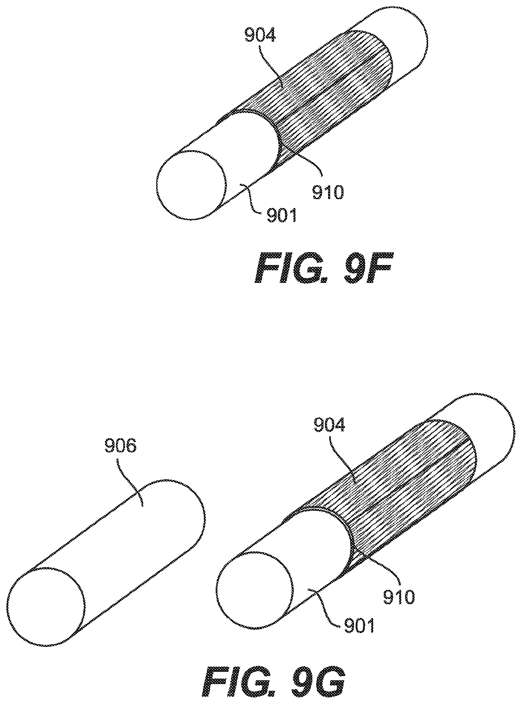

In still further aspects, the methods can further comprise a step of providing a braid or coil (steps **704** and **804).** It is understood that any of the described above braid or coil can be used in this step. In still further aspects, and as shown in step **706** of **FIG. 7****,** the braid or coil is mounted on the inner liner. In some exemplary aspects, and as shown in **FIG. 9F** the braid or coil **904** can be mounted on the first lubricant **910** that can be present on the outer surface of the inner liner. It is understood that in some aspects, the second lubricant can be present only at a portion of the outer surface of the inner liner. In yet other aspects, the disclosed sheath can have segments where the first lubricant is present, and the braid or coil is mounted over it, while it can have other segments where the second lubricant is not present, and the braid or coil is mounted directly on the outer surface of the inner liner. It is understood that the location of these specific segments can be determined by one of ordinary skill in the art depending on the desired application. It is understood that the mounting of the braid or coil can be done by any known in the art methods. In some unlimiting aspects, the braid or coil can be provided as a cylindrical tube, and it can be slid on top of the inner liner or the first lubricant if it is present.

In yet further aspects and as shown in step **708,** the method can further comprise a step of providing a layer of the elastomeric polymer. In such exemplary aspects, this layer of the elastomeric polymer can be used as an outer layer of the sheath. It is understood that any of the disclosed above elastomeric polymers can be used. The specific polymer can be chosen based on the desired properties of the disclosed sheath, such as, for example, level of stiffness, hemostasis, and the like. The layer of the elastomeric polymer can be provided in any form known in the art. In certain and unlimiting aspects, the elastomeric polymer can be provided as a cylindrical tube **906 (****FIG. 9G****).** In a still further aspect, the elastomeric polymer can be mounted on the inner liner and the braid or coil (step **710).** **FIG. 9G****,** for example, depicts an aspect where the cylindrical tube of the elastomeric polymer **906** is used to slide on the inner liner, having a first lubricant **910** overlaying the inner liner's outer surface and the braid or coil **904.**

In yet further aspects, the disclosed method can comprise a step of embedding (step **711,** **FIG. 7****)** the braid or coil into the layer of the elastomeric polymer that serves as the outer layer. It is understood that the sheath can comprise various segments. In some aspects, some of the segments can comprise the braid or coil embedded within the layer of the elastomeric polymer, while in other segments, the braid or coil and the layer of the elastomeric polymer are separate. It is further understood that in some aspects, the sheath can have a braid or coil embedded within the elastomeric polymer over the whole length of the sheath, while in other aspects, the braid or coil is not embedded within the elastomeric polymer over the whole length of the sheath. It is further understood that any methods known in the art can be used to embed the braid or coil within the elastomeric polymer. In some aspects, the application of heat can be utilized. In certain aspects, the use of heat shrink tubing can be utilized to embed the braid or coil within the elastomeric polymer. It is understood that after the step of embedment is complete, the heat shrink tubing is removed. In yet other aspects, the braid or coil can be embedded within the layer of the elastomeric polymer by placing the assembly in an oven or otherwise heating it.

In still further aspects, the method further comprises thermally bonding the elastomeric polymer used as the outer layer to the inner liner through at least a portion of the plurality of bonding sites.

In still further aspects, a soft, atraumatic tip can be provided at the distal end of the resulting sheath (step **712).** In yet further aspects, the outer layer comprising the braid or coil and the layer of the elastomeric polymer is at least partially bonded to the inner liner. It is understood that this bonding can also be achieved by any known in the art methods. In certain aspects, and as shown in step **714,** a heat shrink is applied to the portion that is being bound and heated to form a bonding between the inner liner and the outer layer. In yet other aspects, the bonding between the inner liner and the outer layer can be achieved by placing the assembly in an oven or otherwise heating it. In still further aspects, the bonding is performed by heating at a temperature from about 350 °F to about 550 °F for a time period effective to form a bond between at least a portion of the outer layer and at least a portion of the inner liner. In yet further aspects, the heating can be done at a temperature of about 375 °F, about 400 °F, about 425 °F, about 450 °F, about 475 °F, about 500 °F, or about 525 °F. In yet other aspects, the time period effective to form a bond can comprise from about 1 second to about 60 seconds, including exemplary values of about 5 seconds, about 10 seconds, about 15 seconds, about 20 seconds, about 25 seconds, about 30 seconds, about 35 seconds, about 40 seconds, about 45 seconds, about 50 seconds, and about 55 seconds. However, it is further understood that this time period is not limiting, and it can have any value needed to provide for an effective bond, for example, it can have any value from about 1 second to about 5 hours. It is further understood that if the heat shrink tubing is used to obtain the desired bonding, the heat shrink tubing is removed (step **716,** **FIG. 7****).**

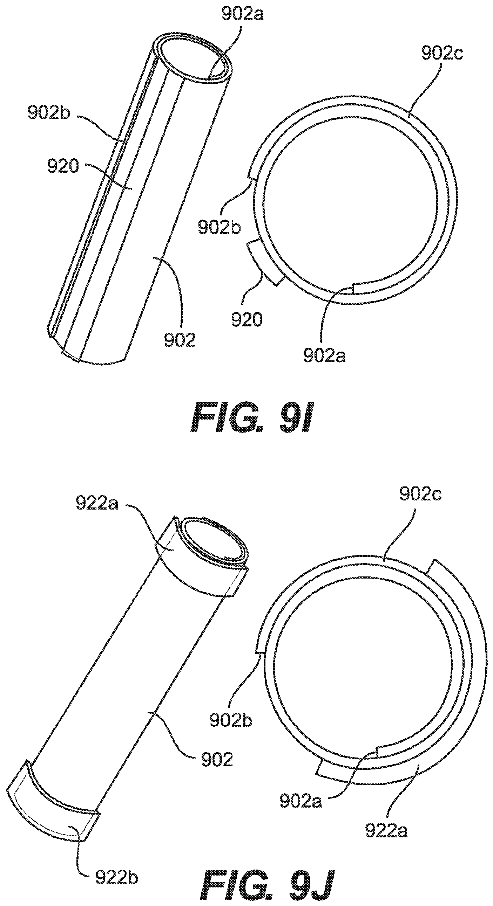

In still further aspects and as shown in **FIG. 9I****,** the bonding step can also comprise a first strip of the elastomeric polymer **920** to be applied along at least a portion of the longitudinal axis of the lumen to at least a portion of the outer surface of the sheet that does not comprise the overlaying portion prior **902c** to or during the step of bonding the at least a portion of the inner surface of the layer of the elastomeric polymer to at least a portion of the outer surface of the sheet of the inner liner. It is understood that in some exemplary aspects and as shown in the **FIG. 9I****,** this first strip can be applied prior to mounting the braid or coil. In still further aspects, the location where the first strip is applied does not comprise the first lubricant. However, it is understood that in such aspects, the first lubricant can be present in other locations.

In still further exemplary aspects, the methods can comprise a second strip **922a** of the elastomeric polymer that can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to mounting the braid or coil. In still further aspects, the location where the second strip is applied does not comprise a first lubricant. However, it is understood that in such aspects, the first lubricant can be present in other locations. In yet other aspects, the method can comprise a third strip of the elastomeric polymer **922b** that can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to mounting the braid or coil. In still further aspects, the location where the third strip is applied does not comprise the first lubricant. However, it is understood that in such aspects, the first lubricant can be present in other locations. In still further aspects, both the second and the third elastomeric polymers are present. While in other aspects, only one of the second or the third elastomeric polymers is present. It is further understood that the first, second, and third elastomeric polymers can be the same or different. It is also understood that the first, second, and third elastomeric polymers can be the same as the layer of the elastomeric polymer present in the outer layer and can comprise any of the elastomeric polymers described herein.

**FIG. 9K** illustrates a cross-section view of the sheath at step **718** of **FIG. 7****.** The sheath **900,** made according to described methods and processes, can be attached or bonded to a housing **101,** such as by bonding the proximal end of the sheath **900** to the polycarbonate housing **101.**

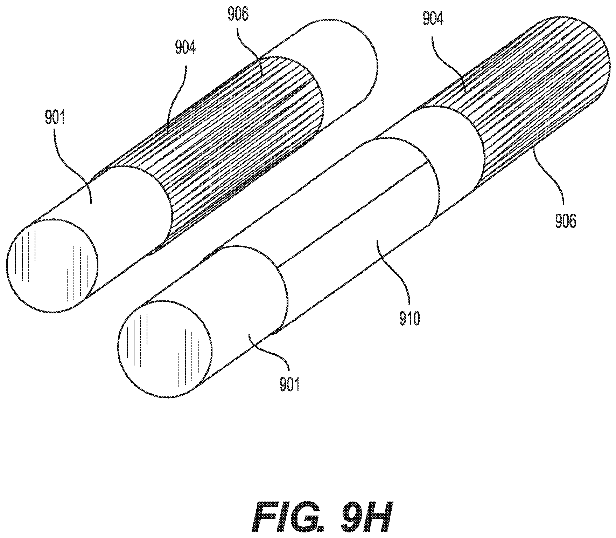

Some alternative aspects are shown in **FIG. 8** and **FIG. 9H****.** In such alternative aspects, the outer layer is pre-formed and then mounted on the inner liner positioned on the mandrel. In such aspects, the provided layer of the elastomeric polymer is first mounted on the braid or coil (step **808)** prior to mounting it on the inner liner. In yet other aspects, the method can also comprise a step of partially embedding the braid or coil within the layer of the elastomeric polymer before mounting both of them on the inner liner (step **809).** However, the step of partially embedding the braid or coil with the layer of the elastomeric polymer can be done after the braid or coil, and the layer of the elastomeric polymer is mounted on the inner liner (step **811).** Steps **812-818** can be performed analogously to steps **712-718.**

In still further aspects, and as shown in **FIG. 9I****,** the bonding step can also comprise a first strip of the elastomeric polymer **920** to be applied along at least a portion of the longitudinal axis of the lumen to at least a portion of the outer surface of the sheet that does not comprise the overlaying portion prior **902c** to or during the step of bonding the at least a portion of the inner surface of the layer of the elastomeric polymer to at least a portion of the outer surface of the sheet of the inner liner. It is understood that in some exemplary aspects and as shown in the **FIG. 9I****,** this first strip can be applied prior to the mounting pre-formed outer layer comprising the braid or coil and the elastomeric polymer. In still further aspects, the location where the first strip is applied does not comprise the first lubricant. However, it is understood that in such aspects, the first lubricant can be present in other locations.

It is also understood that these alternative aspects can also include a step where a second strip **922a** of the elastomeric polymer that can be applied to at least a portion of the outer surface of the sheet at the proximal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this second strip can be applied prior to the mounting pre-formed outer layer comprising the braid or coil and the elastomeric polymer. In still further aspects, the location where the second strip is applied does not comprise a first lubricant. However, it is understood that in such aspects, the first lubricant can be present in other locations. In yet other aspects, these methods can also comprise a third strip of the elastomeric polymer **922b** that can be applied to at least a portion of the outer surface of the sheet at the distal end of the sheath prior to or during the step of bonding the at least a portion of the inner surface of the layer of the elastomeric polymer to at least a portion of the outer surface of the sheet of the inner liner. It is understood that in some exemplary aspects and as shown in the **FIG. 9J****,** this third strip can be applied prior to the mounting pre-formed outer layer comprising the braid or coil and the elastomeric polymer. In still further aspects, the location where the third strip is applied does not comprise the first lubricant. However, it is understood that in such aspects, the first lubricant can be present in other locations. In still further aspects, both the second and the third elastomeric polymers are present.

It is understood that in the aspects where the plurality of bonding sites is present, the bonding between the inner liner and the outer layer (or elastomeric polymer) can be done through the plurality of bonding sites.

Also disclosed herein are the methods of making additional configurations of the sheath. For example, disclosed herein is a method of making a sheath having a proximal and a distal end. Such methods comprising forming a variable diameter inner liner by providing an elongated single lumen tubing comprising at least one polymer layer; longitudinally cutting at least a portion of a circumference of the elongated single lumen tubing to form a sheet having a first longitudinal edge and an opposite second longitudinal edge and having an inner surface and an outer surface; and then forming a variable diameter inner liner by rolling the sheet in a spiral configuration such that at least a portion of the inner surface of the sheet overlays at least a portion of the outer surface of the sheet, thereby forming an overlying portion and wherein the first edge of the sheet is slidable along at least a portion the inner surface of the sheet and the second edge is slidable along at least a portion of the outer surface of the sheet, wherein the inner surface of the sheet defines a lumen of the sheath having a longitudinal axis.

It is understood that the elongated single lumen tubing can be extruded or coextruded from any polymers or compounds disclosed above. For example, and without limitation, the elongated single lumen tubing can comprise at least one polymer comprising a polyolefin, a polyamide, a fluoropolymer, copolymers thereof, co-extrudates thereof, or blends thereof. Yet, in other aspects, a compound material comprising a polyolefin and a lubricious filler. In such exemplary aspects, the polyolefin can be high-density polyethylene. Yet, in other aspects, the lubricous filler can comprise a polytetrafluoroethylene (PTFE) filler. In such aspects, the lubricious filler can be present in an amount from about 5 wt % to about 20 wt % of a total weight of the compound material.

For example, and without limitations, a tubular body can be extruded to form an elongated tubing comprising a compound material. This compound material can comprise a polyolefin present in an amount from greater than 0 wt% to less than 100 wt % based on a total weight of the compound and a lubricious filler present in an amount from about 5 wt % to about 20 wt% of a total weight to the compound material.

The extruded or coextruded elongated single lumen tubing can have a coefficient of friction less than about 0.5.

In still further aspects, the single lumen tubing can be produced by co-extrusion with multiple layers of the same or different polymers as described herein. In yet other aspects and as disclosed above, this tubing can be coextruded with any of the disclosed above tie layers. In such exemplary aspects, where for example, the tie layer is present, the elongated tubing can comprise any of the disclosed above polymers, and the tie layer is disposed on the inner surface of the tubing or/and an outer surface of the tubing.

It is understood that this elongated tubing is used to form the inner liner, and any of the disclosed above materials can be used. In still further aspects prior to cutting the tubing to form the spiral configuration of the inner liner, a lubricious liner can be disposed on the inner surface of the tubing and/or the outer surface of the tubing. It is understood that this lubricious liner can be disposed on the tie layer if it is present. In certain aspects, the tie layer is used to bond the lubricious liner with the polymer layer forming the elongated tubing. In yet further aspects, at least a portion of the inner surface of the elongated tubing can be ribbed. In such exemplary aspects, the ribbed surface facilitates a reduction of contact points with the prosthetic device and can reduce friction.

Yet, in other aspects, where the tie layer and/or lubricious liners are present, at least a portion of the lubricious liner can also be ribbed. Also, in such aspects, the tubing comprising any of the disclosed above polymers, the tie layer, and the lubricious liner has a coefficient of friction less than about 0.5.

The elongated tubing is then positioned on a mandrel and cut at at least a portion of the circumference of the tubing, as disclosed above. In still further aspects, the methods of forming the inner liner are similar to those shown in **FIG. 9A****.** The inner liner can be formed from a single lumen extruded tube **903,** having an inner surface and outer surface and having any thickness that is described above. This extruded tube can be cut **905** along the length to form a sheet.

Additionally, in certain aspects, the inner surface and/or outer surface of any of the disclosed above single lumen tubing can further be surface treated, such as, for example, by plasma etching, chemical etching, or other suitable methods of surface treatment. In some exemplary aspects, where the outer surface of the inner liner is treated, the treatment can provide for better bonding with the outer layer when formed. It is understood that one of ordinary skills in the art can choose the composition of the inner liner depending on the desired application. In certain aspects, the decision to use a specific material for the inner liner can be dependent on the desired stiffness, wall thickness, and lubricious optimization.

The formed sheet can then be positioned on an additional mandrel. Such a mandrel can be provided with an exterior coating, such as a Teflon^{®} coating, and the mandrel's diameter can be predetermined based on the desired rest diameter *dᵣ* of the resulting sheath. As shown in **FIG. 9B****,** the sheet formed by cutting **905** the extruded tube **903** can be rolled in a spiral configuration around the mandrel **901** to form the inner liner **902** such that at least a portion of the inner surface of the sheet overlays at least a portion of the outer surface of the sheet thereby forming an overlying portion **902c** and wherein the first edge (not shown) of the sheet is slidable along at least a portion the inner surface of the sheet and the second edge **902b** is slidable along at least a portion of the outer surface of the sheet. It is understood that any of the disclosed above spiral configurations can be obtained.

For example, the sheet is rolled such that the first and the second edges of the sheath are substantially aligned in a spaced relationship along a vertical axis, passing through a thickness of the sheath. In such aspects, the spaced relationship can comprise a portion of the sheet positioned between the first edge and the second edge along the vertical axis. In certain aspects, the sheet is rolled such that when the sheath is in an unexpanded rest state, the inner liner comprises at least two layers of the sheet overlaying each other along at least a portion of a sheath's circumference. While in other aspects, when the sheath is in an unexpanded rest state the at least a portion of the sheath's circumference comprises three layers of the sheet overlaying each other. Also disclosed are aspects that when the sheath is rolled into the spiral configuration, the first edge of the sheet can be substantially aligned with a vertical axis passing through a thickness of the sheath and the second edge circumferentially offset from the vertical axis. In such configuration, in some aspects, at at least a portion of a circumference of the sheath, the inner liner comprises one layer of the sheet without any overlaying portion.

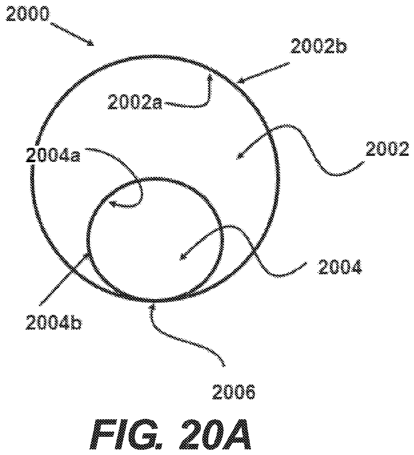

In certain aspects, however, the inner liner can be formed from an extruded double lumen tubing. The extruded double lumen tubing can comprise any of the disclosed above polymers or compounds. Exemplary schematic of the methods of making the inner liner from such double lumen tubing is shown in **FIGS. 20A-20C****.** The double lumen **2000** can comprise a first channel **2002** having an inner surface **2002a** and an outer surface **2002b** and a second channel **2004** having an inner surface **2004a** and an outer surface **2004b.** The second channel **2004** is positioned within the first channel **2002** such that at least a portion of a circumference of the first channel and at least a portion of a circumference of the second channel have at least one shared inner surface and at least one shared outer surface **2006.** In such a configuration, the outer surface **2002b** of the first channel **2002** defines an outer surface of the double-lumen tubing.

It is understood this double lumen tubing can comprise any of the disclosed above layers.

For example, the double-lumen tubing can be coextruded from the compound material described above. In such exemplary aspects, the first channel can comprise the compound material, or the second channel, or both can comprise the described above compound material.

In yet further aspects, the double-lumen tubing can be coextruded with any of the disclosed above tie layers, where the tie layer can be coextruded with the first channel or the second channel, or both. In still further aspects. any of the disclosed above lubricious liners can be disposed at the tie layer. It can be disposed, for example, within the first channel, or the second channel, or both.

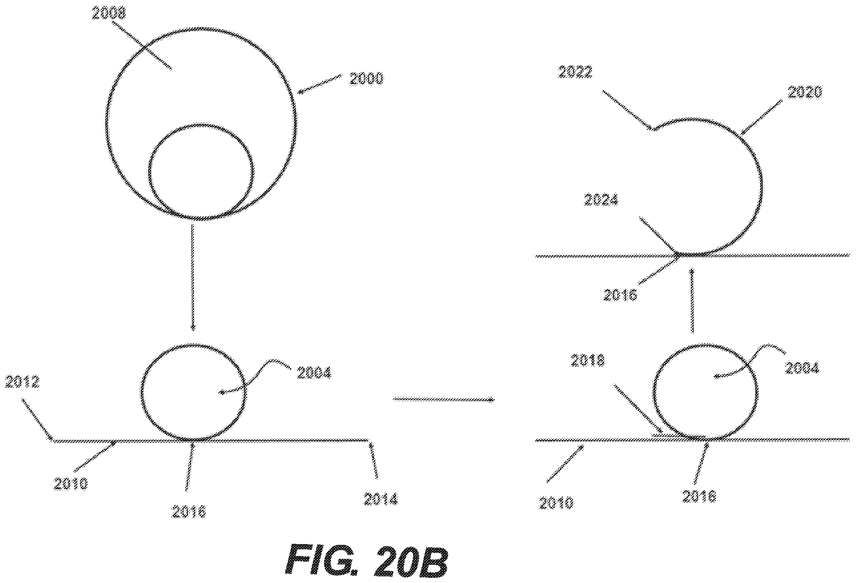

In still further aspects, and as shown in **FIG. 20B****,** the first channel can be cut longitudinally along at least a portion of the first channel circumference 2008 that is not shared with the circumference of the second channel to form a first sheet. The first sheet **2010** has a first edge **2012** and a second edge **2014,** and the second channel disposed longitudinally along at least a portion of the first sheet **2004,** such that at least a portion of the circumference of the second channel has at least a portion that has a shared surface **2016** with the first sheet along a length of the sheet and along a length of the second channel.

The second channel is then longitudinally cut at a portion of the circumference of the second channel **2018** that abuts the shared surface **2016** with the first sheet to form a second sheet **2020** having a first edge **2022** and a second edge **2024,** wherein the second edge is defined by a portion of the shared surface with the first sheet **2016.**

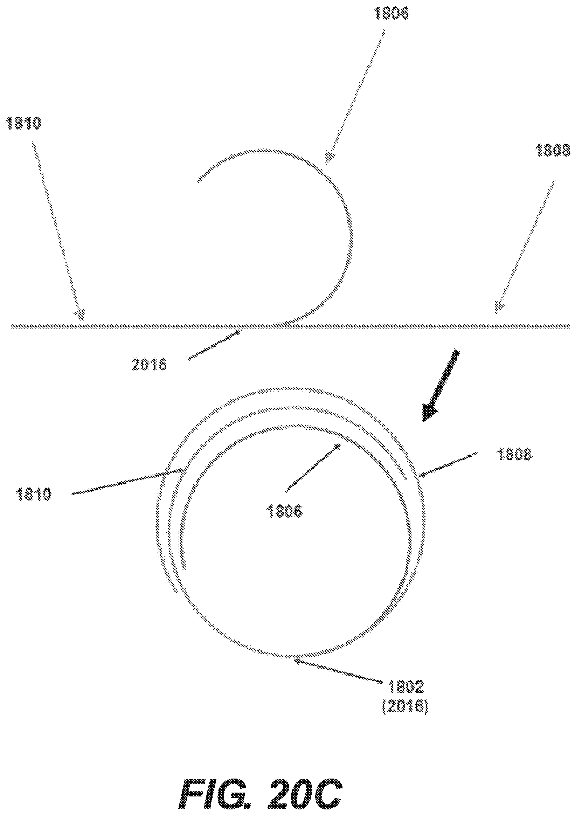

In still further aspects, the first and the second sheets are rolled into a spiral configuration, as shown in **FIG. 20C****,** such that: the shared surface **2016** between the second and the first sheets form a first portion **1802 (2016)** of an inner liner having a first surface and an opposite second surface. At least a portion of the second sheet **1806** forms a first segment of an inner liner having a first surface and an opposite second surface. A portion **1810** of the first sheet adjacent to the second end of the second sheet and extending to the first end of the first sheet forms a second segment **1810** of an inner liner having a first surface and an opposite second surface. While a portion of the first sheet adjacent the second end **1808** of the second sheet and extending to the second end of the first sheet forms a third segment 1808 of an inner liner having a first surface and an opposite second surface. In such a spiral configuration and as shown in **FIG. 20C** and **FIG 18****,** at least a portion of the first surface of the second segment overlaps at least a portion of the second surface of the first segment, wherein at least a portion of the first surface of the third segment overlaps at least a portion of the second surface of the second segment, and wherein at least a portion of the first surface of the third segment overlaps at least a portion of the second surface of the first segment; wherein the first surface of the first portion extends into the first surface of the first segment, the second segment and the third segment, and wherein the second surface of the first portion extends into the second surface of the second and the third segments.

In certain aspects, whether the inner liner is formed from the single lumen tubing or from the double-lumen tubing, prior to the step of cutting, the methods can comprise the step of disposing an amount of lubricant. It is understood that the lubricant can be disposed at any surface of any channel.

In still further aspects, the lubricant can be disposed manually. While in other aspects, the lubricant can be disposed by pad printing. Yet still, in other aspects, the lubricant can be disposed by spraying.

It is understood that any of the disclosed above lubricants can be used.

When the lubricant is disposed manually, any known in the art techniques can be utilized. For example, and without limitations, the lubricant can be disposed with a brush, cloth, pad, and the like.

In some aspects, the lubricant is disposed by pad printing. During pad printing, an amount of lubricant is placed in a recessed channel on a plate. The length and width of the channel can be predetermined as desired. For example, the length and width of the channel can correspond to the desired area on a surface of the inner liner where the lubricant is placed. The volume of the channel can also be defined, partially determining the total amount of lubricant to be transferred. In such aspects, a soft pad can be dipped in the channel, picking up the lubricant, and moved over the desired portion of the inner liner. The pad is then can be stamped onto the predetermined portion of the liner, transferring the lubricant onto the surface of the inner liner.

It is understood that in the aspects disclosed herein, the predetermined amount of lubricant is applied to a set location (predetermined portion) of the inner liner's surface. In such aspects, the methods disclosed herein allow a substantial control of both the application area and the volume of lubricant used. As disclosed above, since the depth of the channel can be controlled, an amount of the lubricant disposed on the surface of the inner liner can also be substantially controlled.

In aspects where the pad-printing is used to transfer the lubricant on the surface of the inner liner, any of the disclosed above lubricants can be utilized. In certain aspects, the lubricant used for the pad-printing methods is substantially viscous. In certain exemplary and unlimiting aspects, the lubricant can have a viscosity from about 600 cP to about 1,200 cP. In yet further aspects, it is understood that the viscosity of the lubricant can be adjusted by adding more solids or more solvents to the lubricant composition. For example, and without limitation, the lubricant such as MED10-6670 (or similar) can be thickened beyond its' standard viscosity.

It is understood that the use of pad-printing allows the lubricant to be applied to a very specific portion of the inner liner with tight tolerance. In certain aspects, the lubricant can comprise a fluorescent material or any other material that would allow it to determine its specific location on the device. In such aspects. for example, a specific lubricant location can be determined under UV light. It can allow easy quality control to determine whether the lubricant has been applied in the desired portion of the sheath.

Yet, in other aspects, the substantially precise pad printing can help reduce push forces by decreasing frictional force in the most relevant area of the sheath while at the same time preventing undesired migration of the lubricant.

In certain aspects, the lubricant can be applied around a portion of the circumference of the inner liner. Yet, in other aspects, the lubricant can be applied around a whole circumference of the outer surface of the inner liner

In yet other aspects, the lubricant can be transferred in a predetermined pattern. For example, it can be transferred in the "stripe" pattern. However, it is understood that such a pattern is only exemplary. The pattern can have any regular or irregular shape. For example, if the pattern is the stripe pattern, lubricant can be disposed in a plurality of stripes along a whole length of the inner liner or along a portion of the inner liner. In still further aspects, the pattern can have a triangular shape, tapered shape, oval shape, circular shape, rectangular shape, or any irregular shape. The pattern can be determined based on the desired application and/or the desired location on the outer surface of the inner liner.

In still further aspects, the inner liner can be rotated by the pad printing machine in between runs so that the printer can make lines (or any other desired shapes) of the lubricant in a new location along the surface of the inner liner. In addition to precisely controlling the amount of lubrication added to the inner liner and the lubricant location, this method allows the manufacturing of the sheath to be more cost-effective by reducing the amount of lubricant and reducing the number of a sheath that could be discarded due to inaccurate application of the lubricant that can affect the overall sheath performance.

In still further aspects, the lubricant can be applied by spray coating. In such methods, the lubricant is loaded into an atomizing machine. The sheath, or, more specifically, the inner liner, is mounted on a mandrel that is able to rotate about the long axis of the sheath. The lubricant is then sprayed onto the rotating sheath and translated down of a desired portion of the inner liner length, ensuring substantially uniform coverage. The amount of the lubricant to be sprayed onto the inner liner, the speed at which the nozzle moves along the sheath, and the sheath rotation speed can all be specified to optimize this application process.

In certain aspects, any of the disclosed above lubricants can be used. In aspects where the spray coating is utilized, the viscosity of the lubricant is equal to or less than 600 cP. Spray coating uses equipment that atomizes a liquid solution input to the spray coating machine. The resulting spray can be used to coat various devices. In the case of the expandable sheath device, the lubricant is mixed and fully prepared, then loaded into the machine.

To apply the lubricant in a substantially uniform manner onto the inner liner, the inner liner is mounted on a mandrel that is configured to rotate along the long axis of the sheath at a specified speed. The nozzle of the coating machine then sprays out atomized droplets of the lubricant solution onto the inner liner. The nozzle translates, moving horizontally along the length of the sheath as it rotates. Speeds can be adjusted to optimize the amount of lubricant sprayed onto the inner liner. It is understood that if more lubricant is needed, the speeds can be slowed, so the nozzle stays at a given location along the surface of the inner liner for a longer period and vice versa. As with pad printing, the lubrication can be comprised of a fluorescing component, so under blacklight, it is clearly visible to see the areas of the sheath coated with lubrication.

In still further aspects, after application of the lubricant on the surface of the inner liner, the lubricant is cured. It is understood that the curing can be done at any condition effective to provide the desired result. In certain exemplary and unlimiting aspects, the curing is performed in an oven. Curing temperature and timing can be defined by the specific lubricant used in the methods disclosed above.

In still further aspects, the methods can further comprise disposing an outer layer over at least a portion of the outer layer of the inner liner to form the sheath that is configured to expand from a predetermined rest diameter *dᵣ* to an expanded diameter *dₑ* by sliding the first longitudinal edge of the sheet along at least a portion of the inner surface and sliding the second longitudinal edge of the sheet along the at least a portion of the outer surface, during application of a radial outward force by passage of a medical device through the lumen of the inner liner.

In certain aspects, the outer layer can be made by extruding a tubular body to form an elongated tube comprising a first polymer layer, wherein the first polymer layer comprises a first compound composition comprising from greater than 0 wt% to less than 100 wt% of a polymer comprising a polyether block amide, a polyurethane, or a combination thereof; less than about 65 wt% of an inorganic filler based on a total weight of the first compound composition; and up to about 20 wt% of a solid lubricant filler based on a total weight of the first compound composition. This elongated tube can then be disposed on any of the disclosed above inner liners to form the outer layer of the sheath. It is understood that any of the disclosed above additional layers can also be present between the inner liner and the outer layer.

In yet other aspects, the outer layer as presented herein can be made by coextruding an elongated bump tubing comprising a first polymer layer and a second polymer layer; wherein the first polymer layer comprises: a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; the second polymer layer comprises a polyurethane wherein the first polymer layer defines an inner surface of the tubing and the second polymer layer defines an outer surface of the tubing; and then disposing such a tubing on the surface of any one of the disclosed herein inner liners. It is understood that any of the disclosed above additional layers can also be present between the inner liner and the outer layer.

In still further aspects, methods also comprise a) forming an inner liner by any of the disclosed above methods and then b) disposing a first polymer layer comprising a first compound composition comprising from greater than 0 % to less than 100 % of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition; wherein the first polymer layer is disposed over a proximal portion of the inner liner and has a length from about 5 cm to about 15 cm; c) disposing a second polymer layer comprising polyurethane over the first polymer layer, wherein the second polymer layer extends along a length of the sheath; wherein the first polymer layer and the second polymer layer together form an outer layer of the sheath.

It is understood that any known in the art methods can be utilized to form any of the disclosed herein compositions. In certain aspects, the components that are present in any of the disclosed herein elongated tubes are provided to form a compound. The compound is then mixed to form a substantially homogeneous mixture. Yet, in other aspects, the mixture is homogeneous. In still further aspects, the mixture is extruded to form an elongated tube having a first polymer layer. The formed first polymer layer can comprise any (and in any combination) of the compositions and characteristics disclosed above.

In yet further aspects, the methods also comprise steps of forming the elongated tube comprising two or more layers, as disclosed above. In such aspects, for example, when the elongated tube comprises any of the disclosed above first polymer and the second polymer layers, such layers can be coextruded to form the elongated tube as disclosed. Any of the known in the art extrusion devices can be used to obtain any of the desired elongated tubes.

Also disclosed herein are methods of bonding the outer layer and inner liner if desired. It is understood that any known in the art methods can be used to form a bond. In certain aspects, heat treatment can be utilized. For example, the sheath can be inserted into a heat-shrink tubing and heated together to a temperature that would allow at least a partial bonding between the inner liner and the outer layer.

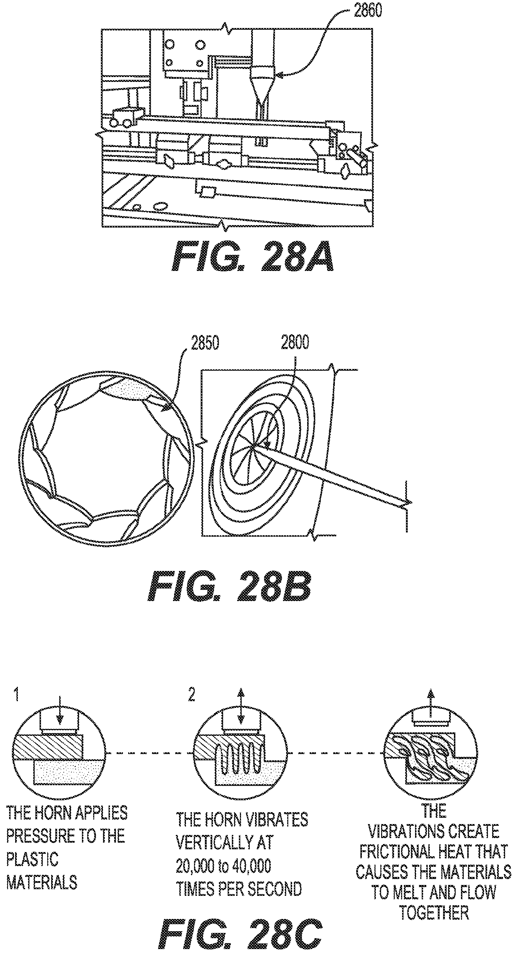

Some additional exemplary aspects can include laser welding, compression head welding or ultrasonic welding, as shown in **FIG. 28A-C.**

In yet other aspects, laser welding can be utilized. In such aspects, an inner liner can be formed by any of the methods disclosed above. The outer layer comprising any of the disclosed above compositions and formed by any of the disclosed above methods is then disposed over the inner liner to form the sheath. It is also understood that any of the disclosed above layers can also be presented between the inner liner and the outer surface. The sheath is then positioned on a mandrel configured to rotate. Laser welding uses a focused laser to heat a part in a selected location. The mandrel is aligned with a laser beam configured to move along a longitudinal axis of the sheath to a predetermined distance at conditions effective to form the bond **(****FIG. 28A****).** In such aspects, the head of the laser bonder is positioned directly above the center of the sheath, and the sheath can be rotated to align the laser with any desired point on the diameter of the sheath. The laser bonder then moves horizontally across the sheath.

A bond can be formed at a predetermined portion of the sheath between at least a portion of an outermost surface of the inner liner and at least a portion of an innermost surface of the outer layer. It is understood, however, while other additional layers such as lubricants or tie layers can be present, the predetermined location where the bond is formed is substantially free of a lubricant and/or a tie layer.

It is understood that settings on the laser welder can be changed to optimize both the level of heating and bonding area on the device. Laser power in terms of wattage changes the heat that is imparted onto the sheath, while the feed rate adjusts the length of time the laser is focused on a given portion of the sheath. Focus position, weld start angle, and welding distance can be used to control the area of the laser, are fine-tuned to create a bond of the correct size.

It is understood that settings on the laser welder can be changed in terms of wattage changes the heat that is imparted onto the sheath, while the feed rate adjusts the length of time the laser is focused on a given portion of the sheath. Focus position, weld start angle, and welding distance can be used to control the area of the laser, are fine-tuned to create a bond of the correct size. Also disclosed herein are additional methods of forming a bond. For example, the use of a compression head bonder **(****FIG. 28B****).** In such methods, the inner and outer layers are formed by any methods disclosed above and to assemble to form a sheath. The sheath is then positioned onto a mandrel into a radial compression head bonder.

Any known in the art compression head bonders can be used. In certain aspects, the compression head bonder can comprise a collapsible aperture configured to compress the sheath to a predetermined diameter. The radial compression head bonder comprises a plurality of dies, wherein at least one of a plurality of dies can be heated to form the bond at the preterminal portion of the sheath.

An exemplary and unlimiting bonder, as shown in **FIG. 28B** can, for example, comprise a total of nine separate dies, eight made from PEEK plastic, for example, and one metallic die. The metallic die can be heated before use. The machine brings the jaws of the aperture together to a smaller diameter, then compresses around a device inserted through the block. The heated die then melts the portion of the inserted component that comes in contact with it. To use with an expandable sheath, a mandrel is inserted into the lumen of the device to prevent compression of the sheath shaft while under the load of the bonder. In certain aspects, fluorinated ethylene propylene can be placed over the desired bonding area of the sheath to transfer the heat evenly, preventing the metal from directly burning the outer layer. The sheath can be supported in the machine using a separate channel to the side. In certain aspects, the width of the bonding dies can be less than the length of the sheath, so to lengthen the bonding area, the sheath can be moved horizontally after each run, and the bonding can be repeated for the desired length along the sheath.

It is understood that the specific bond and its location can be controlled as desired. For example, the temperature of the metal die can be determined to ensure that it is set to a temperature that effective to at least partially melt both the outer layer and inner liner components. In yet further aspects, the compressive force exerted onto the part can also be adjusted such that at least partial melting is obtained without any substantial damage to the remaining components of the sheath. A final diameter the aperture reaches while bonding can also be predetermined. It is understood that the larger the diameter, the more exposure the metal die has to the part, and the larger the bonding area can be obtained. Still further, a length of time the dies are compressed onto the sheath can also impact the degree of melting of the components and the strength of the bond.

In still further aspects, the methods can comprise ultrasonic welding. In such aspects, the sheath components to be bonded are placed on the welding machine, and then a load is applied from a moveable horn. The horn moves at an extremely fast rate, exerting a high amount of vibrational energy. This energy is absorbed by the materials, which can melt in predetermined locations. The melted materials can flow together, achieving a bond when cooled. In some aspects, ultrasonic welding can be used prior to forming a fully assembled sheath. For example, in such aspects, ultrasonic welding can be used to bond any desired parts at any step of the manufacturing.

Also disclosed are methods of making a sheath comprising a reinforcing jacket. In such methods, the sheath components, such as the inner liner and the outer layer, are formed by any of the disclosed above methods and assembled together to form a sheath. Then a reinforcing jacket having a proximal end and a distal end is disposed such that it overlayer at least a portion of the outer layer. The methods further comprise substantially seamlessly bonding the distal end of the reinforcing jacket to at least a portion of the outer layer. It is understood that any of the disclosed above or generally known in the art bonding methods can be used.

The reinforcing jacket can comprise any of the disclosed above components, wherein the reinforcing jacket comprises any of the disclosed above elastomers and any of the disclosed above reinforcing elements.

In still further aspects, the reinforcing jacket can be formed by any methods known in the art. For example, the reinforcing jacket can be formed by injection molding, extruding or reflow process. In still further aspects, any of the disclosed herein reinforcing members can be embedded in a soft polymer to form the reinforcing jacket. For example, and without limitation, during the reflow process, a reinforcing element can be disposed on top of the polymer layer and is exposed to heat to allow the reinforcing element and the polymer to fuse together. In yet other exemplary aspects, the reinforcing jacket can be formed by injection molding. In such aspects, the reinforcing element can be positioned within a mold, and a polymer is injected over it. Yet, in other exemplary and unlimiting aspects, the reinforcing jacket can be formed by an extrusion process. In such exemplary aspects, the heated polymer can be extruded to a tube while in the parallel feed of the reinforcing element to allow the polymer and reinforcing element to combine.

Also are methods of making a sheath comprising a ballooning guard. In such methods, the sheath components, such as the inner liner and the outer layer, are formed by any of the disclosed above methods and assembled together to form a sheath. Then a ballooning guard having a proximal end and a distal end is disposed such that it overlayer at least a portion of the outer layer, and wherein the ballooning guard is configured to remain outside of a subject's vessel and to maintain hemostasis. The methods further connecting the proximal end of the ballooning guard to a most proximal portion of the outer layer and/or a hub of the sheath, wherein the distal end of the ballooning guard radially circumscribes at least a portion of the outer layer and wherein the distal end is not bonded to the outer layer. The ballooning guard, as described herein, is configured to adjust a length of the guard as a function of an insertion depth of the inner liner and outer layer of the sheath into a subject's vessel

In still further aspects, the disclosed herein methods can comprise a step of disposing a hydrophilic coating layer on the outer surface of the layer of the elastomeric polymer. Any disclosed herein hydrophilic coating can be used.

Sheaths of the present disclosure can be used with various methods of introducing a prosthetic device into a patient's vasculature. One such method comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some methods, the expandable sheath can be sutured to the patient's skin at the insertion site so that once the sheath is inserted at the proper distance within the patient's vasculature, it does not move once the implantable device starts to travel through the sheath.

Disclosed aspects of an expandable sheath can be used with other delivery and minimally invasive surgical components, such as an introducer and loader. An introducer can be inserted into the expandable sheath, and the introducer/sheath combination can be fully inserted into vasculature over a guiding device. such as a 0.35" guidewire. Once the sheath and introducer are fully inserted into a patient's vasculature, in some aspects, the expandable sheath can be sutured in place at the insertion site. In this manner, the expandable sheath can be substantially prevented from moving once positioned within the patient.

The introducer can then be removed, and a medical device, such as a transcatheter heart valve, can be inserted into the sheath, in some instances, using a loader. Such methods can additionally comprise placing the tissue heart valve in a crimped state on the distal end portion of an elongated delivery apparatus and inserting the elongated delivery device with the crimped valve into and through the expandable sheath. Next, the delivery apparatus can be advanced through the patient's vasculature to the treatment site, where the valve can be implanted.

Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (*e.g.,* the expanded configuration) just in the area where the medical device is currently located. Once the medical device passes a particular location of the sheath, the sheath can at least partially contract to the smaller diameter of its original configuration. The expandable sheath can thus be expanded without the use of inflatable balloons or other dilators. Once the medical device is implanted, the sheath and any sutures holding it in place can be removed. In some exemplary aspects, the sheath is removed without rotating it.

### EXAMPLES

The following examples are put forth so as to provide those of ordinary skill in the art with a complete disclosure and description of how the compounds, compositions, articles, devices and/or methods claimed herein are made and evaluated and are intended to be purely exemplary and are not intended to limit the disclosure. Efforts have been made to ensure accuracy with respect to numbers (*e.g*., amounts, temperature, etc.), but some errors and deviations should be accounted for.

Unless indicated otherwise, parts are parts by weight, temperature is degrees C or is at ambient temperature, and pressure is at or near atmospheric or full vacuum.

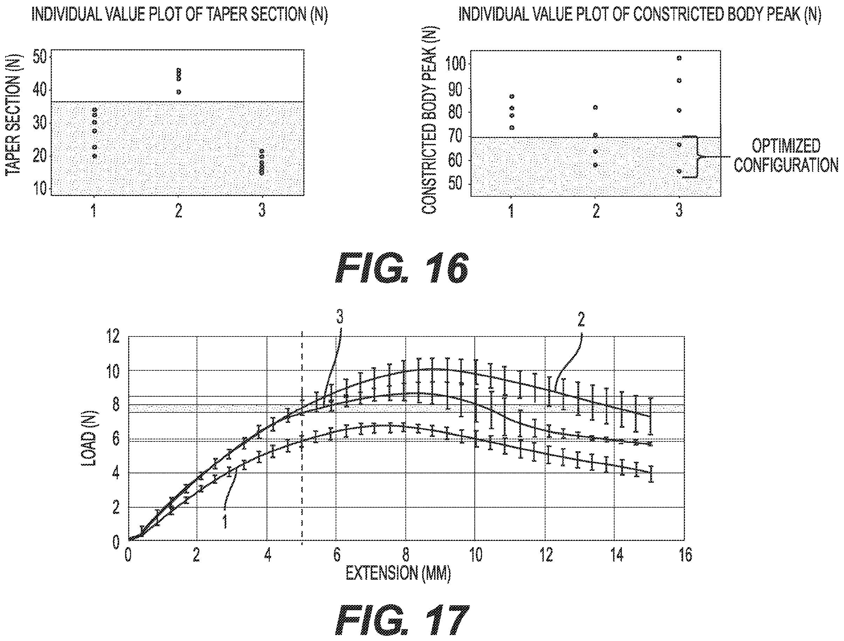

**FIG. 16** depicts exemplary experimental data measured for the insertion force required to insert a medical device in one of the disclosed herein sheaths **3** as compared to commercially available sheaths **1** and **2.** The force was measured using a typical delivery system on a Zwick push force test machine. It can be seen that the required insertion force measured in the taper section of the sheath is lower than the one needed for the commercially available sheaths **1** and **2.** Similarly, the insertion force measured in the constricted body peak shows better results for the disclosed sheath **3** as compared to the commercially available sheaths **1** and **2.**

**FIG. 17** depicts experimental data of the load measured as a function of the extension of one of the disclosed herein sheaths **3** and the commercially available sheaths **1** and **2.** It can be seen that the disclosed sheath demonstrates comparable results with the clinically acceptable commercial sheath.

In view of the described processes and compositions, hereinbelow are described certain more particularly described aspects of the disclosures. These particularly recited aspects should not, however, be interpreted to have any limiting effect on any different claims containing different or more general teachings described herein, or that the "particular" aspects are somehow limited in some way other than the inherent meanings of the language and formulas literally used therein.

In view of the many possible aspects to which the principles of the disclosed disclosure can be applied, it should be recognized that the illustrated aspects are only some examples of the disclosure and should not be taken as limiting the scope of the disclosure. The invention is defined by the following claims.

## Claims

1. An expandable sheath (4400) comprising:
a proximal section (4402) having a proximal end (4414), a distal end (4416), and an elongated body (4418) that extends between the proximal end (4414) and distal end (4416), wherein the elongated body (4418) defines a lumen (4406) extending therethrough, and
a tip section (4404) extending distally from the distal end (4416) of the proximal section (4402) and defining a lumen (4406) extending therethrough, the tip section (4404) comprising:
an inner liner layer (4408);
an intermediate layer (4426) disposed radially outward of the inner liner layer (4408), the intermediate layer (4426) having a lower stiffness than the inner liner layer (4408);
an elastomeric outer layer (4410) disposed radially outward of the intermediate layer (4426);
wherein the intermediate layer (4426) extends distally beyond the inner liner layer (4408) and the elastomeric outer layer (4410) to define a distal end (4422) of the tip section (4404), and
wherein the tip section (4404) has an initial unexpanded configuration wherein a distal end (4422) of the tip section (4404) has a first diameter, and an expanded configuration wherein the distal end (4422) of the tip section (4404) has a second diameter that is greater than the first diameter; and
wherein at least the inner liner layer (4408) defines a slit (4412) extending distally along at least a portion of the tip section (4404).

2. The expandable sheath (4400) of claim 1, wherein the tip section (4404) has a distal taper (4424) wherein the first diameter of the tip section (4404) is less than a diameter of a proximal end (4420) of the tip section (4404).

3. The expandable sheath (4400) of any one of the above claims, wherein the slit (4412) does not extend to the distal end (4422) of the tip section (4404) in the initial unexpanded configuration, such that the distal end (4422) of the tip section (4404) is circumferentially continuous in the initial unexpanded configuration, and/or, wherein the slit (4412) extends to the distal end (4422) of the tip section (4404) when the tip section (4404) is in the expanded configuration, such that the distal end (4422) of the tip section (4404) is circumferentially discontinuous in the expanded configuration,
wherein the slit (4412) includes side edges that are circumferentially displaced from each other when the tip section (4404) is in the expanded configuration, wherein, preferably, the slit (4412) has a generally U-shaped configuration, with the arms of the U-shaped configuration comprising the side edges, when the tip section (4404) is in the expanded configuration,
wherein the side edges are smooth and continuous.

4. The expandable sheath (4400) of any one of the above claims, wherein the slit (4412) extends radially through a thickness that extends between an outer surface of the outer layer (4410) and an inner surface of the inner liner layer (4408),
wherein the thickness includes the inner liner layer (4408), the intermediate layer (4426) and the outer layer (4410).

5. The expandable sheath (4400) of any one of the above claims, wherein the slit (4412) is at least partially reflowed in the initial unexpanded configuration,
wherein the slit (4412) is at least partially visually concealed when at least partially reflowed in the initial unexpanded configuration,
wherein the slit (4412) is at least partially smoothed over when at least partially reflowed in the initial unexpanded configuration.

6. The expandable sheath (4400) of any one of the above claims, wherein the outer layer (4410) extends proximally from the tip section (4404) and over at least a portion of the proximal section (4402),
wherein at least a proximal portion of the outer layer (4410) of the tip section (4404) and the outer layer (4410) of the proximal section (4402) have a uniform thickness.

7. The expandable sheath (4400) of any one of the above claims, wherein the outer layer (4410) is configured to radially compress the inner liner layer (4408),
wherein the outer layer (4410) extends distally beyond the inner liner layer (4408) and/or, wherein the outer layer (4410) is thermally coupled to the inner liner layer (4408) via the intermediate layer (4426).

8. The expandable sheath (4400) of any one of the above claims, wherein the intermediate layer (4426) extends longitudinally from the distal end (4416) of the proximal section (4402) to the distal end (4422) of the tip section (4404), and/or, wherein the intermediate layer (4426) tapers distally.

9. The expandable sheath (4400) of any one of the above claims, wherein the intermediate layer (4426) comprises a plurality of distinct sub-layers (4444), each sub-layer (4444) formed of a material distinct from each other sub-layer (4444), wherein, preferably, the sub-layers (4444) include a polyethylene layer and a tie layer and a polyether block amide layer,
wherein the polyethylene layer is an inner sub-layer (4446) and wherein the polyether block amide layer is an outer sub-layer (4442) and the tie layer couples the polyethylene and polyether block amide layers.

10. The expandable sheath (4400) of any one of the above claims, wherein the tip section (4404) further comprises a tie layer coextruded with the inner liner layer (4408) and the intermediate layer (4426).

11. The expandable sheath (4400) of any one of the above claims, wherein the proximal section (4402) of the sheath (4400) includes a proximal extension of the inner liner layer (4408) defining an additional slit (4412) and wherein the proximal extension of the inner liner layer (4408) has a scroll configuration,
wherein the proximal section (4402) of the sheath (4400) includes a proximal extension of the outer layer (4410) and wherein the outer layer (4410) is configured to radially compress the proximal extension of the inner liner layer (4408).

12. The expandable sheath (4400) of any one of the above claims, wherein the proximal section (4402) includes a proximal portion of the inner liner layer (4408) and wherein the proximal portion of the inner liner layer (4408) has an overlapping portion.

13. The expandable sheath (4400) of any one of the above claims, wherein the intermediate layer (4426) includes three sublayers (4444).

14. The expandable sheath (4400) of claim 13, wherein an intermediate one of the sublayers (4444) extends distally beyond the inner liner layer (4408) and the elastomeric outer layer (4410) to define a distal end (4422) of the tip section (4404), wherein, preferably, all three sublayers (4444) extend distally beyond the inner liner layer (4408) and the elastomeric outer layer (4410) to define a distal end (4422) of the tip section (4404).

15. The expandable sheath (4400) of claim 12, wherein the overlapping portion is overlapping, in a non-expanded configuration, at least 44 degrees up to 120 degrees, or, wherein the overlapping portion is overlapping, in a non-expanded configuration, at least 70 degrees up to 120 degrees.

## Patentansprüche

1. Expandierbare Hülle (4400), umfassend:
eine proximale Sektion (4402) mit einem proximalen Ende (4414), einem distalen Ende (4416) und einem langgestreckten Körper (4418), der sich zwischen dem proximalen Ende (4414) und dem distalen Ende (4416) erstreckt, wobei der langgestreckte Körper (4418) ein Lumen (4406) definiert, das sich durch diesen hindurch erstreckt, und
eine Spitzensektion (4404), die sich distal von dem distalen Ende (4416) der proximalen Sektion (4402) erstreckt und ein Lumen (4406) definiert, das sich durch diese hindurch erstreckt, wobei die Spitzensektion (4404) umfasst:
eine innere Auskleidungsschicht (4408);
eine Zwischenschicht (4426), die radial außerhalb der inneren Auskleidungsschicht (4408) angeordnet ist, wobei die Zwischenschicht (4426) eine geringere Steifigkeit als die innere Auskleidungsschicht (4408) aufweist;
eine elastomere äußere Schicht (4410), die radial außerhalb der Zwischenschicht (4426) angeordnet ist;
wobei sich die Zwischenschicht (4426) distal über die innere Auskleidungsschicht (4408) und die elastomere äußere Schicht (4410) hinaus erstreckt, um ein distales Ende (4422) der Spitzensektion (4404) zu definieren, und
wobei die Spitzensektion (4404) eine anfängliche nicht-expandierte Konfiguration aufweist, bei der ein distales Ende (4422) der Spitzensektion (4404) einen ersten Durchmesser aufweist, und eine expandierte Konfiguration, bei der das distale Ende (4422) der Spitzensektion (4404) einen zweiten Durchmesser aufweist, der größer als der erste Durchmesser ist; und
wobei wenigstens die innere Auskleidungsschicht (4408) einen Schlitz (4412) definiert, der sich distal entlang wenigstens eines Abschnitts der Spitzensektion (4404) erstreckt.

2. Expandierbare Hülle (4400) nach Anspruch 1, wobei die Spitzensektion (4404) eine distale Verjüngung (4424) aufweist, wobei der erste Durchmesser der Spitzensektion (4404) kleiner ist als ein Durchmesser eines proximalen Endes (4420) der Spitzensektion (4404).

3. Expandierbare Hülle (4400) nach einem der vorangehenden Ansprüche, wobei sich der Schlitz (4412) in der anfänglichen nicht-expandierten Konfiguration nicht bis zum distalen Ende (4422) der Spitzensektion (4404) erstreckt, so dass das distale Ende (4422) der Spitzensektion (4404) in der anfänglichen nicht-expandierten Konfiguration in Umfangsrichtung durchgehend ist, und/oder, wobei sich der Schlitz (4412) bis zum distalen Ende (4422) der Spitzensektion (4404) erstreckt, wenn sich die Spitzensektion (4404) in der expandierten Konfiguration befindet, so dass das distale Ende (4422) der Spitzensektion (4404) in der expandierten Konfiguration in Umfangsrichtung unterbrochen ist,
wobei der Schlitz (4412) Seitenränder aufweist, die in Umfangsrichtung gegeneinander versetzt sind, wenn sich die Spitzensektion (4404) in der expandierten Konfiguration befindet, wobei der Schlitz (4412) vorzugsweise eine allgemein U-förmige Konfiguration aufweist, wobei die Arme der U-förmigen Konfiguration die Seitenränder umfassen, wenn sich die Spitzensektion (4404) in der expandierten Konfiguration befindet,
wobei die Seitenkanten glatt und durchgehend sind.

4. Expandierbare Hülle (4400) nach einem der vorangehenden Ansprüche, wobei sich der Schlitz (4412) radial durch eine Dicke erstreckt, die sich zwischen einer äußeren Oberfläche der äußeren Schicht (4410) und einer inneren Oberfläche der inneren Auskleidungsschicht (4408) erstreckt,
wobei die Dicke die innere Auskleidungsschicht (4408), die Zwischenschicht (4426) und die äußere Schicht (4410) umfasst.

5. Expandierbare Hülle (4400) nach einem der vorangehenden Ansprüche, wobei der Schlitz (4412) in der anfänglichen nicht-expandierten Konfiguration wenigstens teilweise wieder verflüssigt wird,
wobei der Schlitz (4412) wenigstens teilweise visuell verdeckt ist, wenn er in der anfänglichen nicht-expandierten Konfiguration wenigstens teilweise wieder verflüssigt wird,
wobei der Schlitz (4412) wenigstens teilweise geglättet wird, wenn er in der anfänglichen, nicht expandierten Konfiguration wenigstens teilweise wieder verflüssigt wird.

6. Expandierbare Hülle (4400) nach einem der vorangehenden Ansprüche, wobei sich die äußere Schicht (4410) proximal von der Spitzensektion (4404) und über wenigstens einen Abschnitt der proximalen Sektion (4402) erstreckt,
wobei wenigstens ein proximaler Abschnitt der äußeren Schicht (4410) der Spitzensektion (4404) und die äußere Schicht (4410) der proximalen Sektion (4402) eine gleichmäßige Dicke aufweisen.

7. Expandierbare Hülle (4400) nach einem der vorangehenden Ansprüche, wobei die äußere Schicht (4410) dazu konfiguriert ist, die innere Auskleidungsschicht (4408) radial zu komprimieren,
wobei sich die äußere Schicht (4410) distal über die innere Auskleidungsschicht (4408) hinaus erstreckt, und/oder wobei die äußere Schicht (4410) über die Zwischenschicht (4426) mit der inneren Auskleidungsschicht (4408) thermisch gekoppelt ist.

8. Expandierbare Hülle (4400) nach einem der vorangehenden Ansprüche, wobei sich die Zwischenschicht (4426) in Längsrichtung vom distalen Ende (4416) der proximalen Sektion (4402) bis zum distalen Ende (4422) der Spitzensektion (4404) erstreckt, und/oder wobei sich die Zwischenschicht (4426) distal verjüngt.

9. Expandierbare Hülle (4400) nach einem der vorangehenden Ansprüche, wobei die Zwischenschicht (4426) mehrere unterschiedliche Teilschichten (4444) umfasst, wobei jede Teilschicht (4444) aus einem Material gebildet ist, das sich von jeder anderen Teilschicht (4444) unterscheidet, wobei die Teilschichten (4444) vorzugsweise eine Polyethylenschicht und eine Bindeschicht und eine Polyetherblockamidschicht umfassen,
wobei die Polyethylenschicht eine innere Teilschicht (4446) ist und wobei die Polyetherblockamidschicht eine äußere Teilschicht (4442) ist und die Bindeschicht die Polyethylenschicht und die Polyetherblockamidschicht koppelt.

10. Expandierbare Hülle (4400) nach einem der vorangehenden Ansprüche, wobei die Spitzensektion (4404) ferner eine Bindeschicht umfasst, die mit der inneren Auskleidungsschicht (4408) und der Zwischenschicht (4426) koextrudiert wird.

11. Expandierbare Hülle (4400) nach einem der vorangehenden Ansprüche, wobei die proximale Sektion (4402) der Hülle (4400) eine proximale Erweiterung der inneren Auskleidungsschicht (4408) aufweist, die einen zusätzlichen Schlitz (4412) definiert, und wobei die proximale Erweiterung der inneren Auskleidungsschicht (4408) eine Rollen-Konfiguration aufweist,
wobei die proximale Sektion (4402) der Hülle (4400) eine proximale Erweiterung der äußeren Schicht (4410) aufweist und wobei die äußere Schicht (4410) dazu konfiguriert ist, die proximale Erweiterung der inneren Auskleidungsschicht (4408) radial zu komprimieren.

12. Expandierbare Hülle (4400) nach einem der vorangehenden Ansprüche, wobei die proximale Sektion (4402) einen proximalen Abschnitt der inneren Auskleidungsschicht (4408) aufweist und wobei der proximale Abschnitt der inneren Auskleidungsschicht (4408) einen überlappenden Abschnitt aufweist.

13. Expandierbare Hülle (4400) nach einem der vorangehenden Ansprüche, wobei die Zwischenschicht (4426) drei Teilschichten (4444) umfasst.

14. Expandierbare Hülle (4400) nach Anspruch 13, wobei sich eine mittlere der Teilschichten (4444) distal über die innere Auskleidungsschicht (4408) und die elastomere äußere Schicht (4410) hinaus erstreckt, um ein distales Ende (4422) der Spitzensektion (4404) zu definieren, wobei sich vorzugsweise alle drei Teilschichten (4444) distal über die innere Auskleidungsschicht (4408) und die elastomere äußere Schicht (4410) hinaus erstrecken, um ein distales Ende (4422) der Spitzensektion (4404) zu definieren.

15. Expandierbare Hülle (4400) nach Anspruch 12, wobei der überlappende Abschnitt in einer nicht-expandierten Konfiguration um wenigstens 44 Grad bis zu 120 Grad überlappt, oder wobei der überlappende Abschnitt in einer nicht-expandierten Konfiguration um wenigstens 70 Grad bis zu 120 Grad überlappt.

## Revendications

1. Gaine extensible (4400) comprenant :
une section proximale (4402) présentant une extrémité proximale (4414), une extrémité distale (4416) et un corps allongé (4418) qui s'étend entre l'extrémité proximale (4414) et l'extrémité distale (4416), le corps allongé (4418) définissant une lumière (4406) s'étendant à travers celui-ci et
une section de pointe (4404) s'étendant de manière distale à partir de l'extrémité distale (4416) de la section proximale (4402) et définissant une lumière (4406) s'étendant à travers celle-ci, la section de pointe (4404) comprenant :
une couche de revêtement interne (4408) ;
une couche intermédiaire (4426) disposée radialement vers l'extérieur par rapport à la couche de revêtement interne (4408), la couche intermédiaire (4426) présentant une rigidité inférieure à celle de la couche de revêtement interne (4408) ;
une couche externe (4410) élastomère disposée radialement vers l'extérieur par rapport à la couche intermédiaire (4426) ;
la couche intermédiaire (4426) s'étendant de manière distale au-delà de la couche de revêtement interne (4408) et de la couche externe (4410) élastomère afin de définir une extrémité distale (4422) de la section de pointe (4404) et
la section de pointe (4404) présentant une configuration initiale non étendue dans laquelle une extrémité distale (4422) de la section de pointe (4404) présente un premier diamètre et une configuration étendue dans laquelle l'extrémité distale (4422) de la section de pointe (4404) présente un second diamètre qui est supérieur au premier diamètre ; et
au moins la couche de revêtement interne (4408) définissant une fente (4412) s'étendant de manière distale le long d'au moins une partie de la section de pointe (4404).

2. Gaine extensible (4400) selon la revendication 1, la section de pointe (4404) présentant un effilement distal (4424), le premier diamètre de la section de pointe (4404) étant inférieur à un diamètre d'une extrémité proximale (4420) de la section de pointe (4404).

3. Gaine extensible (4400) selon l'une quelconque des revendications précédentes, la fente (4412) ne s'étendant pas jusqu'à l'extrémité distale (4422) de la section de pointe (4404) dans la configuration initiale non étendue, de telle sorte que l'extrémité distale (4422) de la section de pointe (4404) est circonférentiellement continue dans la configuration initiale non étendue et/ou la fente (4412) s'étendant jusqu'à l'extrémité distale (4422) de la section de pointe (4404) lorsque la section de pointe (4404) est dans la configuration étendue, de telle sorte que l'extrémité distale (4422) de la section de pointe (4404) est circonférentiellement discontinue dans la configuration étendue,
la fente (4412) comprenant des bords latéraux qui sont déplacés circonférentiellement l'un par rapport à l'autre lorsque la section de pointe (4404) est dans la configuration étendue, de préférence, la fente (4412) présentant une configuration généralement en forme de U, les bras de la configuration en forme de U comprenant les bords latéraux, lorsque la section de pointe (4404) est dans la configuration étendue,
les bords latéraux étant lisses et continus.

4. Gaine extensible (4400) selon l'une quelconque des revendications précédentes, la fente (4412) s'étendant radialement à travers une épaisseur qui s'étend entre une surface externe de la couche externe (4410) et une surface interne de la couche de revêtement interne (4408),
l'épaisseur comprenant la couche de revêtement interne (4408), la couche intermédiaire (4426) et la couche externe (4410).

5. Gaine extensible (4400) selon l'une quelconque des revendications précédentes, la fente (4412) étant au moins partiellement rétractée dans la configuration initiale non étendue,
la fente (4412) étant au moins partiellement dissimulée visuellement lorsqu'elle est au moins partiellement rétractée dans la configuration initiale non étendue,
la fente (4412) étant au moins partiellement lissée lorsqu'elle est au moins partiellement rétractée dans la configuration initiale non étendue.

6. Gaine extensible (4400) selon l'une quelconque des revendications précédentes, la couche externe (4410) s'étendant de manière proximale à partir de la section de pointe (4404) et sur au moins une partie de la section proximale (4402),
au moins une partie proximale de la couche externe (4410) de la section de pointe (4404) et la couche externe (4410) de la section proximale (4402) présentant une épaisseur uniforme.

7. Gaine extensible (4400) selon l'une quelconque des revendications précédentes, la couche externe (4410) étant conçue pour comprimer radialement la couche de revêtement interne (4408),
la couche externe (4410) s'étendant de manière distale au-delà de la couche de revêtement interne (4408) et/ou la couche externe (4410) étant accouplée thermiquement à la couche de revêtement interne (4408) par l'intermédiaire de la couche intermédiaire (4426).

8. Gaine extensible (4400) selon l'une quelconque des revendications précédentes, la couche intermédiaire (4426) s'étendant longitudinalement à partir de l'extrémité distale (4416) de la section proximale (4402) vers l'extrémité distale (4422) de la section de pointe (4404) et/ou la couche intermédiaire (4426) s'effilant de manière distale.

9. Gaine extensible (4400) selon l'une quelconque des revendications précédentes, la couche intermédiaire (4426) comprenant une pluralité de sous-couches (4444) distinctes, chaque sous-couche (4444) étant formée d'un matériau distinct de celui de chaque autre sous-couche (4444), de préférence, les sous-couches (4444) comprenant une couche de polyéthylène et une couche de liaison et une couche de polyéther-bloc-amide,
la couche de polyéthylène étant une sous-couche interne (4446) et la couche de polyéther-bloc-amide étant une sous-couche externe (4442) et la couche de liaison accouplant les couches de polyéthylène et de polyéther-bloc-amide.

10. Gaine extensible (4400) selon l'une quelconque des revendications précédentes, la section de pointe (4404) comprenant en outre une couche de liaison coextrudée avec la couche de revêtement interne (4408) et la couche intermédiaire (4426).

11. Gaine extensible (4400) selon l'une quelconque des revendications précédentes, la section proximale (4402) de la gaine (4400) comprenant un prolongement proximal de la couche de revêtement interne (4408) définissant une fente (4412) supplémentaire et le prolongement proximal de la couche de revêtement interne (4408) présentant une configuration en spirale,
la section proximale (4402) de la gaine (4400) comprenant un prolongement proximal de la couche externe (4410) et la couche externe (4410) étant conçue pour comprimer radialement le prolongement proximal de la couche de revêtement interne (4408).

12. Gaine extensible (4400) selon l'une quelconque des revendications précédentes, la section proximale (4402) comprenant une partie proximale de la couche de revêtement interne (4408) et la partie proximale de la couche de revêtement interne (4408) présentant une partie de chevauchement.

13. Gaine extensible (4400) selon l'une quelconque des revendications précédentes, la couche intermédiaire (4426) comprenant trois sous-couches (4444).

14. Gaine extensible (4400) selon la revendication 13, une sous-couche intermédiaire des sous-couches (4444) s'étendant de manière distale au-delà de la couche de revêtement interne (4408) et de la couche externe (4410) élastomère afin de définir une extrémité distale (4422) de la section de pointe (4404), de préférence, toutes les trois sous-couches (4444) s'étendant de manière distale au-delà de la couche de revêtement interne (4408) et de la couche externe (4410) élastomère afin de définir une extrémité distale (4422) de la section de pointe (4404).

15. Gaine extensible (4400) selon la revendication 12, la partie de chevauchement chevauchant, dans une configuration non étendue, au moins 44 degrés jusqu'à 120 degrés ou la partie de chevauchement chevauchant, dans une configuration non étendue, au moins 70 degrés jusqu'à 120 degrés.
